# EUROPEAN PATENT APPLICATION

(11) **EP 4 454 559 A1**
(43) Date of publication of application: **30.10.2024**
(21) Application number: 24169679.8
(22) Date of filing: 11.04.2024
(51) Int. Cl.: A61B 5/268, A61B 5/28, C08F 212/14, C08F 220/18, C08F 220/24, C08F 220/38, C08F 230/02, C08F 230/08

(54) **BIO-ELECTRODE AND METHOD FOR MANUFACTURING BIO-ELECTRODE**

(30) Priority: 26.04.2023 JP 2023072064
(71) Applicant: SHIN-ETSU CHEMICAL CO., LTD., Tokyo 1000005 (JP)
(72) Inventor: Hatakeyama, Jun, Niigata (JP)
(74) Representative: Sonnenhauser, Thomas Martin

(57) **Abstract**

The present invention is a bio-electrode, including: a living-body contact layer; and a substrate, wherein the living-body contact layer has a micropillar having a diameter within a range of 0.01 to 500 um and a height within a range of 0.1 to 1,000 um, and has an ionic polymer (A) as a material, and the component (A) has a repeating unit-a of one or more selected from a fluorosulfonic acid, a fluorosulfonimide, and a fluorosulfonamide, and has a weight-average molecular weight within a range of 1,000 to 500,000. This provides: a bio-electrode that is a thin film and highly transparent, that has high sensitivity to a bio-signal, excellent bio-compatibility, and a lightweight property, that can be manufactured at a low cost, that does not considerably deteriorate the sensitivity to the bio-signal even when being wet, dried, or attached to skin for a long term, and that is comfortable and free from itching, red spots, rash, etc. of the skin; and a method for manufacturing a bio-electrode.

## Description

### TECHNICAL FIELD

The present invention relates to a bio-electrode and a method for manufacturing a bio-electrode.

### BACKGROUND ART

Recent growing popularity of Internet of Things (IoT) has accelerated development of wearable devices. In the fields of medicine and sports, a wearable device for constantly monitoring a physical state is also demanded, and is a growing field in the future. Particularly, the worldwide epidemic of the new coronavirus (COVID-19) has caused heavy medical burden, and there is a crying need for home care for people who are not infected by the virus and acceleration of the home care.

In the field of medicine, for example, sold is a wearable device that monitors the state of an organ of the body by sensing a small current, like electrocardiographic measurement for sensing motion of the heart using an electric signal. In the electrocardiographic measurement, electrodes on which a hydrated gel is applied are put on the body for measurement, which is only single-time measurement for a short period of time. In contrast, the development of the medical wearable device as described above aims at developing a device that constantly monitors a health state for consecutive several weeks. Thus, a bio-electrode used in the medical wearable device is required to be capable of acquiring a bio-signal even when used for a long term in daily life with showering, bathing, sweating, and the like, be free from itching and skin allergy, and have comfortableness. In addition to these requirements, the bio-electrode is also required to be light and thin to such an extent as to provide no feeling of wearing, and be able to be manufactured at a low cost with high productivity.

As the medical wearable device, there is a type adhering to the body and a type incorporated into clothing. As the type adhering to the body, proposed is a bio-electrode made of a water-soluble gel containing water and an electrolyte, which are materials for a conductive paste (Patent Document 1). The water-soluble gel contains, as the electrolytes, sodium, potassium, and calcium in a water-soluble polymer to retain water, and converts a change in concentration of ions from the skin to an electricity. Meanwhile, as the type incorporated into clothing, proposed is a method using a cloth in which a conductive polymer such as poly-3,4-ethylenedioxythiophene-polystyrenesulfonate (PEDOT-PSS) or a silver paste is incorporated into fibers for an electrode (Patent Document 2).

However, use of the above water-soluble gel containing water and an electrolyte has a problem that conductivity is lost when the water-soluble gel runs dry to loss water. Meanwhile, use of metal with high ionization tendency such as copper has a problem of a risk of causing skin allergy for some people. Use of the conductive polymer such as PEDOT-PSS also has the problem of the risk of causing skin allergy due to the strong acidity of the conductive polymer, and has a problem of peeling away of the conductive polymer from fibers during laundry.

It has been investigated to use metal nanowire, carbon black, carbon nanotube, etc. as the electrode material because of excellent conductivity (Patent Documents 3, 4, and 5). Since the metal nanowire increases contact probability of the wires, current can be carried at a small addition amount. However, the metal nanowire is a material with a pointed tip, and thereby causes skin allergy. As above, the material itself not causing an allergic reaction may deteriorate bio-compatibility due to a shape or irritation of the material, and it has been difficult to achieve both the conductivity and the bio-compatibility.

A metal film can be considered to function as an excellent bio-electrode due to extremely high conductivity, but not necessarily so. Emitted from skin with heartbeat are not only a small current but also sodium ions, potassium ions, and calcium ions. Thus, a change in concentration of the ions need to be converted into current, but a precious metal, which is hardly ionized, has poor efficiency of conversion of the ions from skin into current. Accordingly, a bio-electrode using the precious metal has high impedance, and current carrying with skin causes high resistance.

It is also necessary to have adhesiveness as the bio-electrode. The bio-electrode having adhesiveness can obtain an accurate bio-signal even when the body is moving (Non Patent Document 1).

Bio-electrode materials in which an ionic polymer is mixed with a silicone adhesive have been proposed (Patent Documents 6 and 7). These materials has high ion conductivity, and in addition, the materials into which a conductive powder such as carbon and silver is added also has high electron conductivity, and thereby function as an excellent bio-electrode. The ionic polymer that has high ion conductivity and that does not penetrate the skin is combined with the silicone adhesive that is less allergic to the skin, that has high water repellency, and that provides an effect of preventing occurrence of itching and redness of the skin after the bio-electrode material is peeled off, whereby bio-signals can be stably obtained without peeling-off of the bio-electrode material even when the bio-electrode material has been attached for a long term including daily bathing and exercise. However, the bio-electrode material has been required to improve comfortableness at the time of being attached and worn for a longer period of time.

A gecko can climb a vertical window glass without scaffold. A Van der Waals force between fine hairs on the soles and a surface of the window glass enables move without falling from the window glass (Non Patent Document 2). By using this principle, an adhesive bio-electrode in which a silicone containing conductive particles such as silver is processed into a fine mushroom shape has been proposed (Non Patent Document 3).

A method for producing a mold to produce cilia on the gecko's pads has been proposed (Patent Document 8). Production of an adhesive sheet with a form of the gecko's pads with the mold has a merit of high productivity.

When the mushroom-shaped cilia having a wide tip on the gecko's pads are produced with the mold, a stress may be applied at the cilia part in releasing the mold to tear the cilia. To reduce the stress in releasing the mold, a method for releasing a curved substrate has been proposed (Patent Document 9).

### CITATION LIST

### PATENT LITERATURE

Patent Document 1: WO 2013/039151 A1
Patent Document 2: JP 2015-100673 A
Patent Document 3: JP 05-095924 A
Patent Document 4: JP 2003-225217 A
Patent Document 5: JP 2015-019806 A
Patent Document 6: JP 2018-126496 A
Patent Document 7: JP 2018-130533 A
Patent Document 8: JP 2023-501603 A
Patent Document 9: US 9566722 B2

### NON PATENT LITERATURE

Non Patent Document 1: JOM Vol. 68, No. 4, p. 1145, (2016)
Non Patent Document 2: Nature Vol. 405, p 681, June (2000)
Non Patent Document 3: Adv. Healthcare Mater. (2018), 7, 1700994

### SUMMARY OF INVENTION

### TECHNICAL PROBLEM

The present invention has been made to solve the above problems. An object of the present invention is to provide: a bio-electrode that is a thin film and highly transparent, that has good adhesiveness to a living body, high sensitivity to a bio-signal, and excellent bio-compatibility and a lightweight property, that can be manufactured at a low cost, that does not considerably deteriorate the sensitivity to the bio-signal even when being wet, dried, or attached to the skin for a long term, and that is comfortable and free from itching, red spots, rash, etc. of the skin; and a method for manufacturing a bio-electrode.

### SOLUTION TO PROBLEM

To solve the above problem, the present invention provides a bio-electrode comprising:
a living-body contact layer; and
a substrate, wherein
the living-body contact layer has a micropillar having a diameter within a range of 0.01 to 500 um and a height within a range of 0.1 to 1,000 um, and has an ionic polymer (A) as a material, and
the component (A) has a repeating unit-a of one or more selected from a fluorosulfonic acid, a fluorosulfonimide, and a fluorosulfonamide, and has a weight-average molecular weight within a range of 1,000 to 500,000.

Such a bio-electrode is the bio-electrode that is a thin film and highly transparent, that has good adhesiveness to a living body, high sensitivity to a bio-signal, and excellent bio-compatibility and a lightweight property, that can be manufactured at a low cost, that does not considerably deteriorate the sensitivity to the bio-signal even when being wet, dried, or attached to the skin for a long term, and that is comfortable and free from itching, red spots, rash, etc. of the skin.

In the present invention, the repeating unit-a preferably has a partial structure represented by the following general formulae (1)-1 to (1)-4, 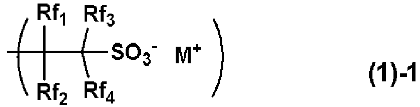 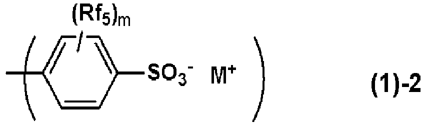 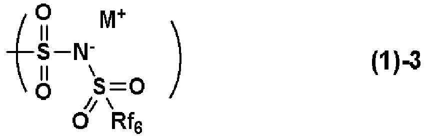 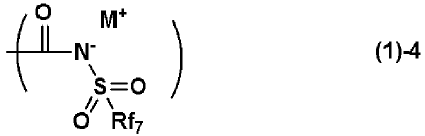 wherein Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; M⁺ represents an ion selected from an ammonium ion, a sodium ion, a potassium ion, and a silver ion; and "m" represents an integer of 1 to 4.

The repeating unit-a having such a structure yields the bio-electrode having more excellent conductivity and bio-compatibility.

In the present invention, the repeating unit-a preferably has one or more selected from repeating units-A1 to -A4 represented by the following general formulae (2), 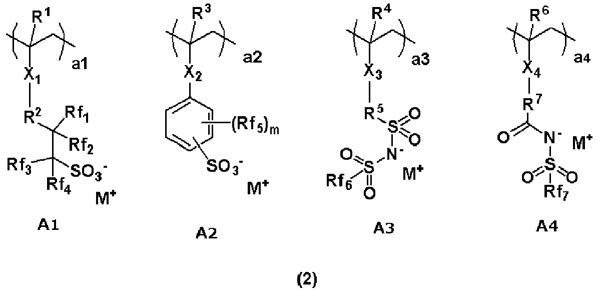 wherein R¹, R³, R⁴, and R⁶ each independently represent a hydrogen atom or a methyl group; R², R⁵, and R⁷ each independently represent a single bond or a linear, branched, or cyclic hydrocarbon group having 1 to 13 carbon atoms and optionally having an ester group, an ether group, or both of them; X₁, X₂, X₃, and X₄ each independently represent any one of a single bond, a phenylene group, a naphthylene group, an ether group, an ester group, and an amide group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; "m" represents an integer of 1 to 4; a1, a2, a3, and a4 satisfy 0≤a1≤1.0, 0≤a2≤1.0, 0≤a3≤1.0, 0≤a4≤1.0, and 0<a1+a2+a3+a4≤1.0; and M⁺ represents an ion selected from an ammonium ion, a sodium ion, a potassium ion, and a silver ion.

The repeating unit-a having such a structure yields the bio-electrode having further excellent conductivity and bio-compatibility.

In the present invention, the repeating unit-a preferably has one or more selected from repeating units-A5 to -A7 represented by the following general formulae (4), 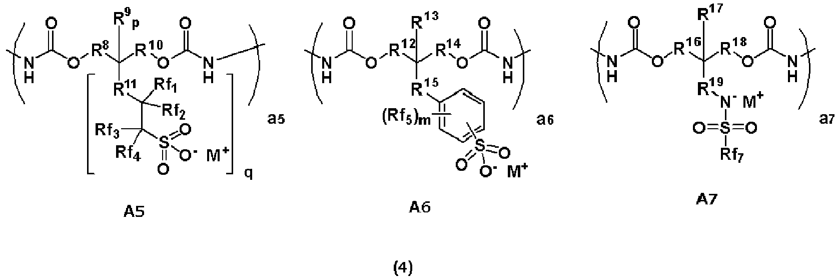 wherein R⁸, R¹⁰, R¹², R¹⁴, R¹⁶, and R¹⁸ each independently represent a single bond or a linear or branched alkylene group having 1 to 6 carbon atoms; R⁹, R¹³, and R¹⁷ each independently represent a hydrogen atom, a hydroxy group, or a linear or branched alkyl group or alkoxy group having 1 to 6 carbon atoms; R¹¹ and R¹⁹ represent a single bond or a linear, branched, or cyclic hydrocarbylene group having 1 to 20 carbon atoms and optionally having one or more selected from a carbonyl group, an ether group, and an ester group, and optionally bonded to R⁸ or R¹⁶ to form a ring; R¹⁵ represents a single bond or a linear, branched, or cyclic hydrocarbylene group having 1 to 20 carbon atoms and optionally having one or more selected from a carbonyl group, an ether group, and an ester group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₇ represents a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₇ having at least one or more fluorine atoms; "m" represents an integer of 1 to 4; a5, a6, and a7 satisfy 0≤a5≤1.0, 0≤a6≤1.0, 0≤a7≤1.0, and 0<a5+a6+a7≤1.0; M⁺ represents an ion selected from an ammonium ion, a sodium ion, a potassium ion, and a silver ion; "p" represents 0 or 1; and "q" represents 1 or 2.

The repeating unit-a having such a structure yields the bio-electrode having further excellent conductivity and bio-compatibility.

In the present invention, the repeating unit-a preferably contains an ammonium ion represented by the following general formula (3) as the ammonium ion constituting the ammonium salt, 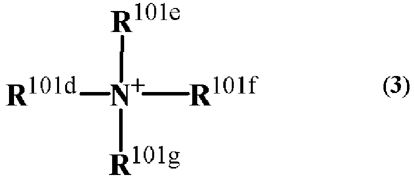 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, optionally having one or more selected from an ether group, a carbonyl group, an ester group, a hydroxy group, a carboxyl group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring together with a nitrogen atom to which these groups are bonded, and when forming the ring, R^{101d} and R^{101e}, or R^{101d}, R^{101e}, and R^{101f} form an alkylene group having 3 to 10 carbon atoms or a heteroaromatic ring having the nitrogen atom in the general formula (3) in the ring.

The bio-electrode containing the component (A) containing such an ammonium ion yields the bio-electrode having further excellent conductivity and bio-compatibility.

In the present invention, the bio-electrode preferably further comprises a resin other than the component (A) as a component (B) contained in the material of the living-body contact layer.

Containing such a component (B) is compatible with the component (A) to prevent precipitation of a salt, and can retain the adhesiveness to the substrate.

In this case, the component (B) is preferably one or more selected from a silicone resin, a (meth)acrylate resin, and a urethane resin.

Such a component (B) can further improve the adhesiveness.

In addition, the present invention provides a method for manufacturing the aforementioned bio-electrode, the method comprising a step of manufacturing the micropillar by inserting a resin containing the component (A) into a mold to be a template, and releasing the resin from the mold.

Further, the present invention provides a method for manufacturing the aforementioned bio-electrode, the method comprising a step of manufacturing the micropillar by inserting a resin containing the component (A) into a pattern of a resin to be a template, and dissolving a pattern of the resin with water or an alkaline aqueous solution to release the resin containing the component (A).

By such a method for manufacturing a bio-electrode, the bio-electrode that is a thin film and highly transparent, that has good adhesiveness to a living body, high sensitivity to a bio-signal, and excellent bio-compatibility and a lightweight property, that can be manufactured at a low cost, that does not considerably deteriorate the sensitivity to the bio-signal even when being wet, dried, or attached to the skin for a long term, and that is comfortable and free from itching, red spots, rash, etc. of the skin can be manufactured.

### ADVANTAGEOUS EFFECTS OF INVENTION

As above, the bio-electrode and method for manufacturing a bio-electrode of the present invention can provide: the bio-electrode that has good adhesiveness to a living body, high sensitivity to a bio-signal, and excellent bio-compatibility, that is a thin film, lightweight, and highly transparent, that can be manufactured at a low cost, that does not considerably deteriorate the sensitivity to the bio-signal even when being wet, dried, or attached to the skin for a long term, and that is comfortable and free from itching, red spots, rash, etc. of the skin; and a method for manufacturing a bio-electrode.

### BRIEF DESCRIPTION OF DRAWINGS

FIG. 1 is a sectional view of a mold for manufacturing a bio-electrode of the present invention.
FIG. 2 is a sectional view after a living-body contact layer of a bio-electrode of the present invention is formed on a conductive layer.
FIG. 3 is a sectional view when a mold is crimped onto a living-body contact layer of a bio-electrode of the present invention.
FIG. 4 illustrates a sectional structure of a bio-electrode of the present invention with formed micropillars after a mold is released.
FIG. 5 is an example of a sectional view of a mold for manufacturing a bio-electrode of the present invention.
FIG. 6 is an example of a sectional view when a mold is crimped onto a living-body contact layer of a bio-electrode of the present invention.
FIG. 7 illustrates an example of a sectional structure of a bio-electrode of the present invention with formed micropillars after a mold is released.
FIG. 8 is a sectional view when a substrate is released from a mold with rounding the substrate.
FIG. 9 illustrates a sectional structure when a bio-electrode of the present invention is attached to skin to measure a bio-signal.
FIG. 10 is a plane view of a mold for manufacturing a bio-electrode of the present invention.
FIG. 11 is another example of a plane view of a mold for manufacturing a bio-electrode of the present invention.
FIG. 12 is an example of a sectional view of a mold for manufacturing a bio-electrode of the present invention.
FIG. 13 illustrates an example of a sectional structure of a bio-electrode of the present invention with formed micropillars after a mold is released.
FIG. 14 is an example of a sectional view of a mold for manufacturing a bio-electrode of the present invention.
FIG. 15 illustrates an example of a sectional structure of a bio-electrode of the present invention with formed micropillars after a mold is released.
FIG. 16 is an example of a sectional view of a mold for manufacturing a bio-electrode of the present invention.
FIG. 17 illustrates an example of a sectional structure of a bio-electrode of the present invention with formed micropillars, and a mold is released.
FIG. 18 illustrates an example of a sectional structure of a mold for manufacturing a bio-electrode of the present invention.
FIG. 19 is an example of a sectional view when a mold is crimped onto an ionic polymer layer of a bio-electrode of the present invention.
FIG. 20 illustrates an example of a sectional structure of a bio-electrode of the present invention with formed micropillars, and a mold is released.
FIG. 21 is an example of a sectional view of a mold for manufacturing a bio-electrode of the present invention.
FIG. 22 is an example of a sectional view when a mold is crimped onto an ionic polymer layer of a bio-electrode of the present invention.
FIG. 23 illustrates an example of a sectional structure of a bio-electrode of the present invention with formed micropillars, and a mold is released.
FIG. 24 illustrates a sectional structure after a soluble layer is formed on a substrate and a resist layer is formed thereon.
FIG. 25 illustrates a sectional structure of a resist layer and a soluble layer after exposure and development.
FIG. 26 illustrates a sectional structure after a living-body contact layer is formed on a pattern of a resist layer and a soluble layer.
FIG. 27 illustrates a sectional structure after a substrate with a formed conductive layer adheres to a living-body contact layer, and the substrate is released.
FIG. 28 illustrates a sectional structure after a pattern of a resist layer and a soluble layer is released and top and bottom are inversed.
FIG. 29 illustrates a sectional structure of a substrate in which conductive pillars are formed on a conductive layer.
FIG. 30 is a sectional view in which top and bottom of a substrate are inversed, and a conductive layer and conductive pillars are immersed in an ionic-polymer-containing solution.
FIG. 31 is a sectional view in which a substrate is moved above an ionic-polymer-containing solution and dried.
FIG. 32 is a plane view after a conductive layer is printed on a substrate.
FIG. 33 is a plane view after a living-body contact layer is printed on a conductive layer.
FIG. 34 is a plane view after micropillars are formed on a living-body contact layer.
FIG. 35 is a view illustrating a position where a bio-electrode of the present invention is attached for obtaining a bio-signal.

### DESCRIPTION OF EMBODIMENTS

As noted above, there has been a demand for development of a bio-electrode that has high conductivity and excellent bio-compatibility to exhibit highly sensitive bio-signal with low noise, that is highly transparent and a lightweight thin film, that can be manufactured at a low cost, that can measure a bio-signal even when being wet and dried, that is free from skin roughness and itching even when attached to the skin for a long term, that has adhesiveness even when repeatedly attached to and released from the skin, that does not deteriorate adhesiveness even when attached to the skin for a long term, that allows the skin to breathe even when attached to the skin for a long term, that is comfortable and free from stuffiness even when the skin perspires, and that is lightweight and a thin film; a method for manufacturing the same; and a method for measuring a bio-signal.

A gecko can ascend and descend on a smooth and slippery vertical window glass. Gecko's pads do not adhere to the windows glass by sucking or adhesion, but have numerous fine cilia with a spoon-shaped tip on the soles, as described in Non Patent Document 2. The weight is supported by a Van der Waals force between these cilia and the glass surface.

A force in the vertical direction relative to the window glass is weak, and the gecko pulls the pads in the vertical direction when released. Since a force in the horizontal direction is strong, the gecko does not fall from the vertical window glass on which the load is applied in the horizontal direction. Although a strongly adhesive tape requires a large force when released, the gecko's pads can be easily released with a relatively weak force only by changing the releasing direction.

For use of the bio-electrode that is attached to and released from the skin, a strongly adhesive bio-electrode requiring a strong force for release has a problem of pain of the skin with releasing. Meanwhile, the strongly adhesive characteristics are required for continuous attaching to the skin for a long term. To break the trade-off relationship between the strong adhesiveness and light release, the adhesion mechanism of the gecko's pads is effective.

Sodium, potassium, and calcium ions are emitted from the skin surface in conjunction with a heartbeat. The bio-electrode needs to covert an increase and decrease of ions emitted from the skin into an electric signal. Thus, a material that has excellent ion conductivity for transmitting the increase and decrease of ions is required. A potential of the skin surface also fluctuates in conjunction with the heartbeat. This fluctuation of the potential is subtle, and electron conductivity for transmitting a small current to a device is also required.

A hydrophilic gel containing sodium chloride or potassium chloride has high ion conductivity, but loses the conductivity when dried. In addition, elution of the sodium chloride or the potassium chloride to the outside of the bio-electrode by bathing or showering also decreases conductivity.

A bio-electrode using a metal such as gold and silver detects only a small current and has low ion conductivity, and thus has low sensitivity as the bio-electrode. Carbon, which also has electron conductivity similarly to the metal, has lower electron conductivity than the metal, and has lower sensitivity than the metal as the bio-electrode.

A bio-electrode containing a gecko's pad-shaped conductive filler described in Non Patent Document 3 has an adhesive force but low sensitivity as the bio-electrode.

A fluorosulfonic acid, a fluorosulfonimide, and a fluorosulfonamide have high polarity and high ion conductivity. Combination of a polymer thereof and a conductive filler can exhibit both the high ion conductivity and electron conductivity.

A gecko's pad-shaped bio-electrode containing the polymer containing one or more selected from a fluorosulfonic acid, a fluorosulfonimide, and a fluorosulfonamide can yield both a sufficient adhesive force and high sensitivity as the bio-electrode.

The present inventors have earnestly studied the aforementioned problem as above, and consequently found a bio-electrode having the following configuration and a method for manufacturing a bio-electrode. These findings have led to completion of the present invention.

Specifically, the present invention is a bio-electrode, comprising:
a living-body contact layer; and
a substrate, wherein
the living-body contact layer has a micropillar having a diameter within a range of 0.01 to 500 um and a height within a range of 0.1 to 1,000 um, and has an ionic polymer (A) as a material, and
the component (A) has a repeating unit-a of one or more selected from a fluorosulfonic acid, a fluorosulfonimide, and a fluorosulfonamide, and has a weight-average molecular weight within a range of 1,000 to 500,000.

Hereinafter, the present invention will be described in detail, but the present invention is not limited thereto.

### <Bio-Electrode>

A bio-electrode of the present invention is a bio-electrode including:
a living-body contact layer; and
a substrate, wherein
the living-body contact layer has a micropillar having a diameter within a range of 0.01 to 500 um and a height within a range of 0.1 to 1,000 um, and has an ionic polymer (A) as a material, and
the component (A) has a repeating unit-a of one or more selected from a fluorosulfonic acid, a fluorosulfonimide, and a fluorosulfonamide, and has a weight-average molecular weight within a range of 1,000 to 500,000.

The diameter of the micropillar is within a range of 0.01 to 500 µm, preferably within a range of 0.05 to 300 µm, and more preferably within a range of 0.08 to 200 um. The height of the micropillar is within a range of 0.1 to 1,000 µm, preferably within a range of 0.2 to 800 um, and more preferably within a range of 0.3 to 500 µm.

The bio-electrode of the present invention can measure not only electrocardiogram but also electromyogram, brain waves, and breathing rate. The bio-electrode can not only measure a signal emitted from the skin but also provide an electric signal to the skin to transmit a signal to muscles and control brain waves. For example, the bio-electrode is expected to be used for purposes of applying a stimulus to muscles during swimming to increase performance or reduce fatigue, and of enhancing relaxation during bathing.

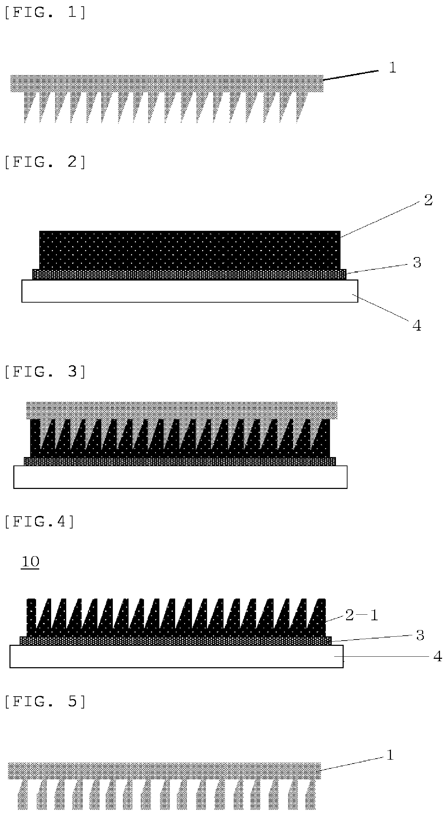

The simple description will be made by using the figures. FIG. 4 illustrates a cross section of an example of the bio-electrode of the present invention. A bio-electrode 10 of the present invention has a living-body contact layer 2-1, a conductive layer 3, and a substrate 4, for example.

FIGS. 1 to 4 illustrate a method for manufacturing a micropillar-type bio-electrode using a mold. In the sectional view of a mold 1 in FIG. 1, the tip tapers off. FIG. 2 is a sectional view in which the conductive layer 3 is formed on the substrate 4, and the living-body contact layer 2 is formed thereon. FIG. 3 is a sectional view in which the mold is pressed against the living-body contact layer. The living-body contact layer may have a functional group crosslinkable by a radical, an acid, or a base to be cured by light irradiation or heating. Alternatively, the living-body contact layer may be thermoplastic. In this case, the substrate or the mold may be heated in order to soften the ionic polymer to enhance filling the mold. In a case of the photocurable living-body contact layer, the mold is necessarily transparent.

To eliminate a gap between the micropillar and the mold, the molding may be performed under a reduced-pressure environment. The mold is released to form the bio-electrode 10 with the formed micropillar structure, illustrated in FIG. 4. To perform smooth release from the mold, a releasing agent may be applied on the mold in advance. As the releasing agent, a silicone-based or fluorine-based material may be used.

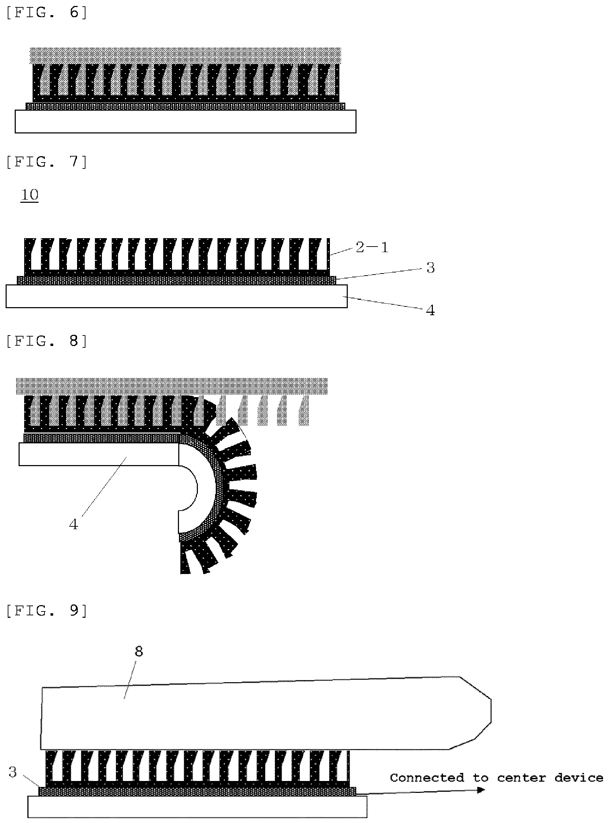

The micropillar illustrated in FIG. 4 has a pointed tip, and a small contacting area with the skin, which may deteriorate the sensitivity of the bio-signal. To increase the contacting area with the skin, for example, the mold 1 illustrated in FIG. 5 is used, and crimping onto the living-body contact layer is performed as illustrated in FIG. 6 to form the bio-electrode 10 as illustrated in FIG. 7 having the living-body contact layer 2-1 having the mushroom shape and a large contacting area with the skin surface, the conductive layer 3, and the substrate 4.

As illustrated in FIG. 8, curving the substrate 4 widens an opening of the micropillar to facilitate the releasing.

FIG. 9 is a sectional view in which the bio-electrode is contacted with skin 8 to receive a bio-signal. The conductive layer 3 is electrically connected to a device in order to analyze the bio-signal. A gap between the micropillars allows the skin to breathe, and since the micropillar absorbs sweat if present, no gap is generated between the bio-electrode and the skin.

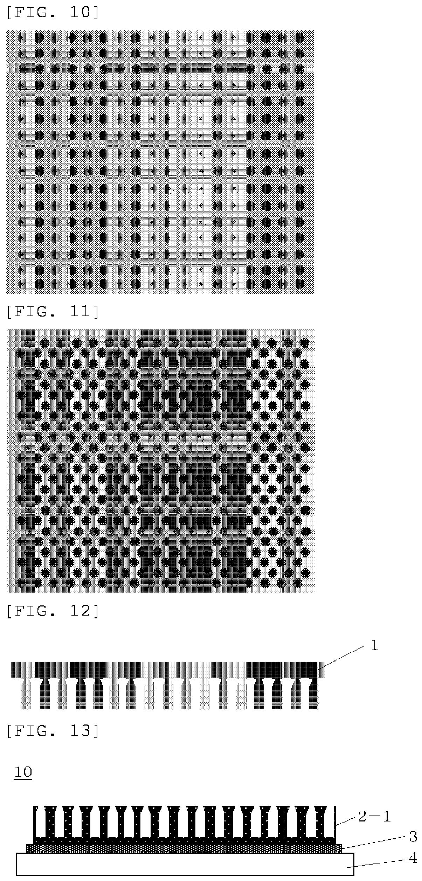

FIG. 10 is a plane view of the mold. A black portion is a concave portion, and the living-body contact layer is inserted thereinto to form the micropillar. FIG. 10 illustrates a hole layout in vertical, and FIG. 11 illustrates a hole layout in an oblique direction. The layout in FIG. 11 has a larger number of holes per unit area, which can form micropillars at a high density. The arrangement of the micropillars may not be the regular arrangement as described in FIG. 10 or FIG. 11.

FIG. 12 illustrates a case of the mold 1 with both tapered sides. This case can form, as illustrated in FIG. 13, the bio-electrode 10 having the living-body contact layer 2-1 having mushroom-shaped micropillars, the conductive layer 3, and the substrate 4. Although a stress tends to be applied at the tip of the micropillar in releasing the mold, the stress in the release is reduced by a releasing method illustrated in FIG. 8.

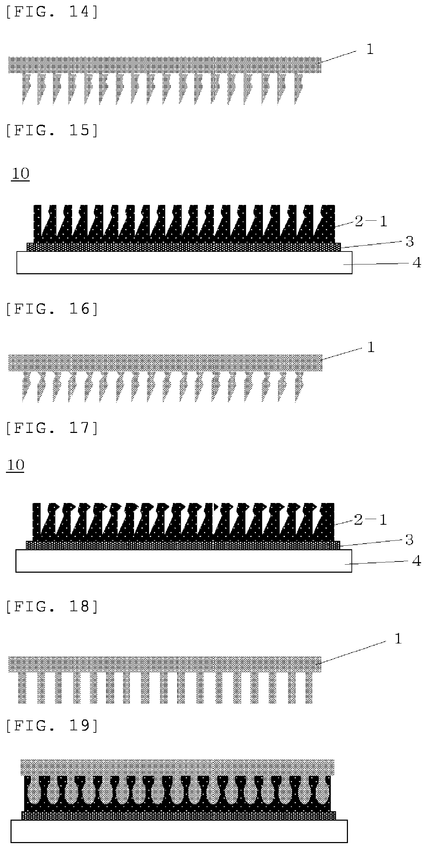

The mold 1 illustrated in FIG. 14 has the pointed tip and thereby is easily inserted into the living-body contact layer, resulting in an advantage of a low stress in releasing the mold. This case can form the bio-electrode 10 as illustrated in FIG. 15 having the living-body contact layer 2-1 having the micropillar, the conductive layer 3, and the substrate 4.

The mold 1 illustrated in FIG. 16 has a pointed tip and an unsymmetrical shape, and thereby disperses and reduces the stress in the micropillar in releasing the mold to have an advantage of further easiness of releasing. This case can form the bio-electrode 10 as illustrated in FIG. 17 having the living-body contact layer 2-1 having the micropillar, the conductive layer 3, and the substrate 4.

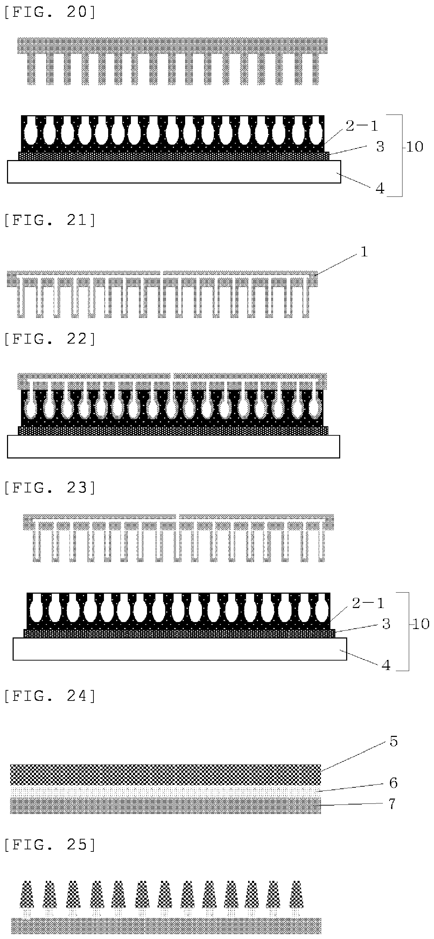

The mold 1 illustrated in FIG. 18 is deformed in compressing against the living-body contact layer in FIG. 19 and recovered to the original shape in releasing to improve the releasing ability. A soft metal having stress deformability and a structure deformable by a stress may be used. This case can form the bio-electrode 10 as illustrated in FIG. 20 having the living-body contact layer 2-1 having the micropillar, the conductive layer 3, and the substrate 4.

In the mold 1 illustrated in FIG. 21, compressed air is introduced toward the tip of the mold to deform the tip of the mold in compressing against the living-body contact layer in FIG. 22. In releasing, a pressure of the air is reduced to recover the shape to facilitate the releasing. This case can form the bio-electrode 10 as illustrated in FIG. 23 having the living-body contact layer 2-1 having the micropillar, the conductive layer 3, and the substrate 4.

FIGS. 24 to 28 illustrate micropillar formation by using lithography. In FIG. 24, a soluble layer 6 and a resist layer 5 are formed on a substrate 7. The soluble layer 6 is a material having no photosensitivity but higher solubility in a developer than the resist layer 5. The resist is applied on the soluble layer 6. Since the resist layer 5 and the soluble layer 6 are required not to cause mixing, the soluble layer 6 is preferably an aqueous-solution-based material and the resist layer 5 is preferably an organic-solvent-based material. For the resist layer 5, a chemically amplified resist may be used. After light exposure, baking (PEB) is performed, and development is performed. For the developer, an alkali aqueous solution may be used. FIG. 25 is a sectional view of the pattern after the development. The soluble layer 6 exhibits a dissolution rate in the developer higher than that of the resist layer 5, which yields an undercut shape.

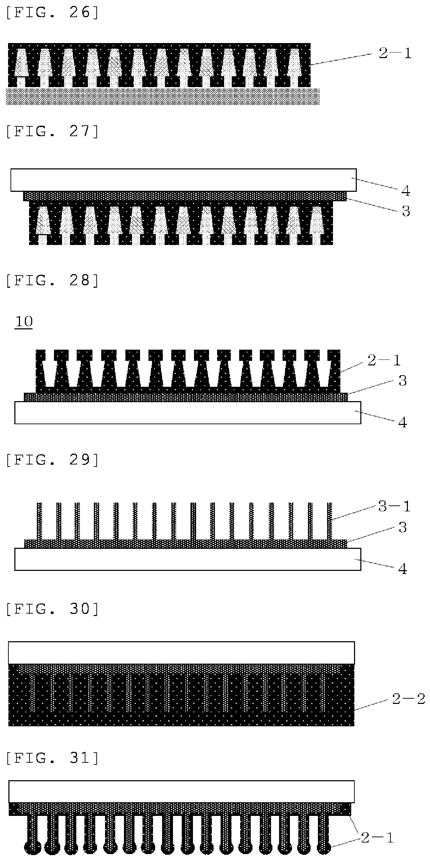

The pattern after the development in FIG. 25 is entirely exposed, and PEB is performed to change the polarity of the resist layer to be hydrophilic, and reduces solubility in an organic solvent. Accordingly, the resist pattern can be retained without dissolution when the ionic-polymer-containing solution is applied thereon to form the living-body contact layer 2-1 in FIG. 26.

The ionic polymer solution is applied, baking is performed, then the substrate is released or the substrate 4 on which the conductive layer 3 is applied illustrated in FIG. 27 adheres before releasing the substrate, and the resist layer and the pattern of the soluble layer are released to form the bio-electrode 10 as illustrated in FIG. 28 having the living-body contact layer 2-1 having the micropillar, the conductive layer 3, and the substrate 4.

FIGS. 29 to 31 illustrate a method for applying the living-body contact layer on the substrate on which the conductive layer with the conductive pillar is formed.

A conductive pillar 3-1 on the conductive layer 3 applied on the substrate 4 illustrated in FIG. 29 is immersed in an ionic-polymer-containing solution 2-2 as illustrated in FIG. 30, and pulled up as illustrated in FIG. 31 and dried to form the living-body contact layer 2-1.

### <Material for Living-Body Contact Layer>

Hereinafter, each component to form the living-body contact layer of the bio-electrode of the present invention will be described in more detail.

### (A) Ionic Polymer (Salt)

The ionic polymer (A) used for the bio-electrode of the present invention has one or more repeating units-a selected from a fluorosulfonic acid, a fluorosulfonimide, and a fluorosulfonamide, and has a weight-average molecular weight within a range of 1,000 to 500,000. As the ionic material of (A), a polymer having an ionic repeating unit selected from an ammonium salt, a sodium salt, a potassium salt, and a silver salt of any one of the fluorosulfonic acid, the fluorosulfonimide, and the N-carbonylfluorosulfonamide may be contained.

### (Repeating Unit-a)

The repeating unit-a may have a partial structure represented by the following general formulae (1)-1 to (1)-4.

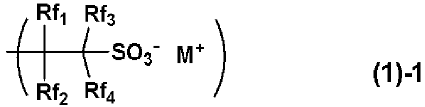

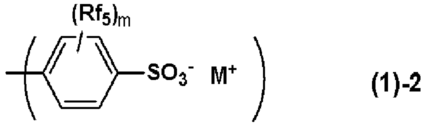

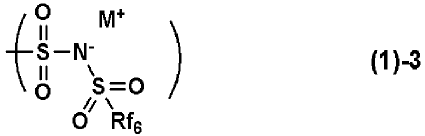

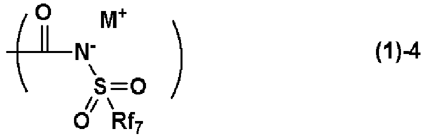

In the formulae, Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; M⁺ represents an ion selected from an ammonium ion, a sodium ion, a potassium ion, and a silver ion; and "m" represents an integer of 1 to 4.

The repeating unit-a preferably has one or more selected from repeating units-A1 to -A4 represented by the following general formulae (2).

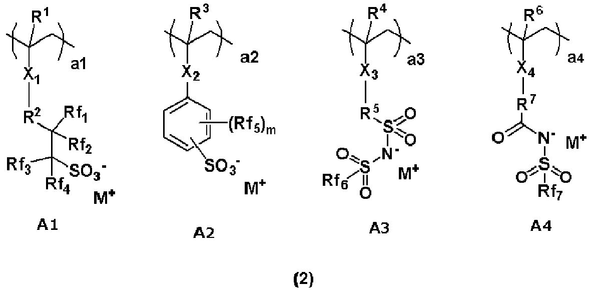

In the general formulae (2), R¹, R³, R⁴, and R⁶ each independently represent a hydrogen atom or a methyl group; R², R⁵, and R⁷ each independently represent a single bond or a linear, branched, or cyclic hydrocarbon group having 1 to 13 carbon atoms and optionally having an ester group, an ether group, or both of them; X₁, X₂, X₃, and X₄ each independently represent any one of a single bond, a phenylene group, a naphthylene group, an ether group, an ester group, and an amide group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; "m" represents an integer of 1 to 4; a1, a2, a3, and a4 satisfy 0≤a1≤1.0, 0≤a2≤1.0, 0≤a3≤1.0, 0≤a4≤1.0, and 0<a1+a2+a3+a4≤1.0; and M⁺ represents an ion selected from an ammonium ion, a sodium ion, a potassium ion, and a silver ion.

In the general formulae (2), a1 to a4 represent proportions of the repeating units-A1 to -A4, respectively.

The repeating unit-a preferably has one or more selected from repeating units-A5 to -A7 represented by the following general formulae (4).

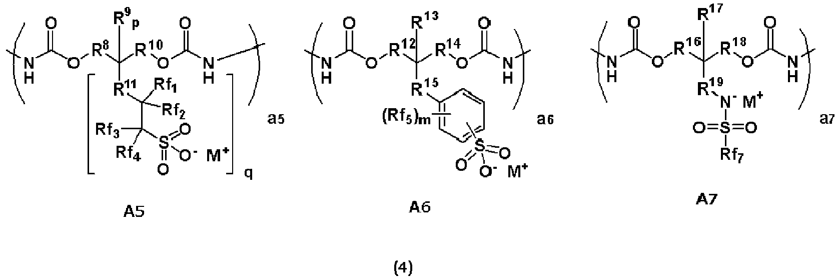

In the general formulae (4), R⁸, R¹⁰, R¹², R¹⁴, R¹⁶, and R¹⁸ each independently represent a single bond or a linear or branched alkylene group having 1 to 6 carbon atoms; R⁹, R¹³, and R¹⁷ each independently represent a hydrogen atom, a hydroxy group, or a linear or branched alkyl group or alkoxy group having 1 to 6 carbon atoms; R¹¹ and R¹⁹ represent a single bond or a linear, branched, or cyclic hydrocarbylene group having 1 to 20 carbon atoms and optionally having one or more selected from a carbonyl group, an ether group, and an ester group, and optionally bonded to R⁸ or R¹⁶ to form a ring; R¹⁵ represents a single bond or a linear, branched, or cyclic hydrocarbylene group having 1 to 20 carbon atoms and optionally having one or more selected from a carbonyl group, an ether group, and an ester group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₇ represents a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₇ having at least one or more fluorine atoms; "m" represents an integer of 1 to 4; a5, a6, and a7 satisfy 0≤a5≤1.0, 0≤a6≤1.0, 0≤a7≤1.0, and 0<a5+a6+a7≤1.0; M⁺ represents an ion selected from an ammonium ion, a sodium ion, a potassium ion, and a silver ion; "p" represents 0 or 1; and "q" represents 1 or 2.

In the general formulae (4), a5 to a7 represent proportions of the repeating units-A5 to -A7, respectively.

### (Repeating Units-A1 to -A4)

Among the repeating units-A1 to -A4 represented by the general formulae (2), specific examples of a fluorosulfonate salt monomer for obtaining the repeating unit-A1 or -A2 include the following monomers.

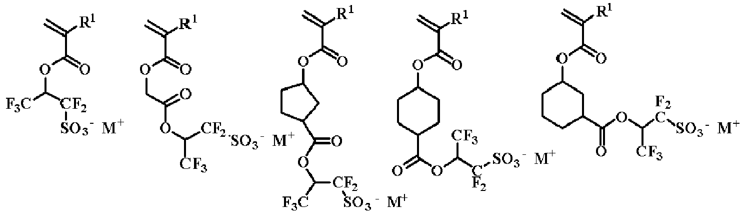

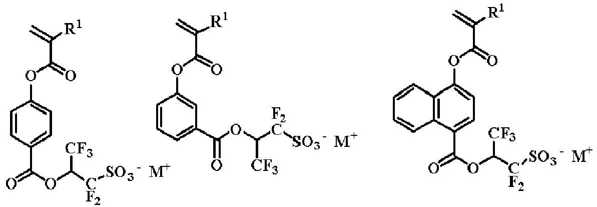

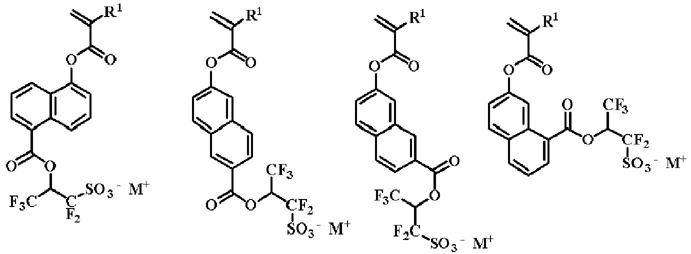

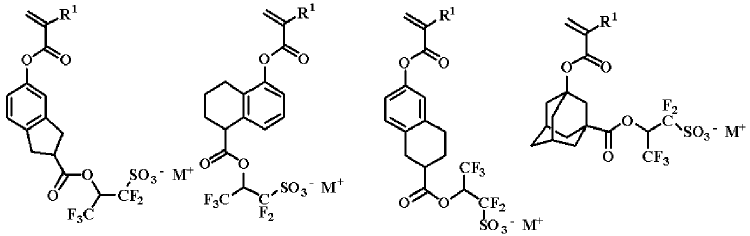

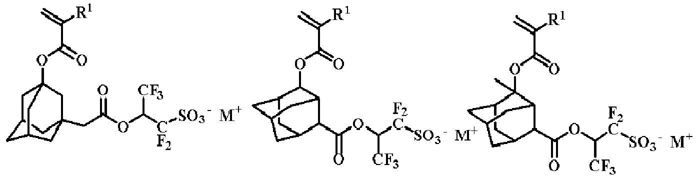

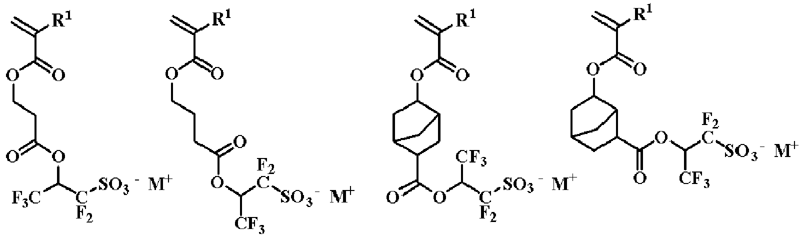

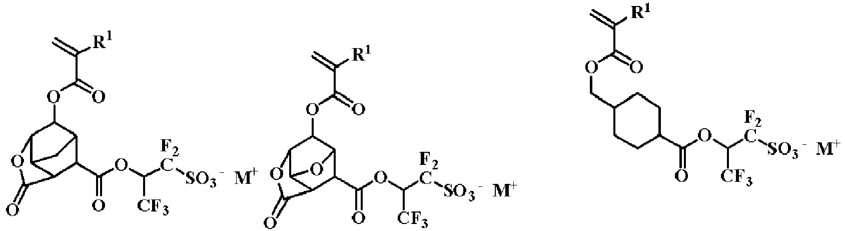

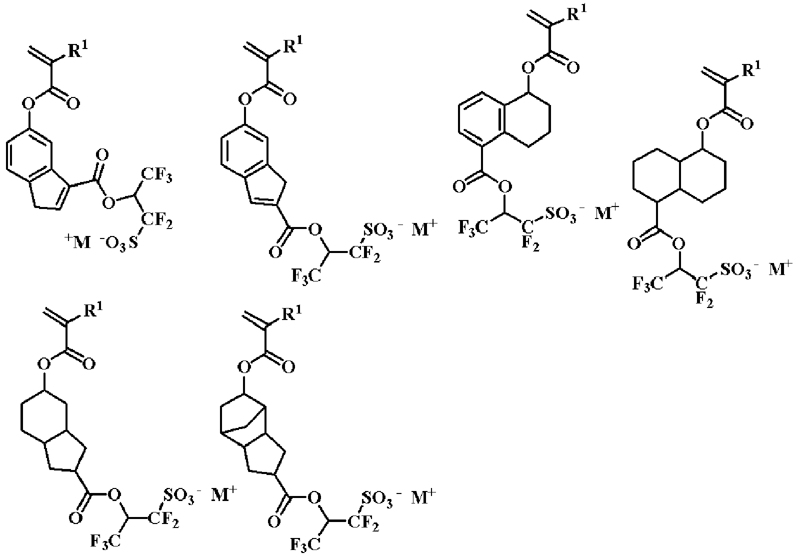

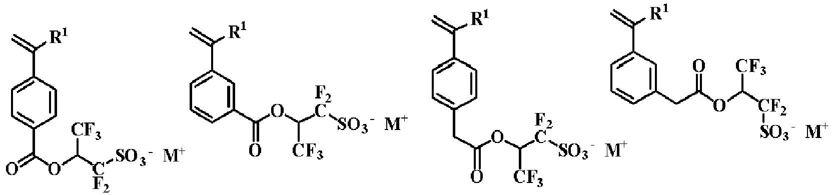

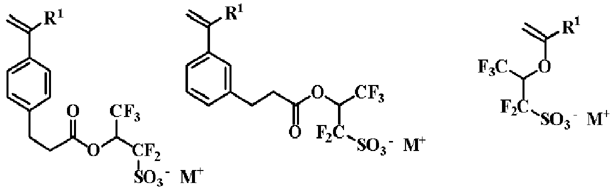

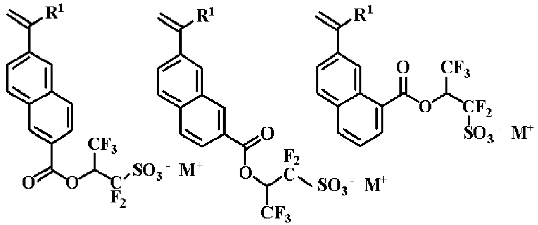

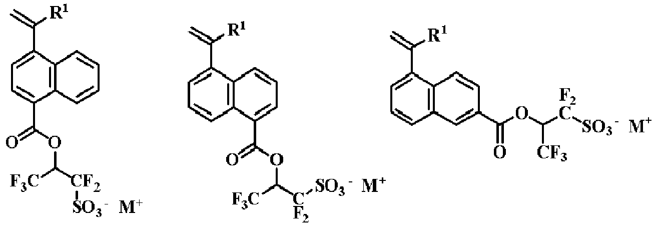

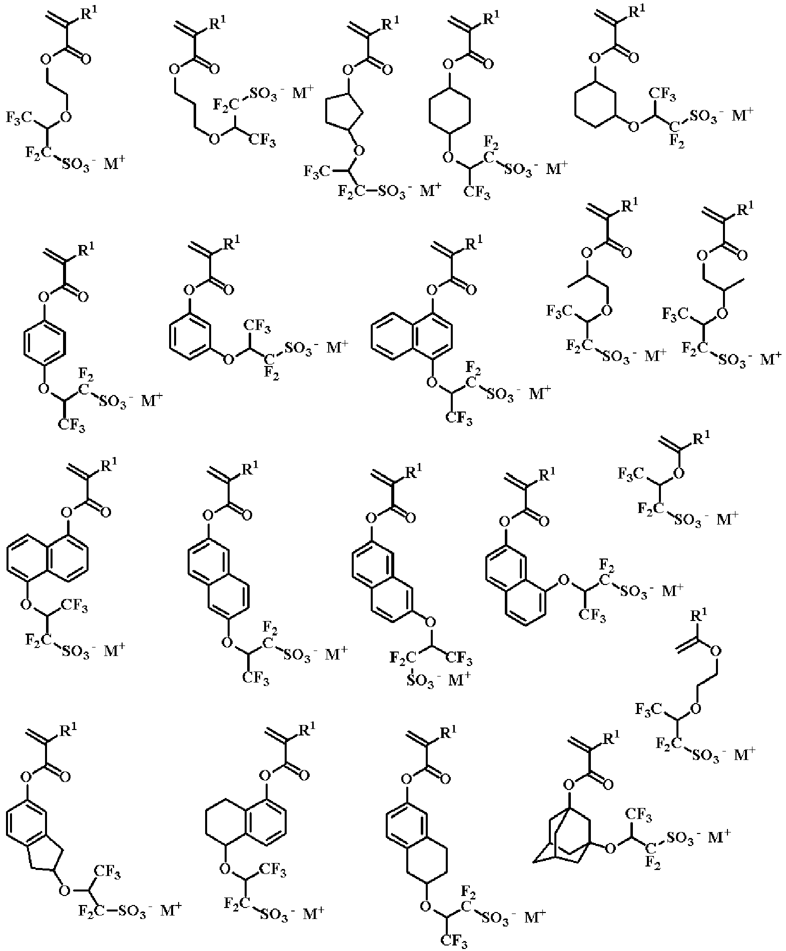

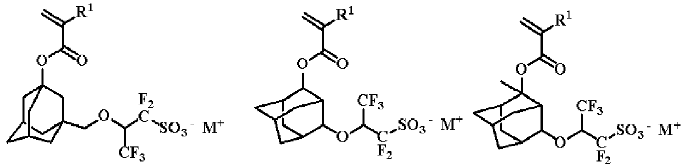

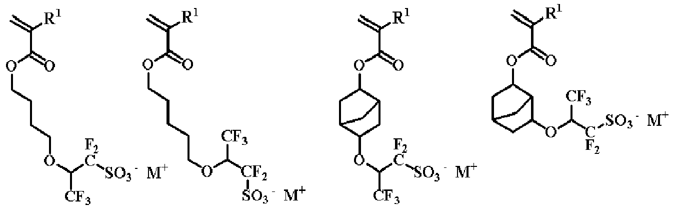

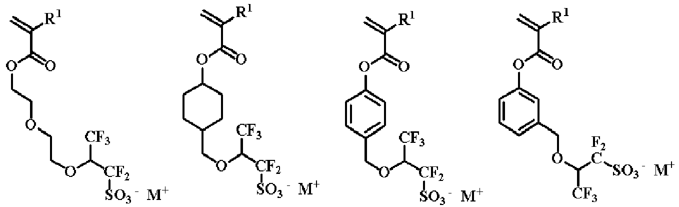

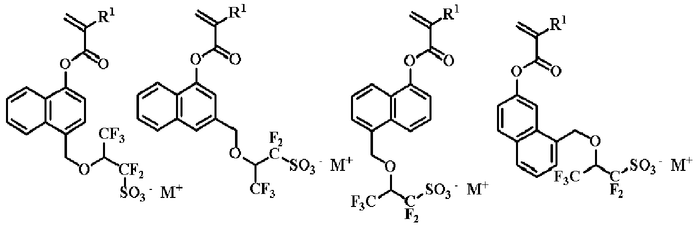

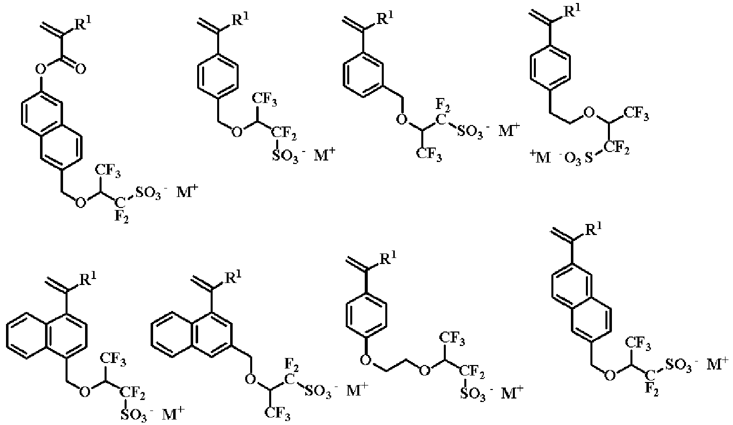

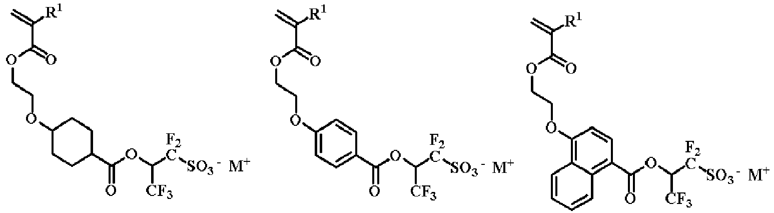

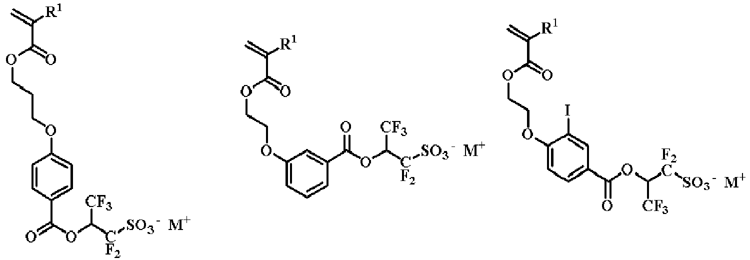

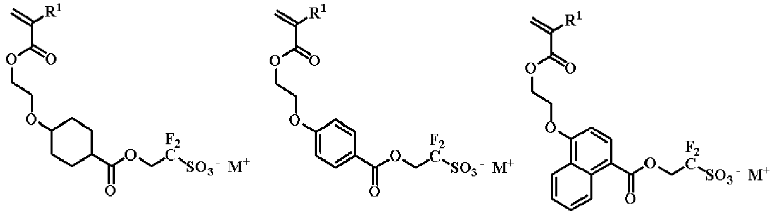

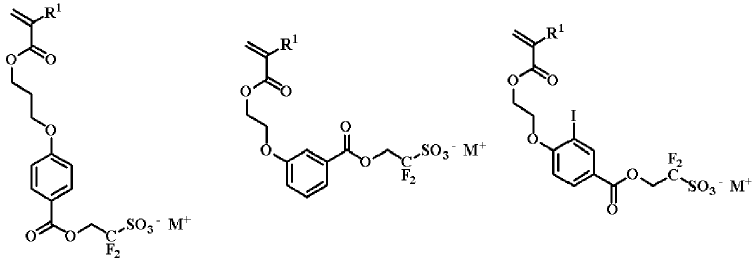

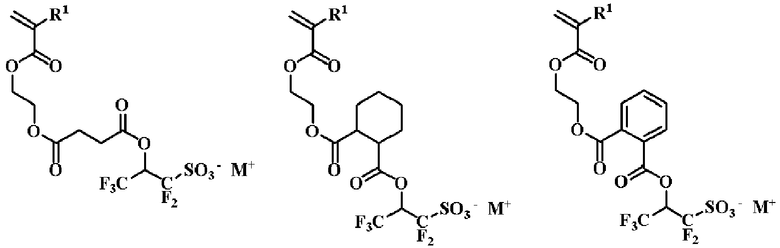

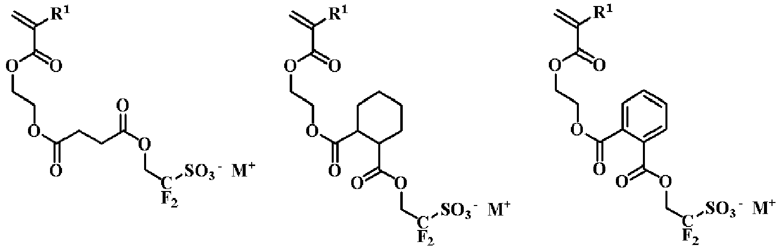

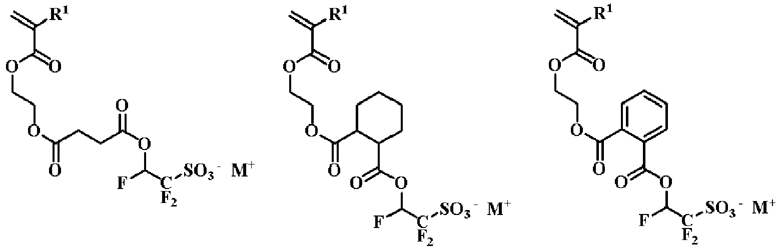

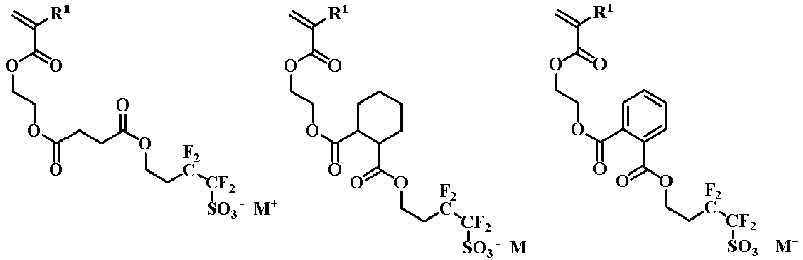

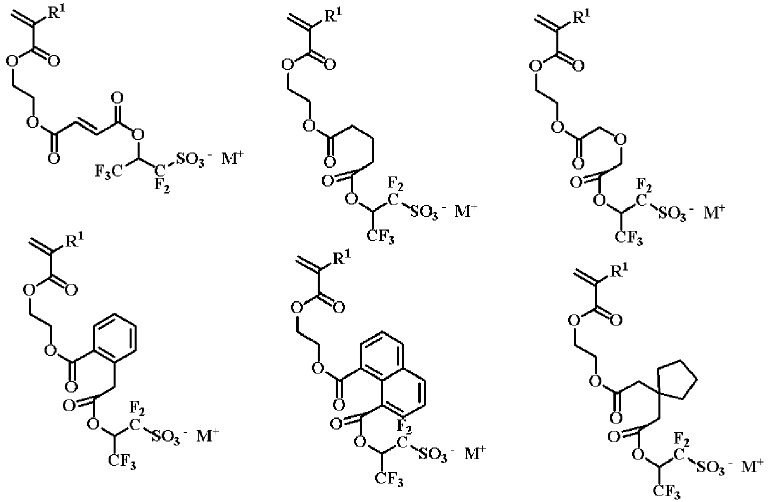

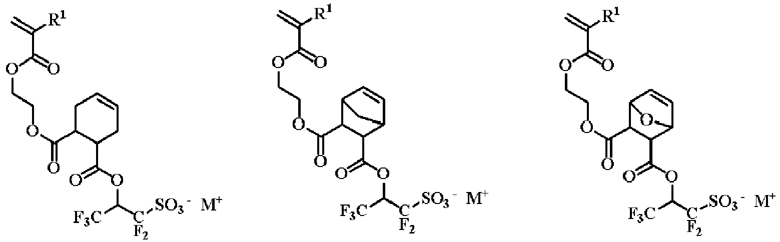

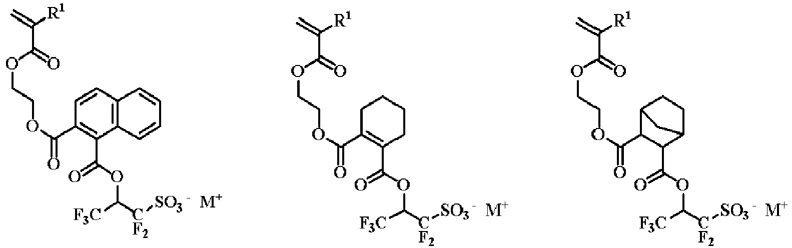

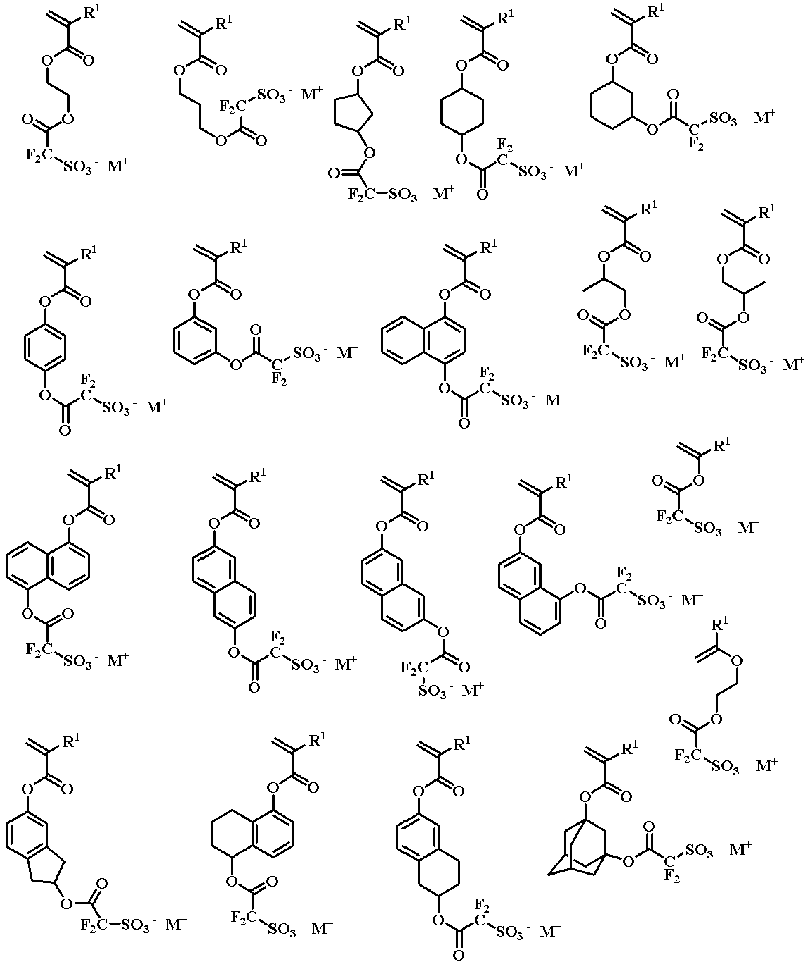

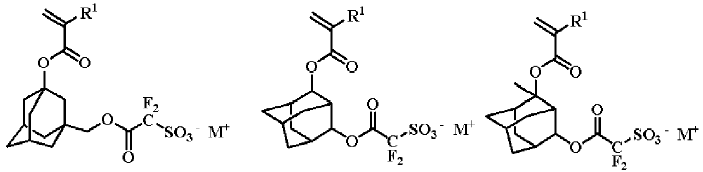

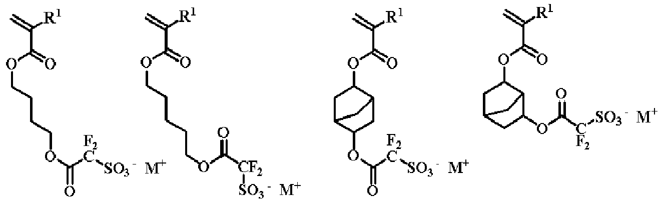

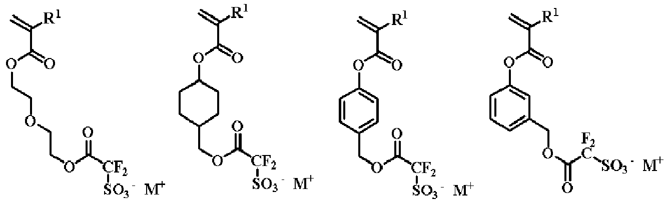

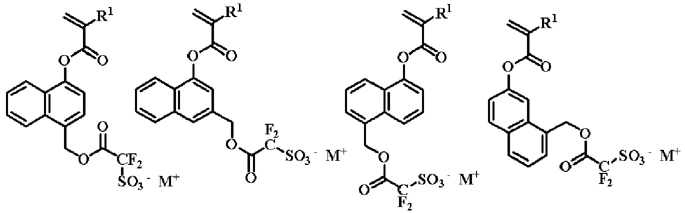

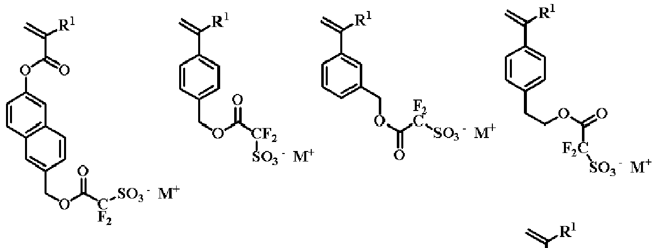

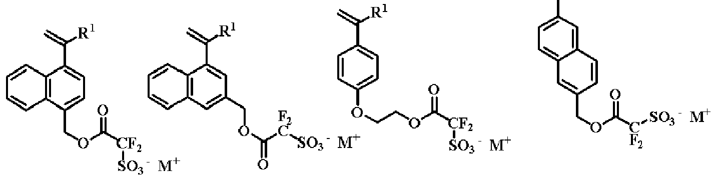

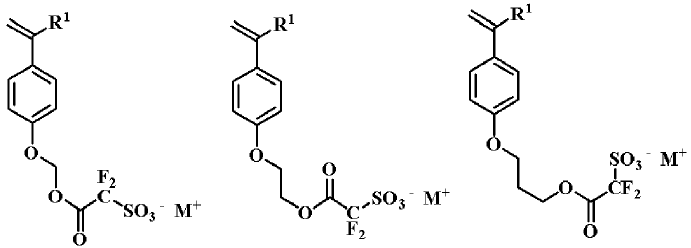

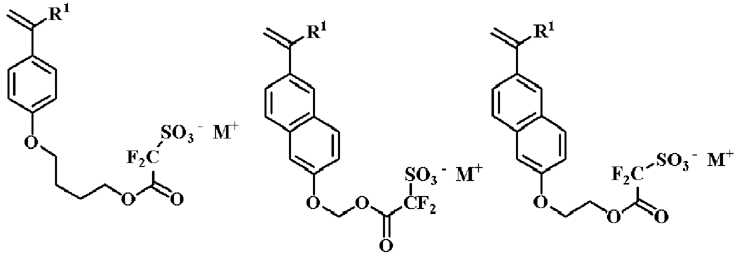

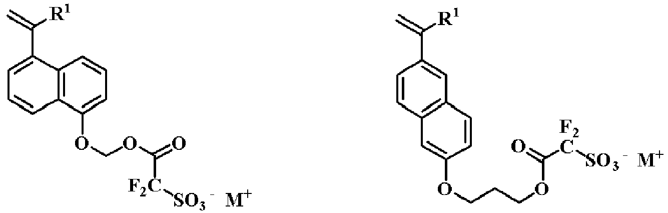

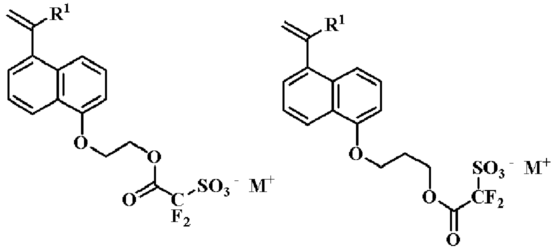

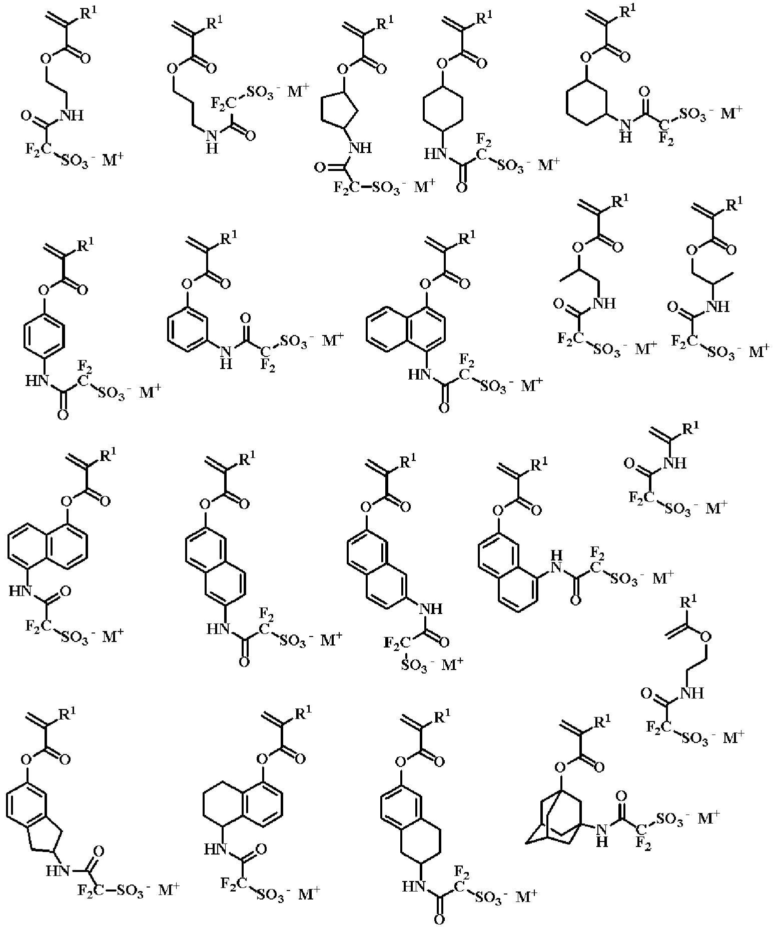

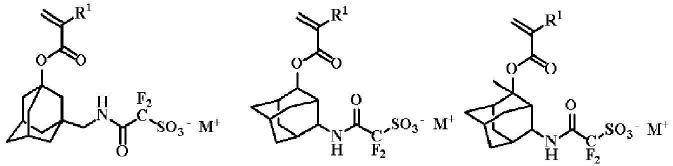

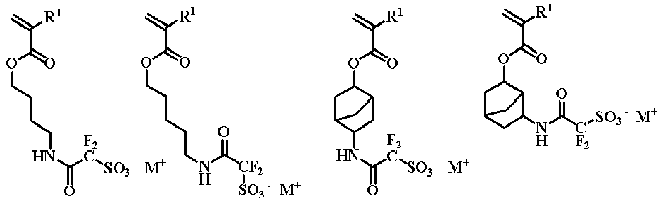

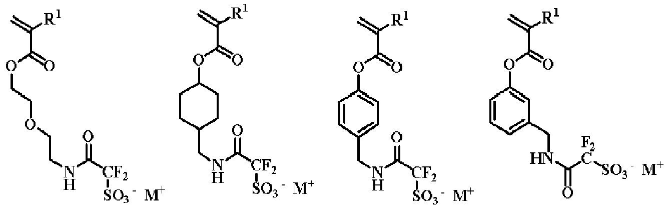

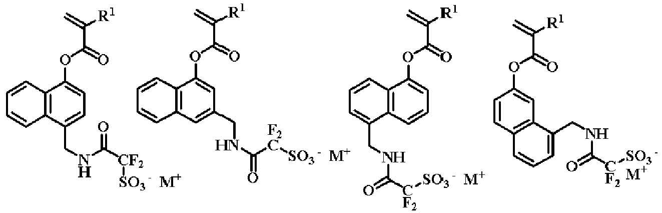

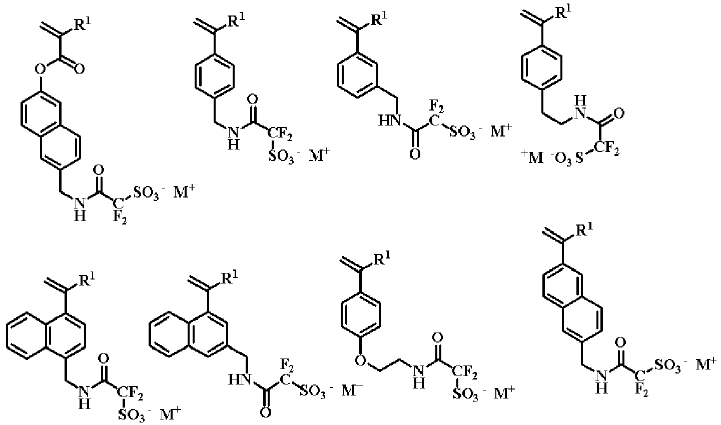

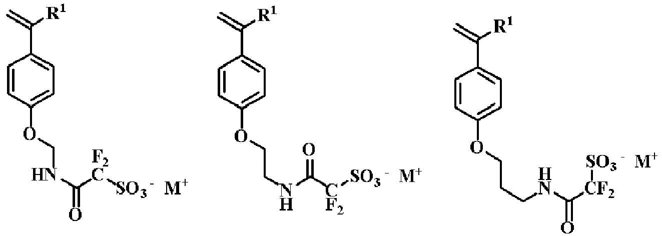

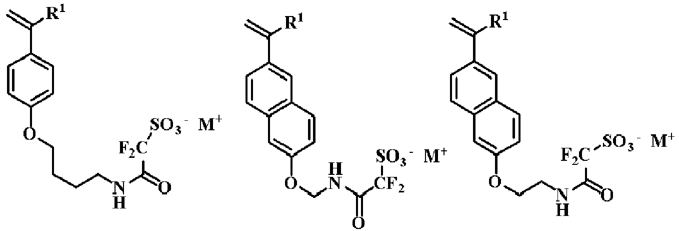

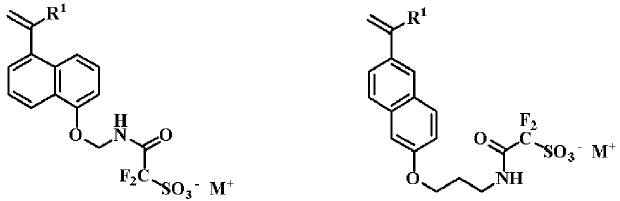

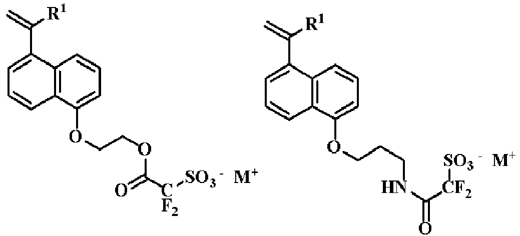

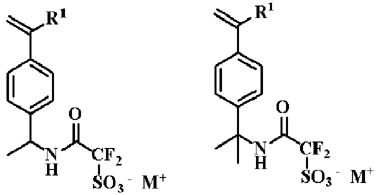

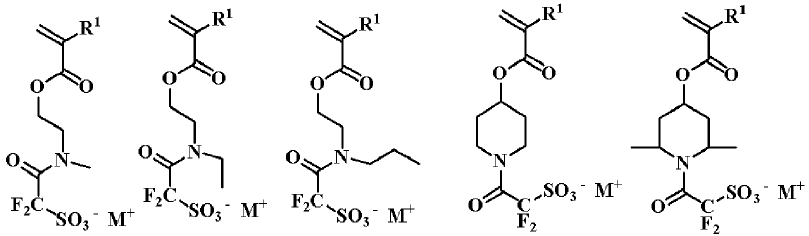

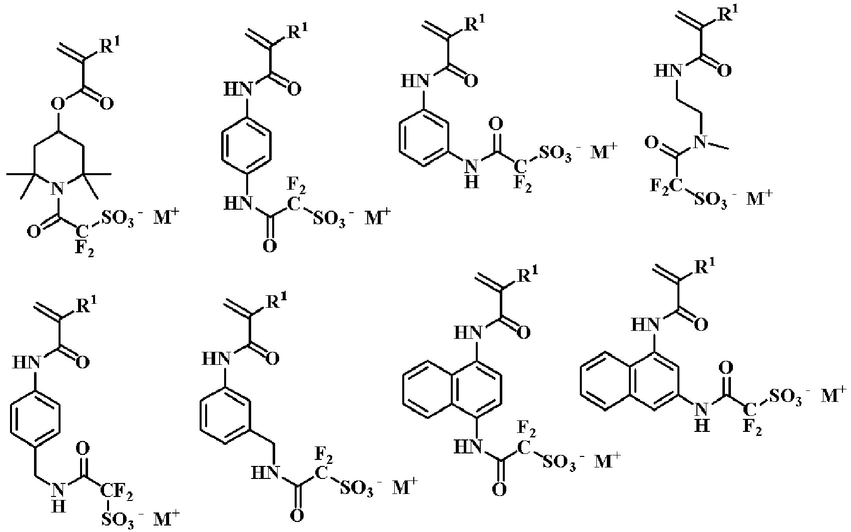

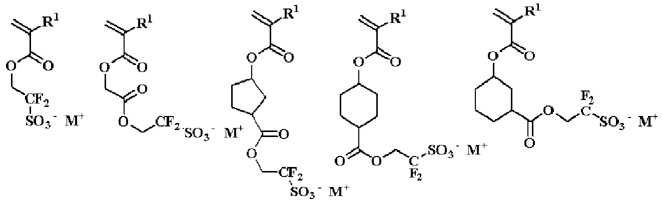

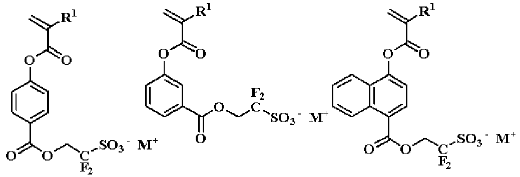

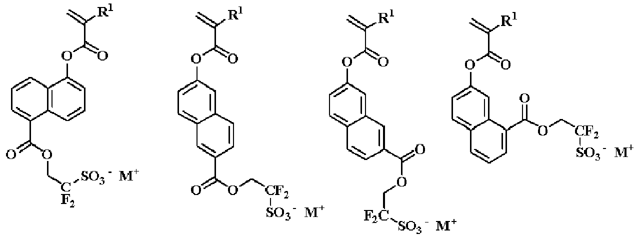

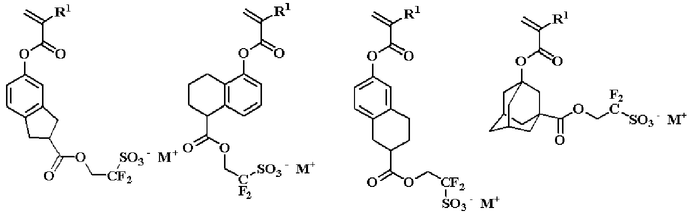

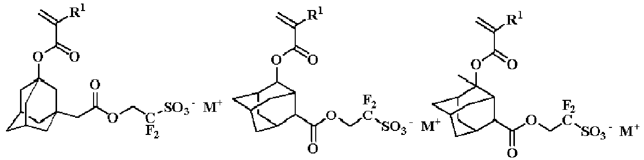

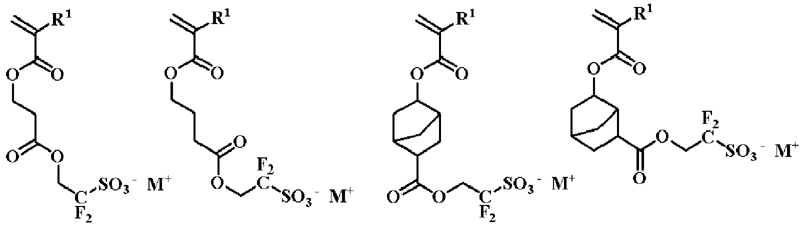

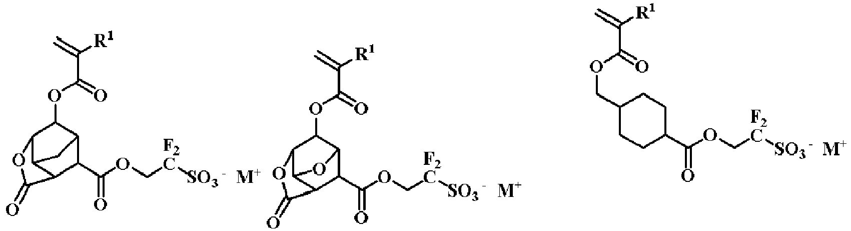

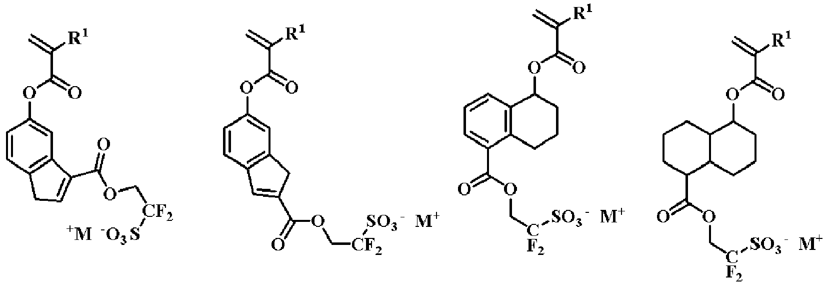

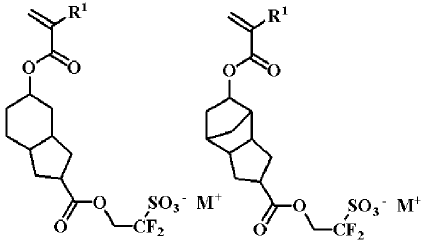

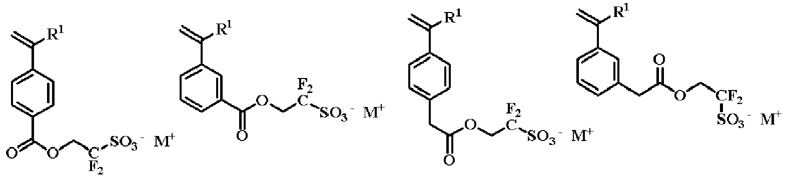

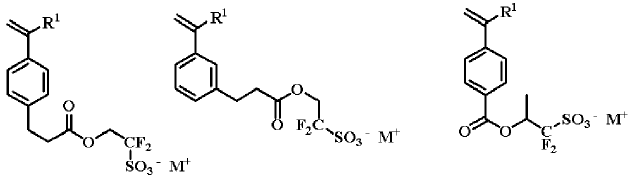

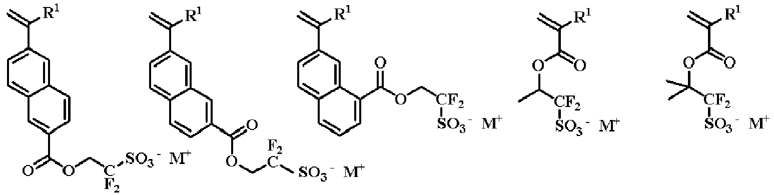

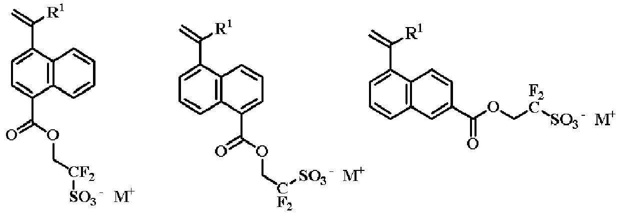

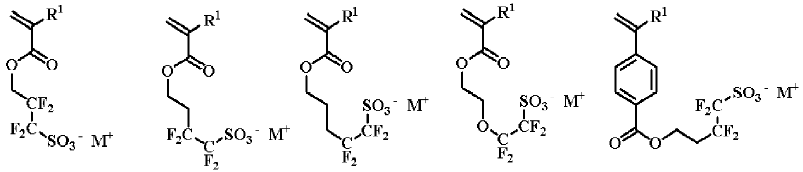

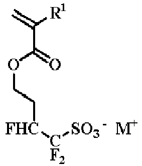

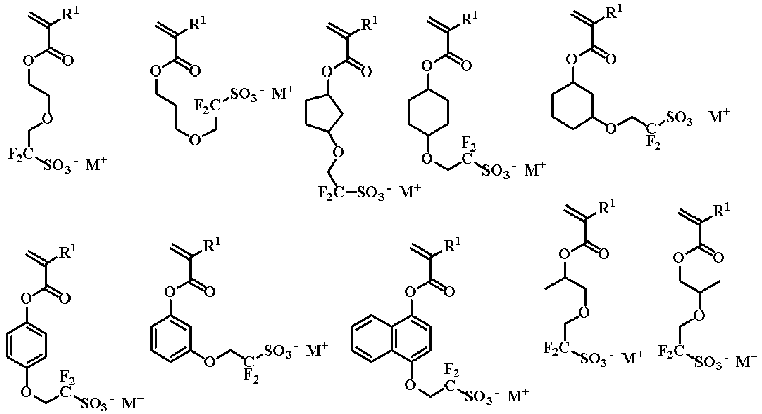

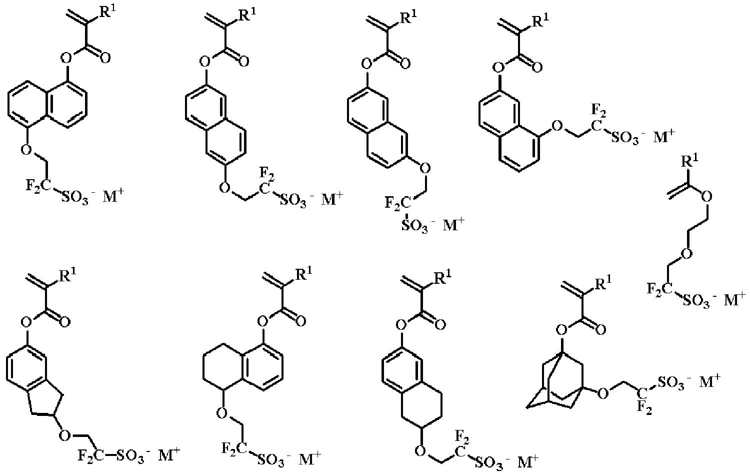

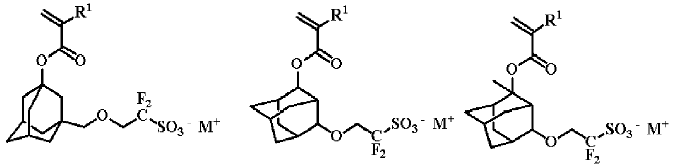

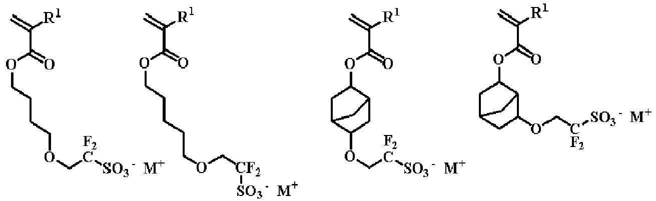

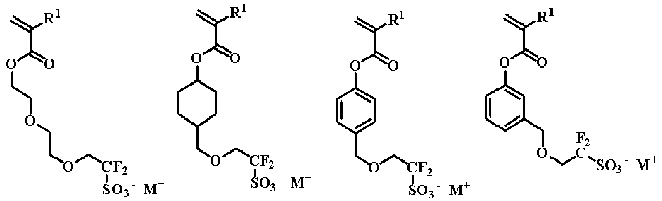

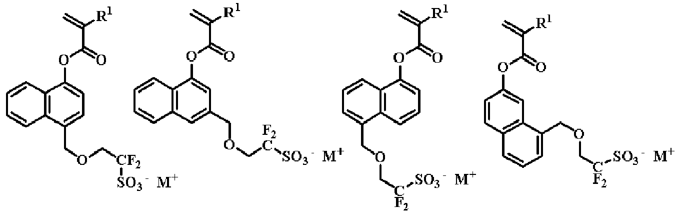

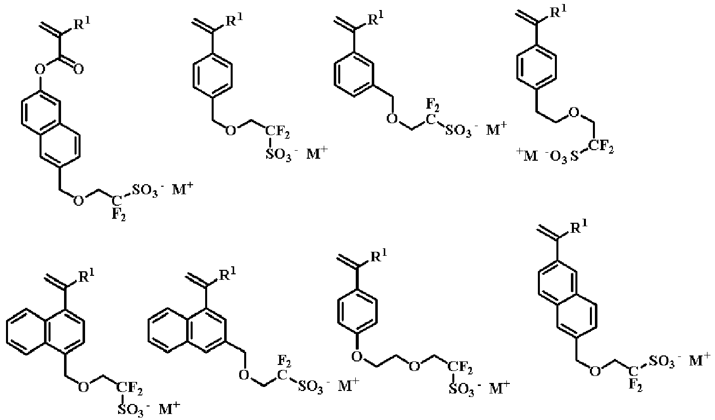

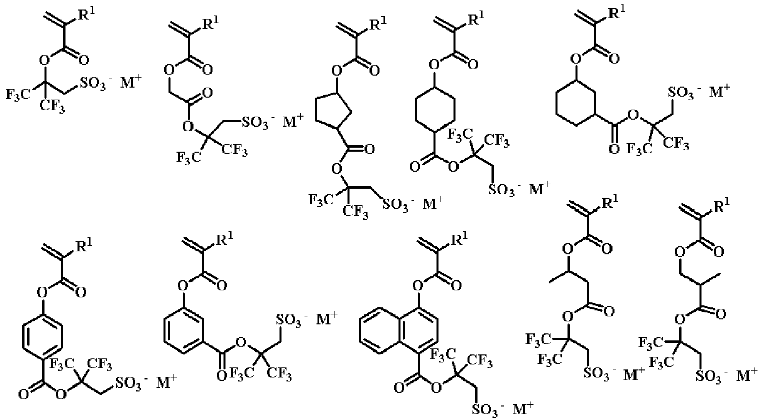

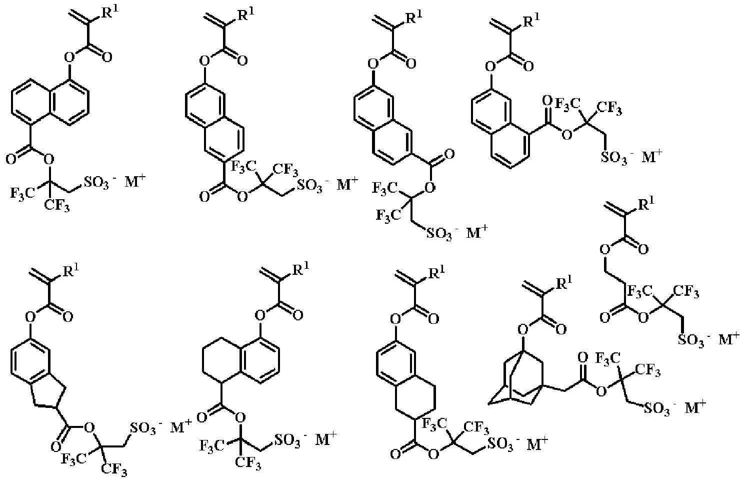

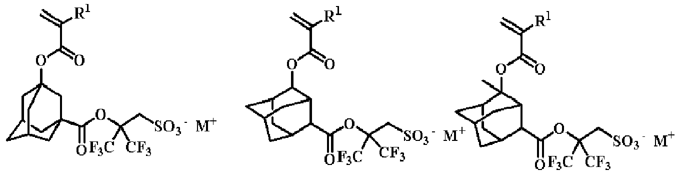

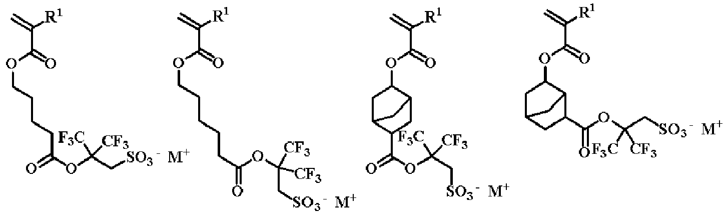

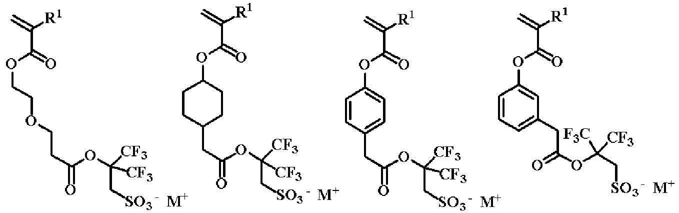

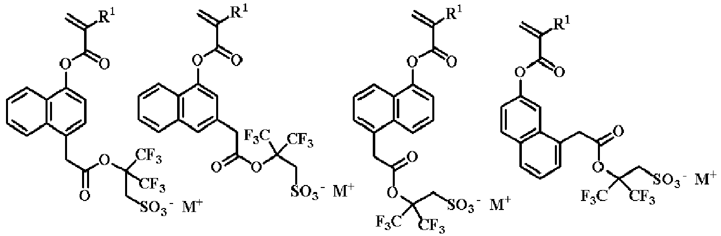

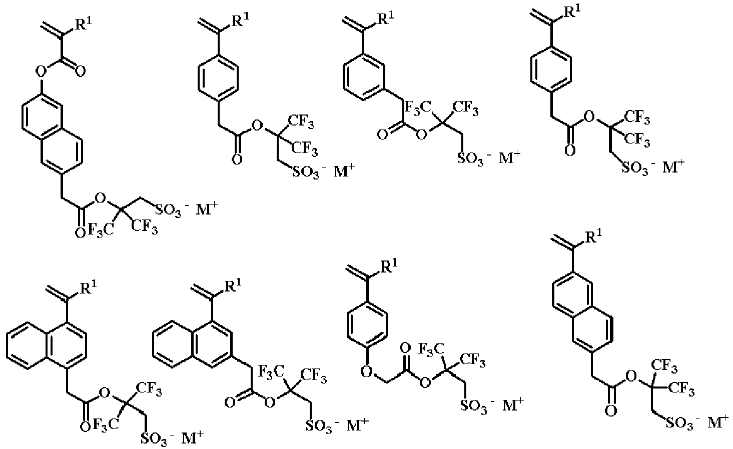

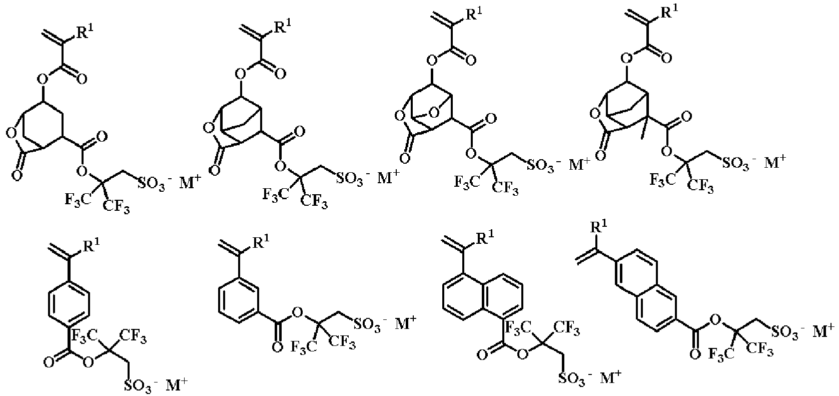

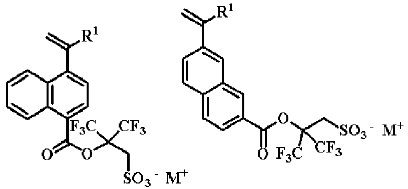

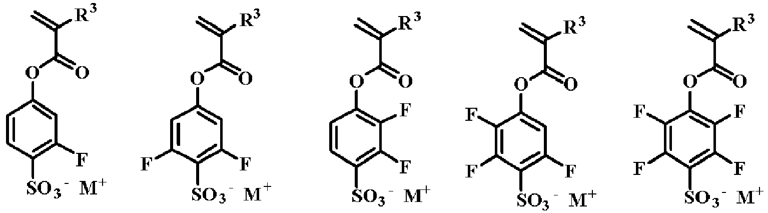

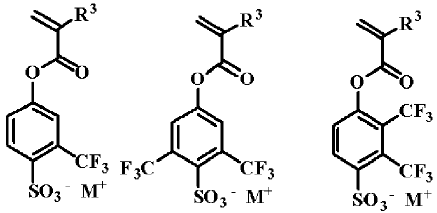

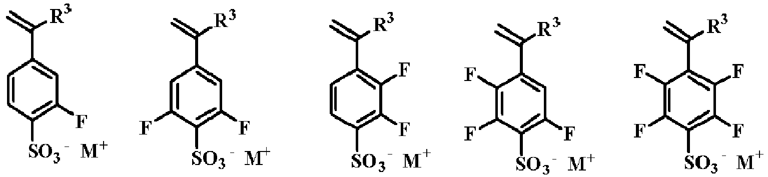

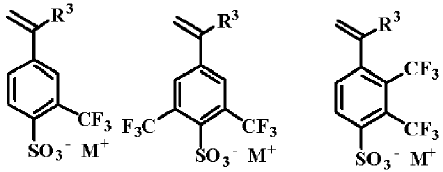

Specific examples of a sulfonimide salt monomer for obtaining the repeating unit-A3 represented by the general formula (2) include the following monomers.

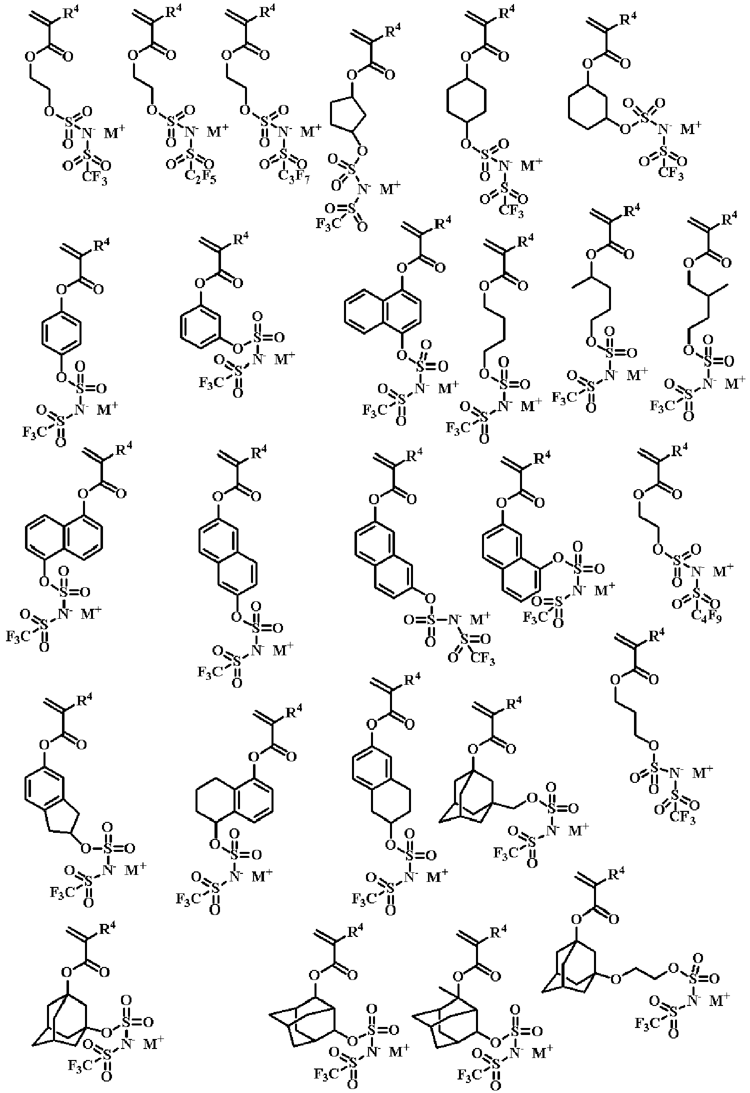

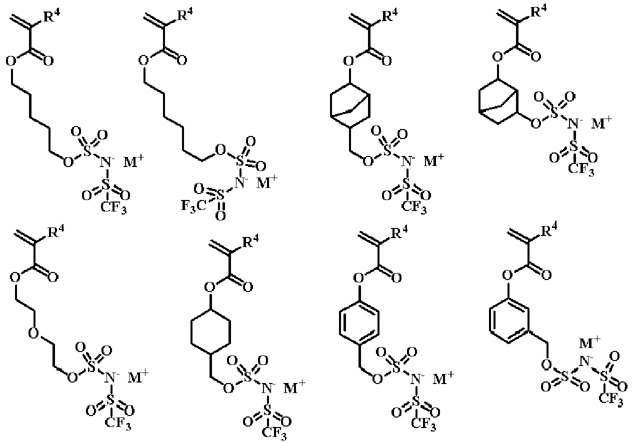

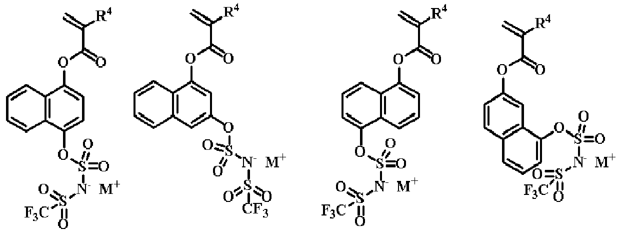

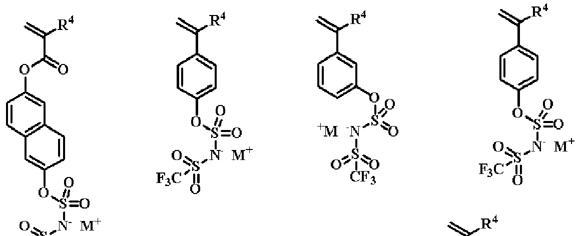

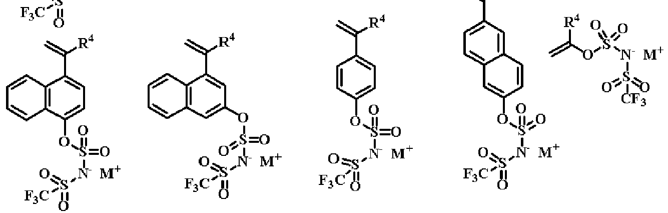

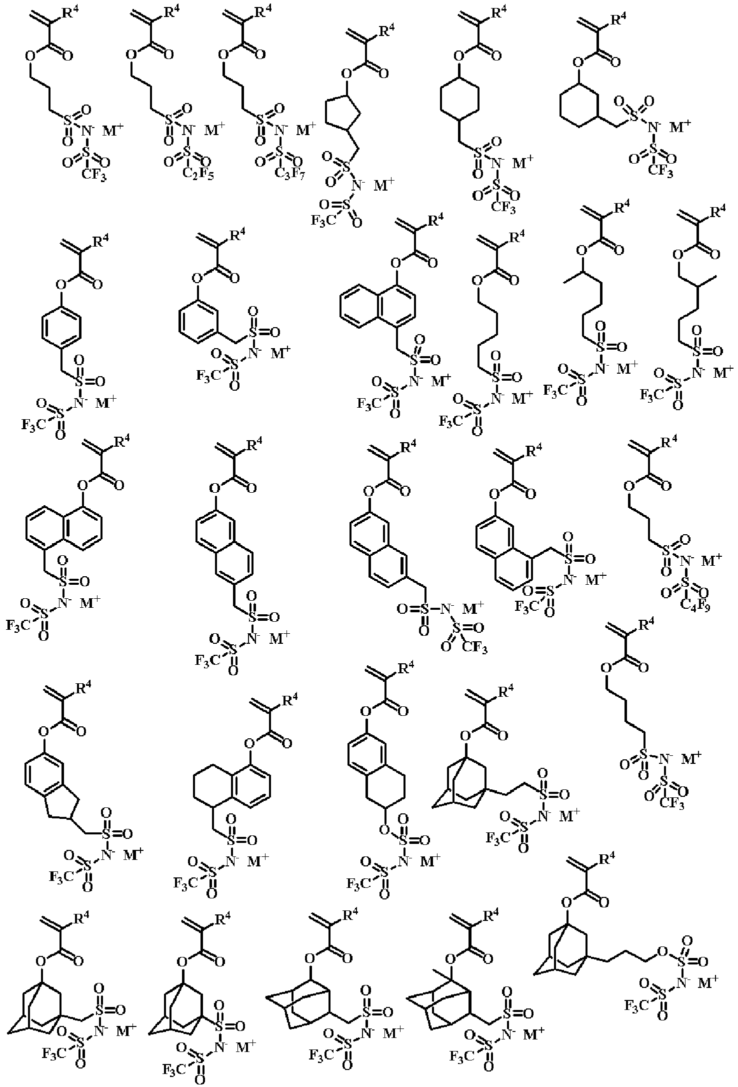

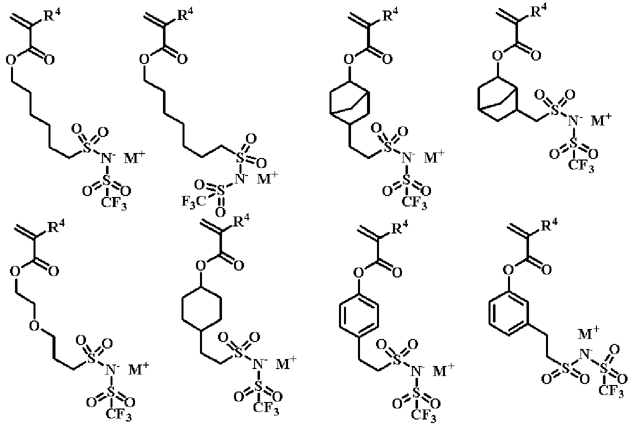

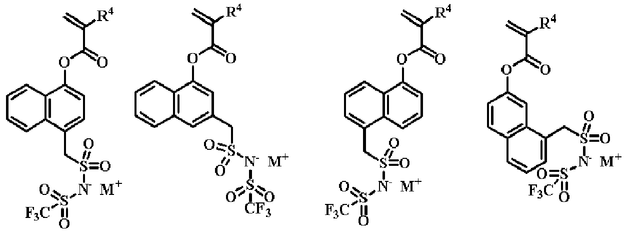

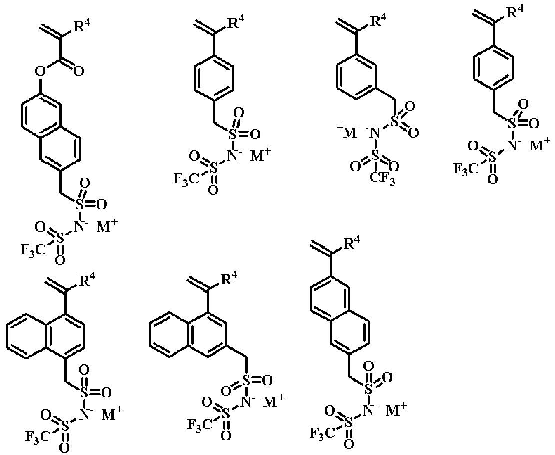

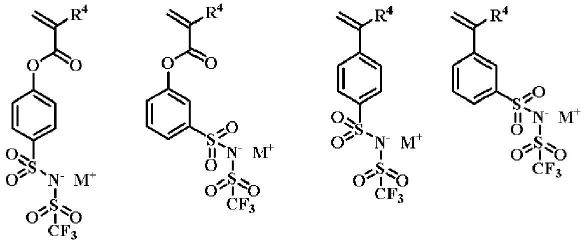

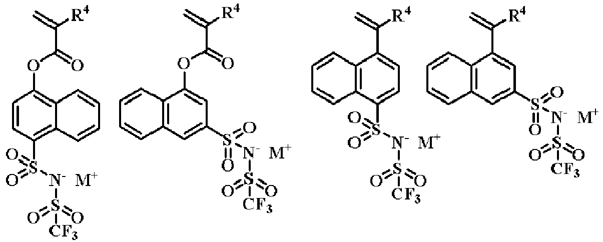

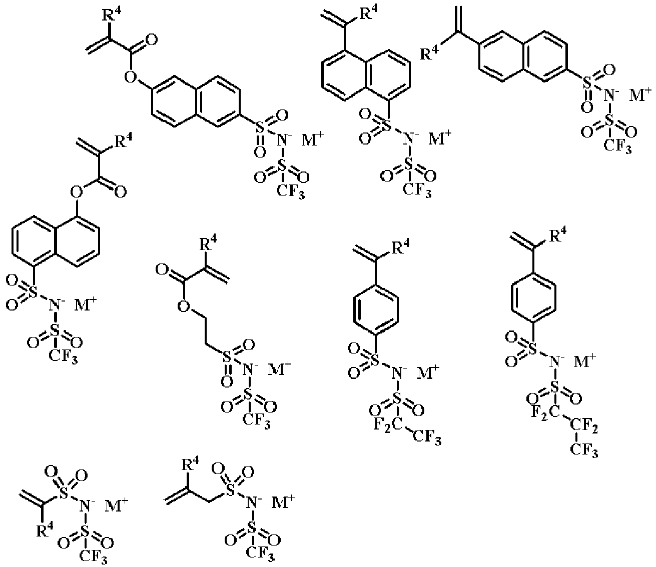

Specific examples of an N-carbonylsulfonamide salt monomer for obtaining the repeating unit-A4 represented by the general formula (2) include the following monomers.

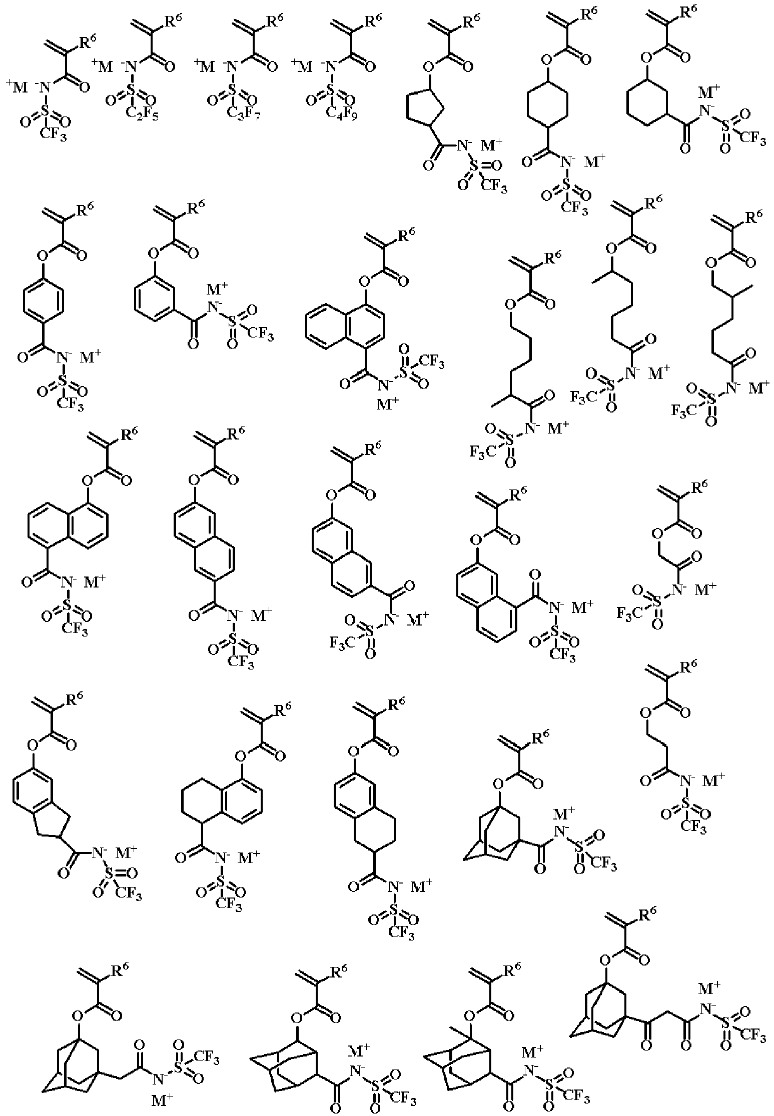

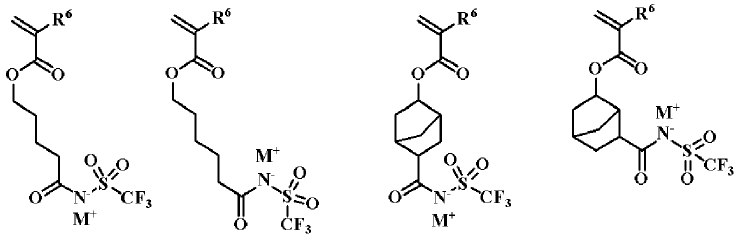

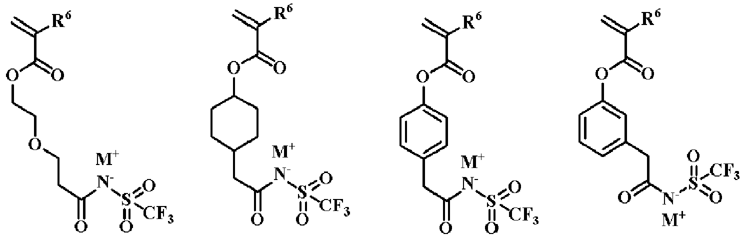

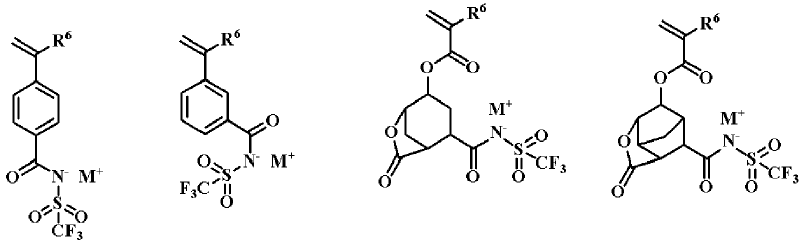

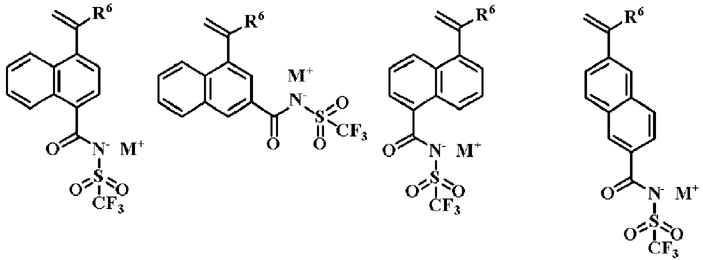

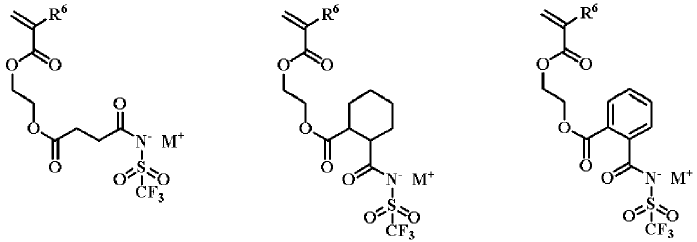

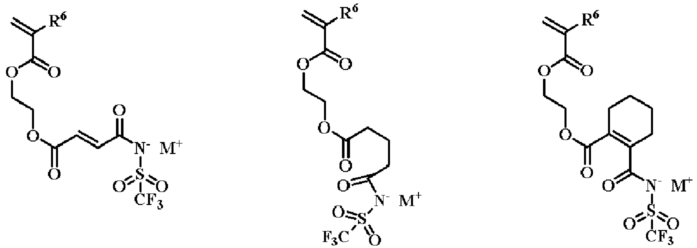

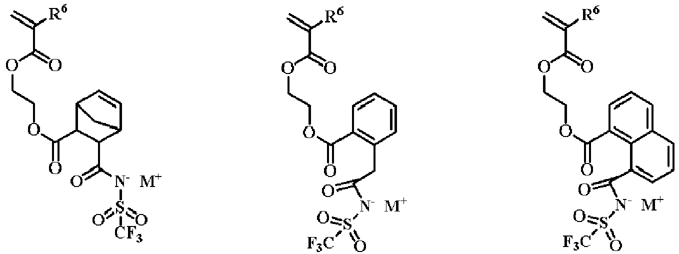

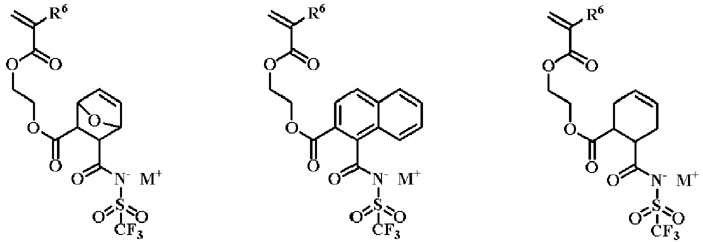

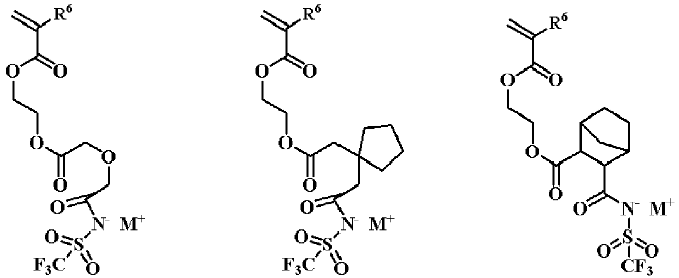

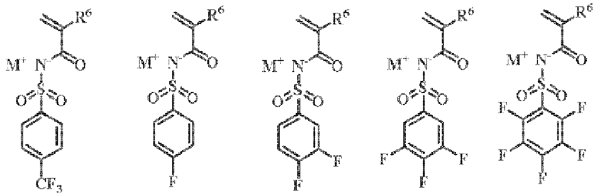

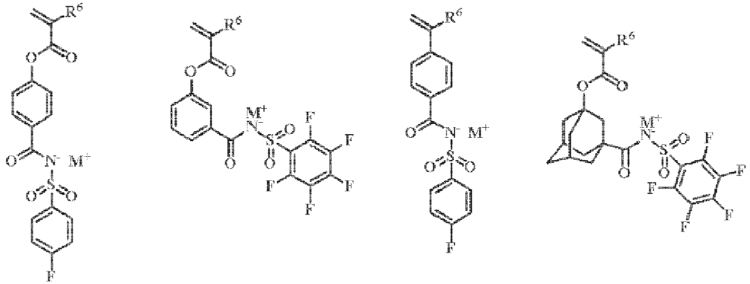

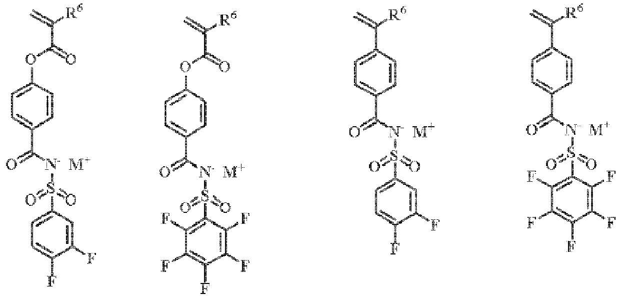

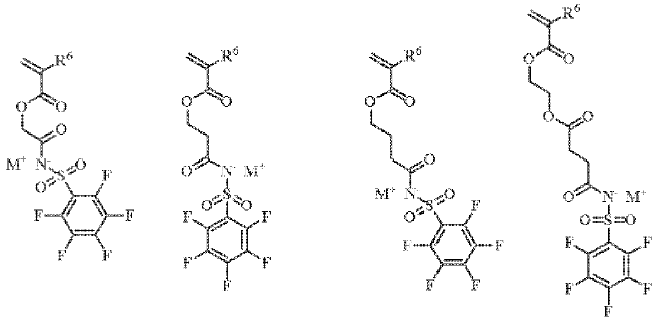

In the formulae, R¹, R³, R⁴, and R⁶ are as above.

### (Repeating Unit-b)

For the component (A), a repeating unit-b having a glyme chain may be copolymerized in addition to the repeating units-A1 to -A4 in order to improve the ion conductivity. Specific examples of a monomer for obtaining the repeating unit-b having a glyme chain include the following monomers. Copolymerizing the repeating unit having a glyme chain can assist moving of ions, which is released from skin, in a dry electrode film to improve sensitivity of the dry electrode.

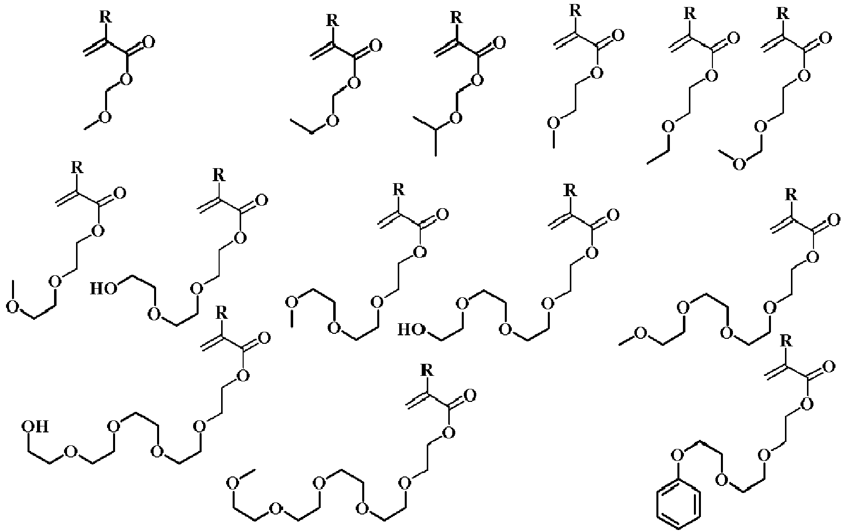

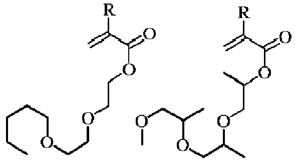

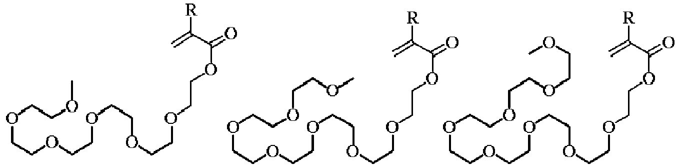

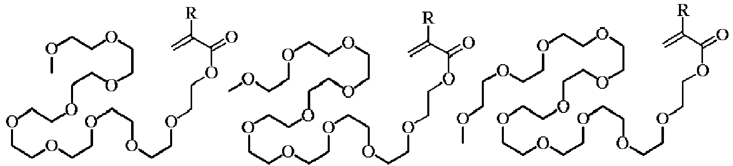

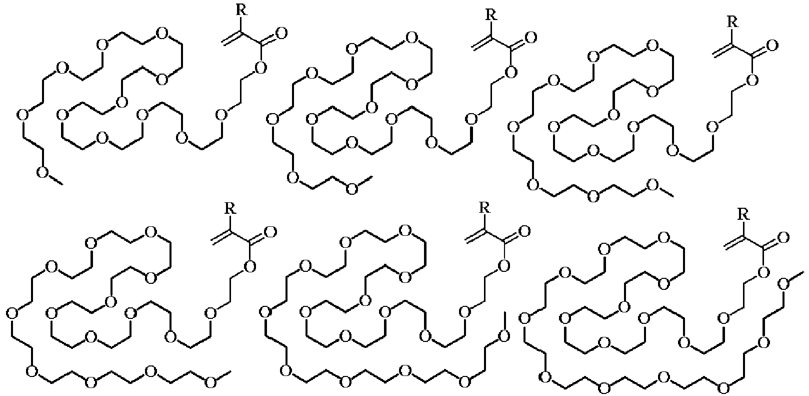

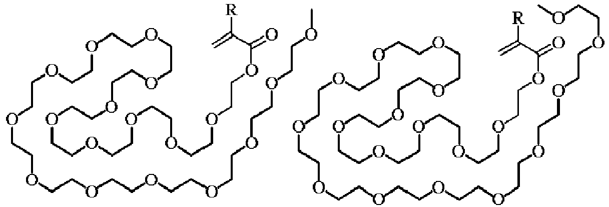

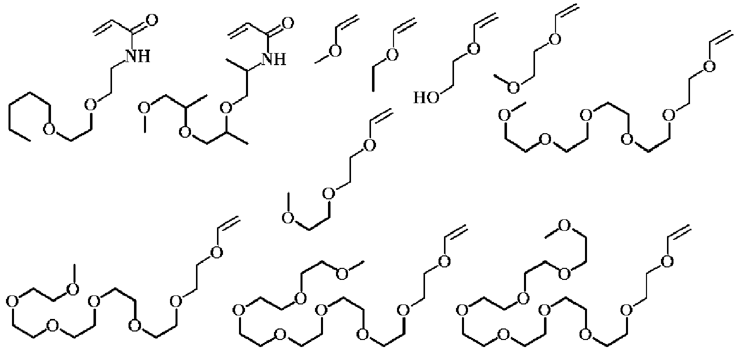

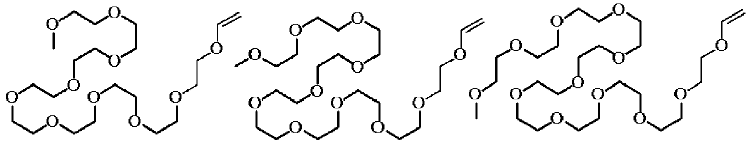

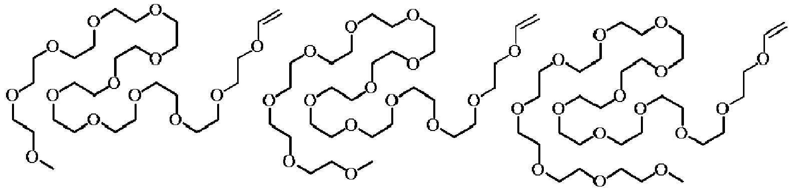

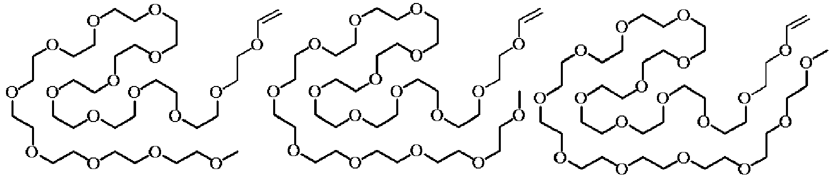

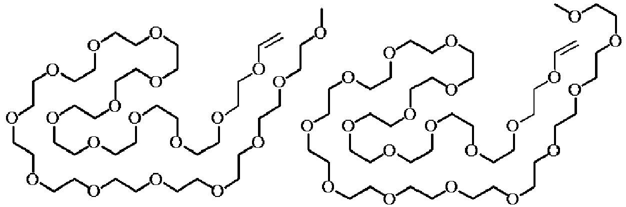

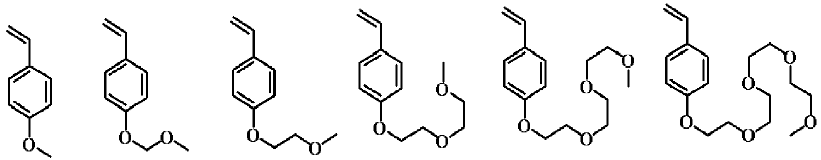

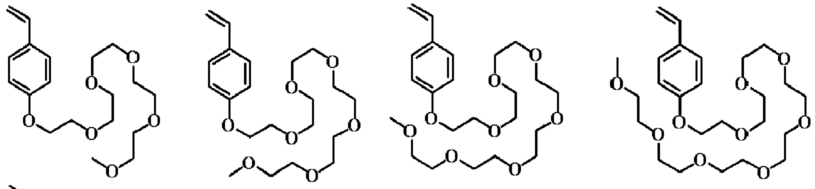

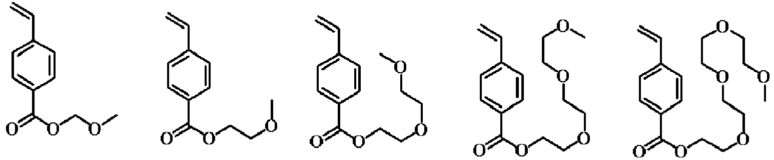

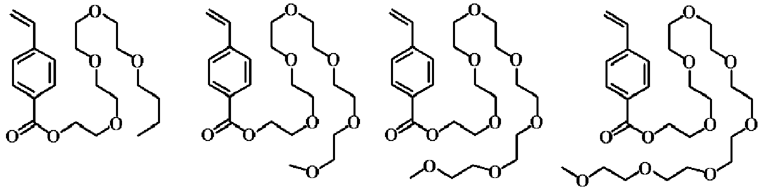

In the formulae, R represents a hydrogen atom or a methyl group.

### (Repeating Unit-c)

For the component (A), a hydrophilic repeating unit-c having a hydroxy group, a carboxyl group, an ammonium salt, a betaine, an amide group, pyrrolidone, a lactone ring, a lactam ring, a sultone ring, a sulfonic acid, a sodium salt of a sulfonic acid, or a potassium salt of a sulfonic acid may be copolymerized in addition to the repeating units-A1 to -A4 and -b in order to improve the conductivity. Specific examples of a monomer for obtaining the hydrophilic repeating unit-c having include the following monomers. Copolymerizing the repeating units having these hydrophilic groups can increase sensibility of ions released from skin to improve sensitivity of the dry electrode.

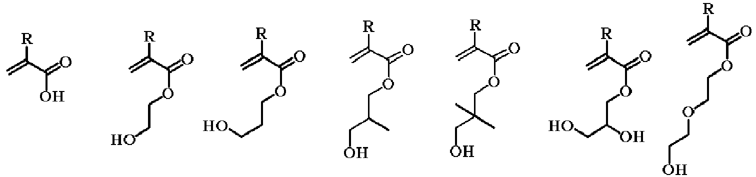

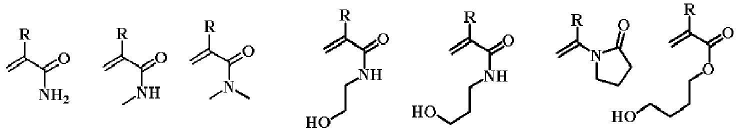

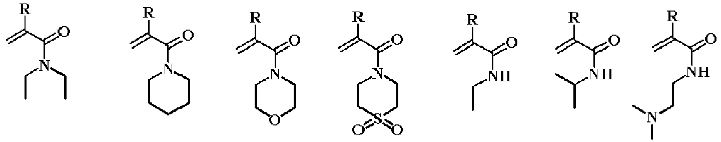

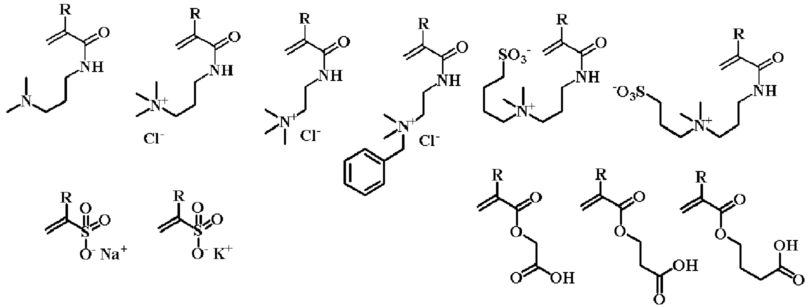

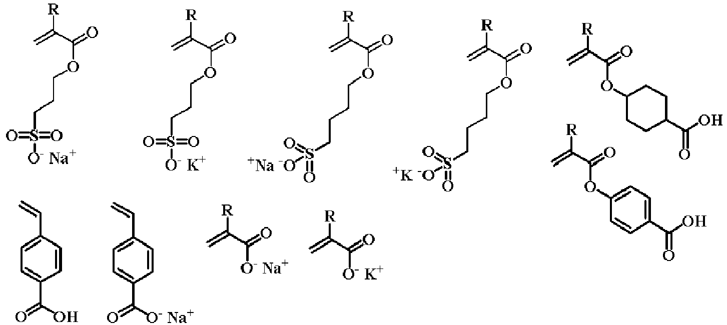

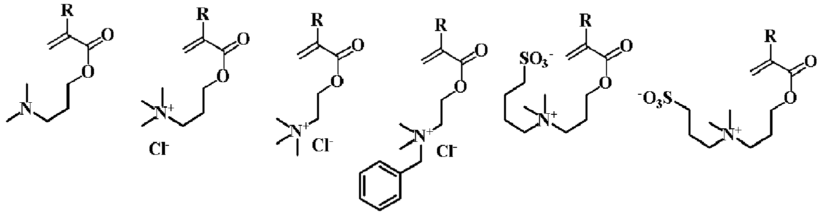

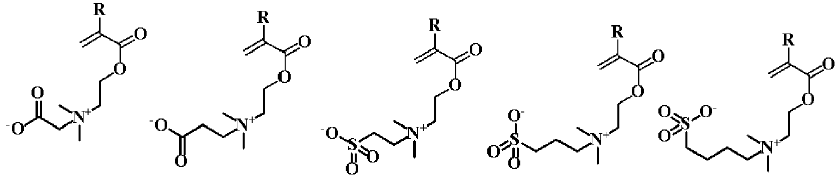

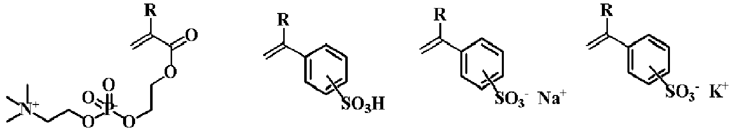

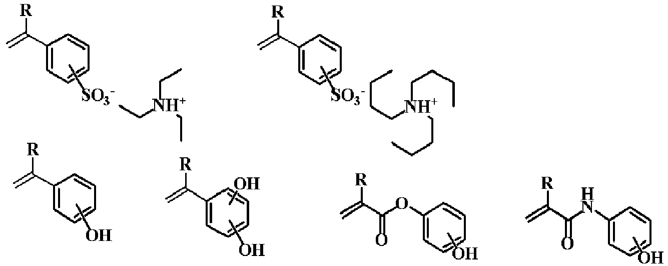

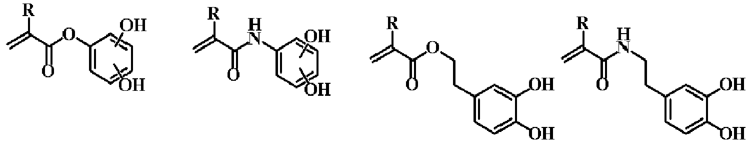

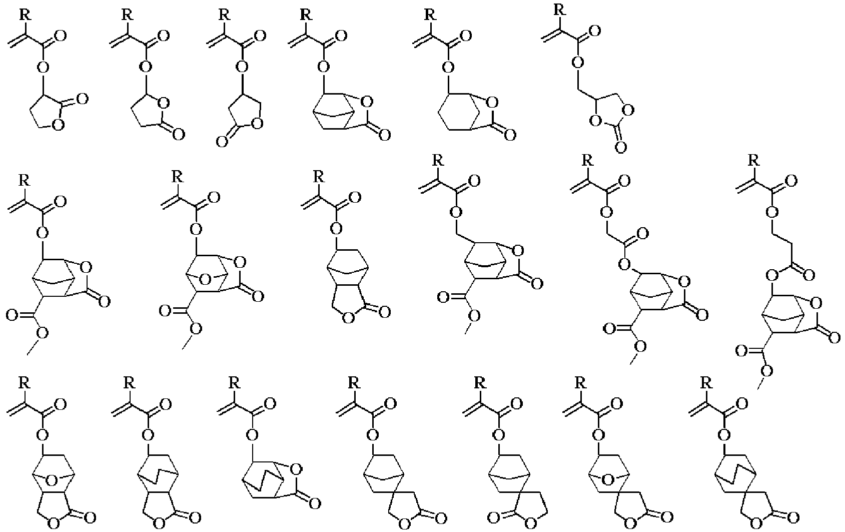

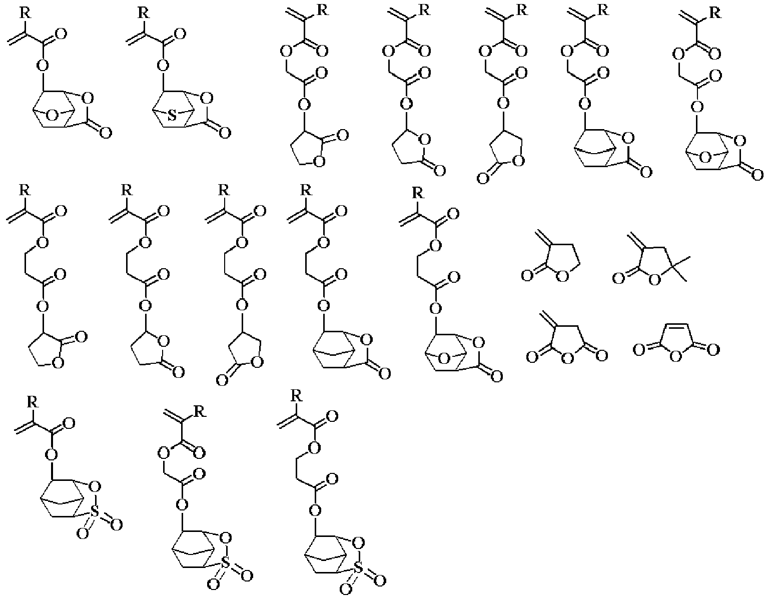

In the formula, R represents a hydrogen atom or a methyl group.

### (Repeating Unit-d)

The component (A) may have a repeating unit-d having fluorine in addition to the repeating units-A1 to -A4, -b, and -c.

Specific examples of a monomer for obtaining the repeating unit-d having fluorine include the following monomers.

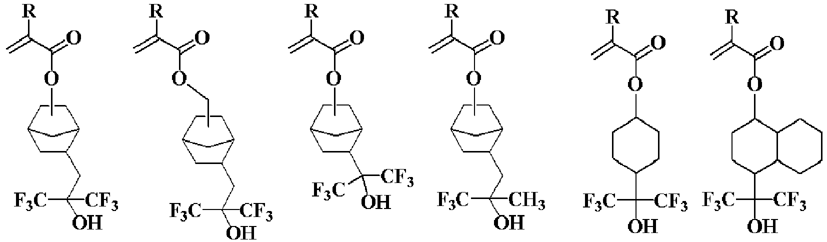

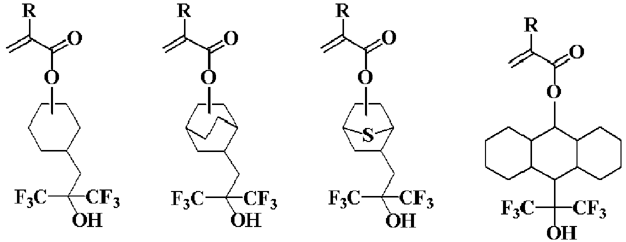

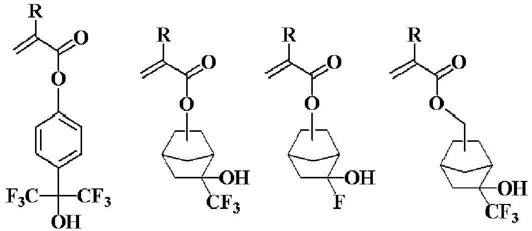

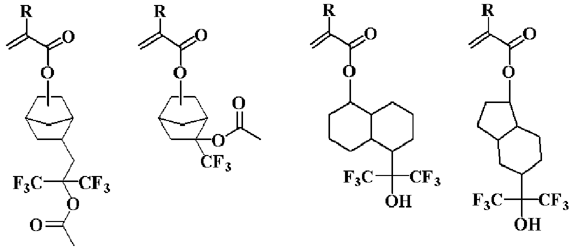

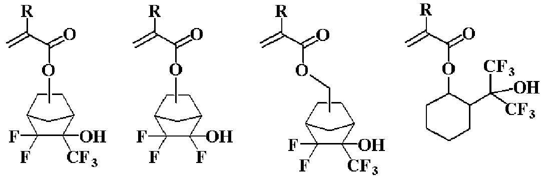

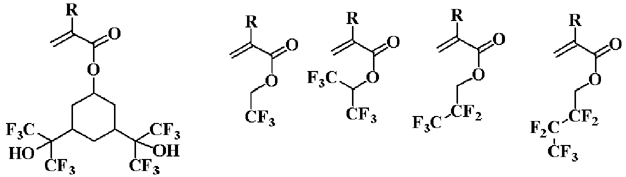

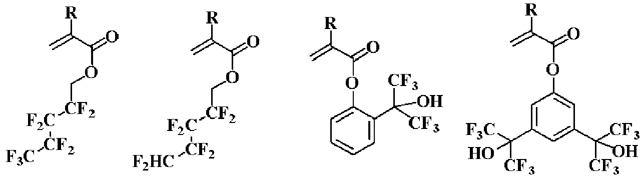

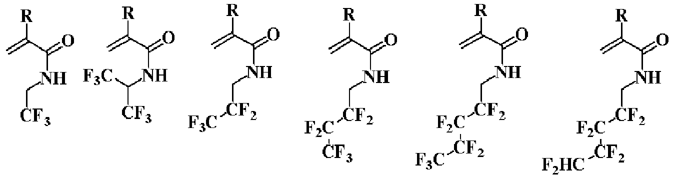

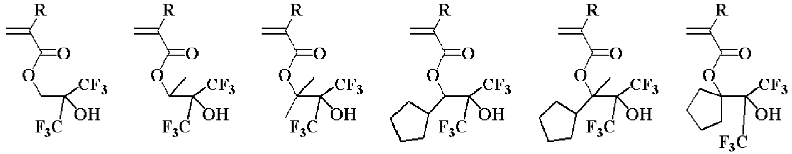

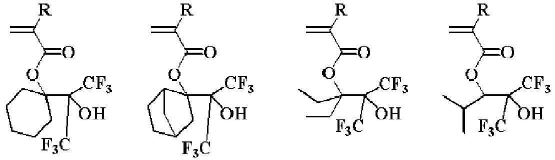

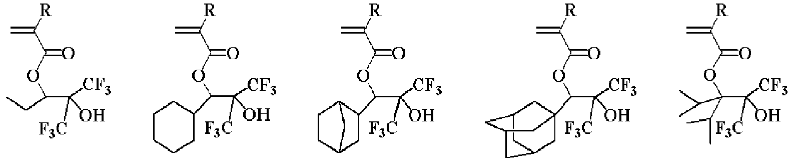

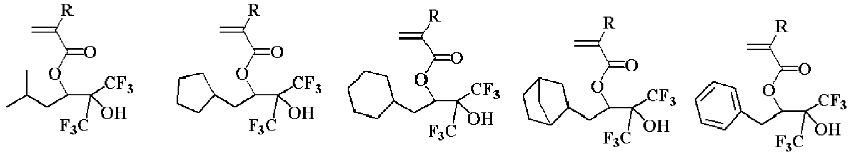

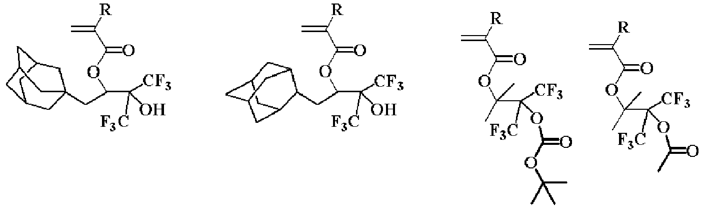

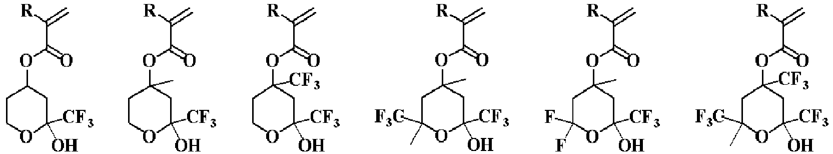

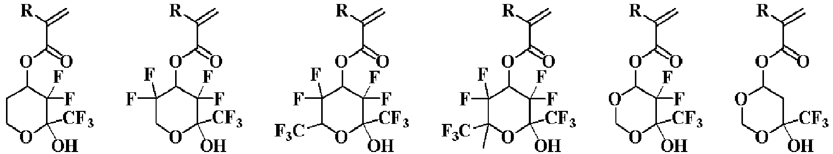

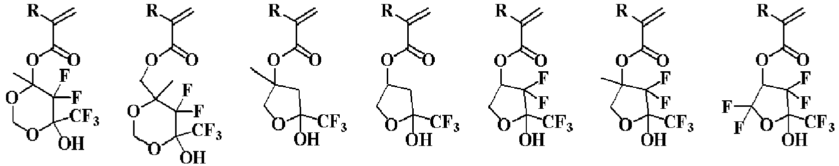

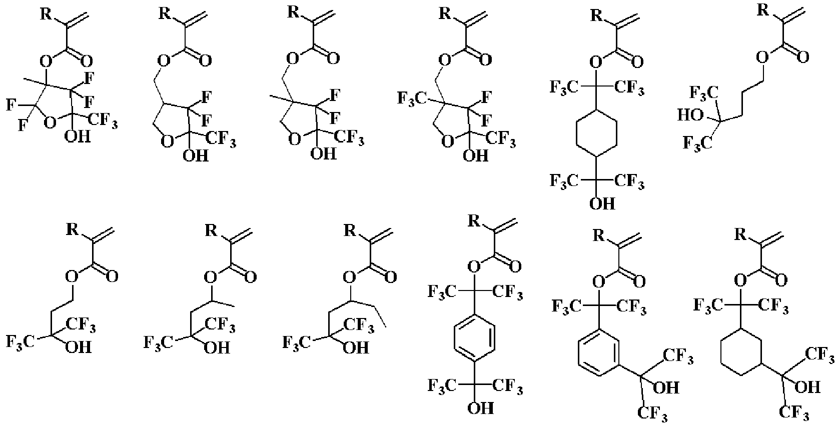

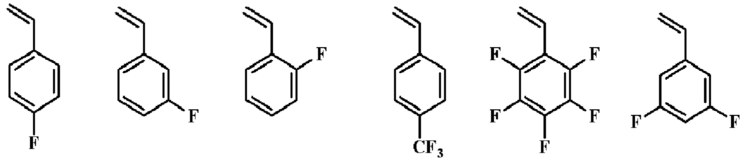

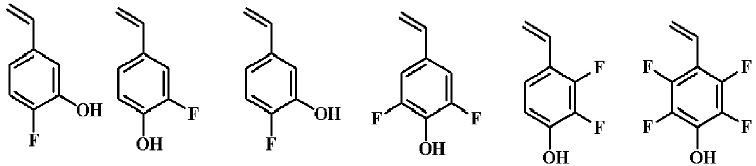

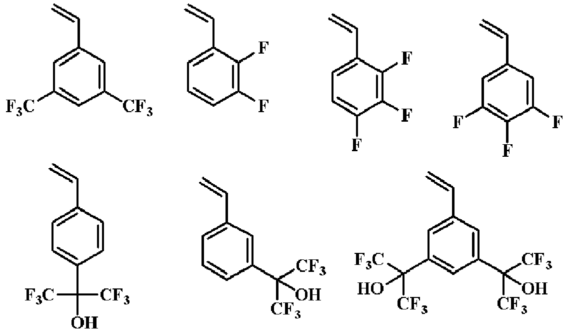

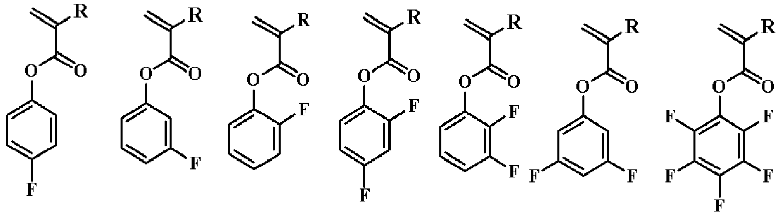

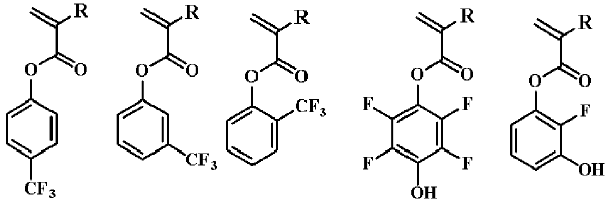

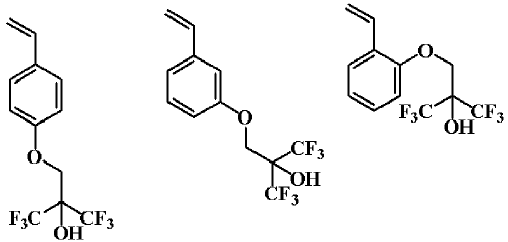

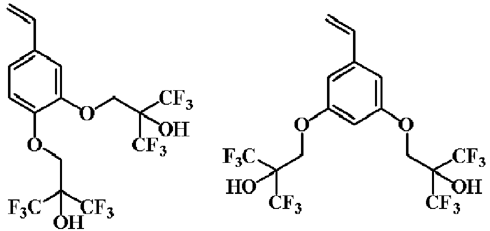

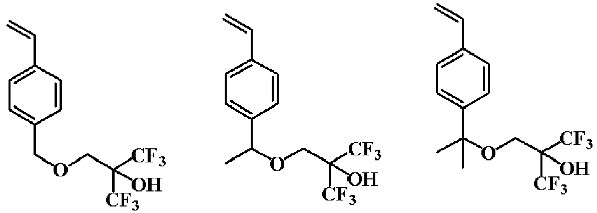

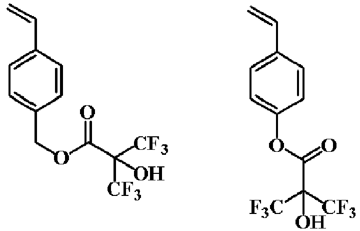

In the formulae, R represents a hydrogen atom or a methyl group.

### (Repeating Unit-e)

The component (A) may further have a repeating unit-e having a nitro group in addition to the repeating units-A1 to -A4, -b, -c, and -d.

Specific examples of a monomer for obtaining the repeating unit-e having a nitro group include the following monomers. 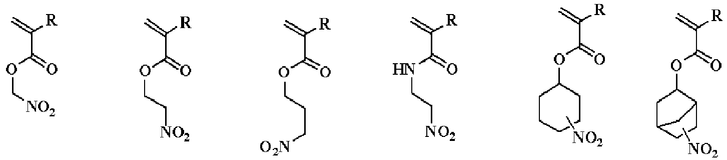 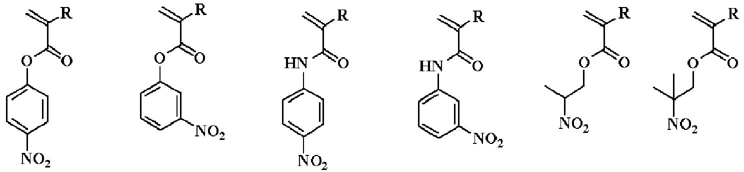 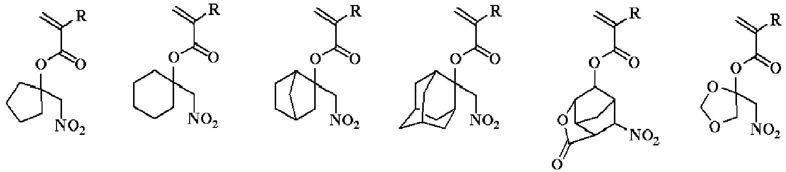 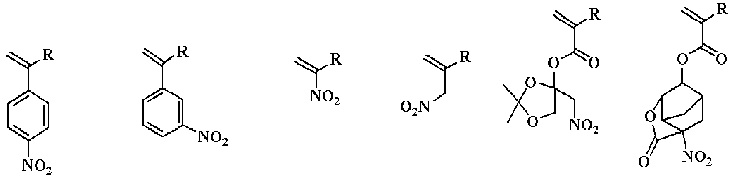 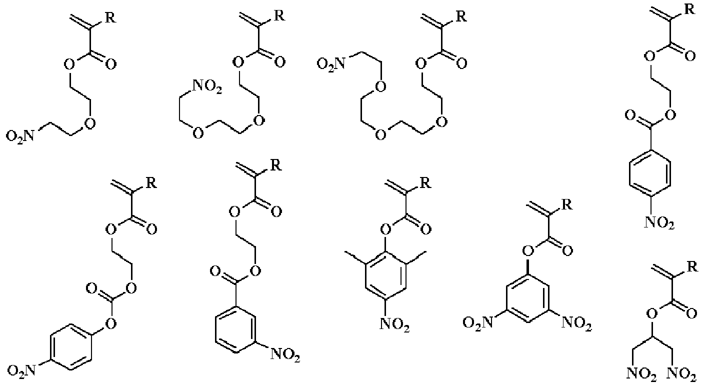 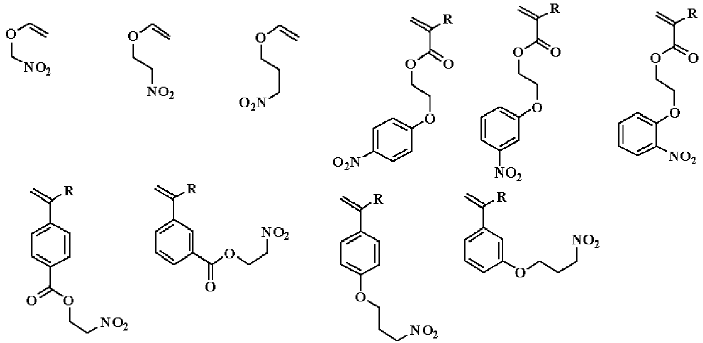 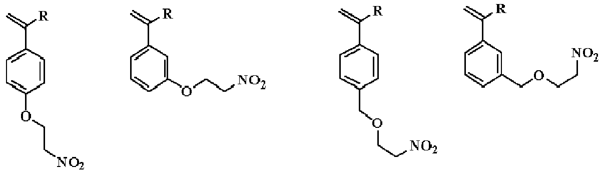 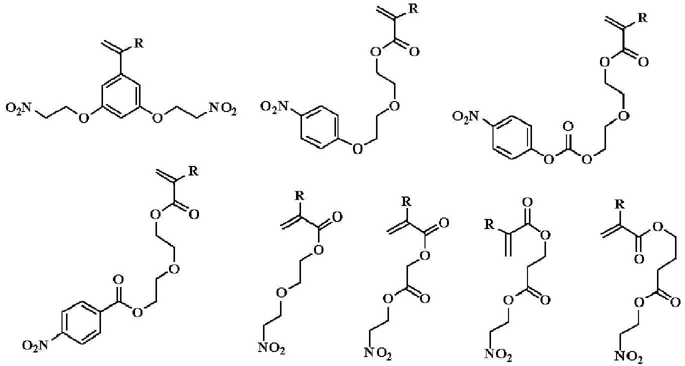 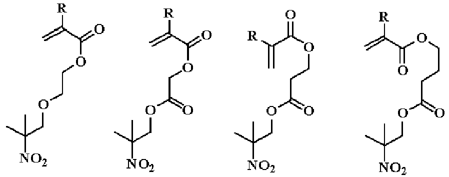 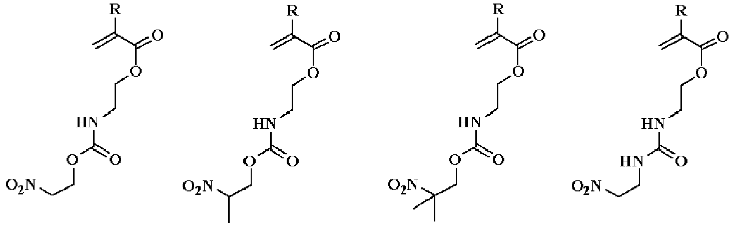 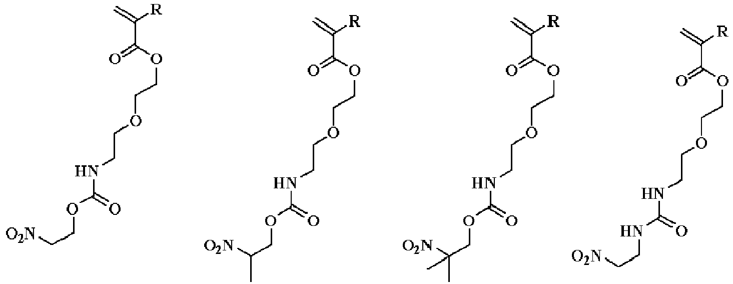 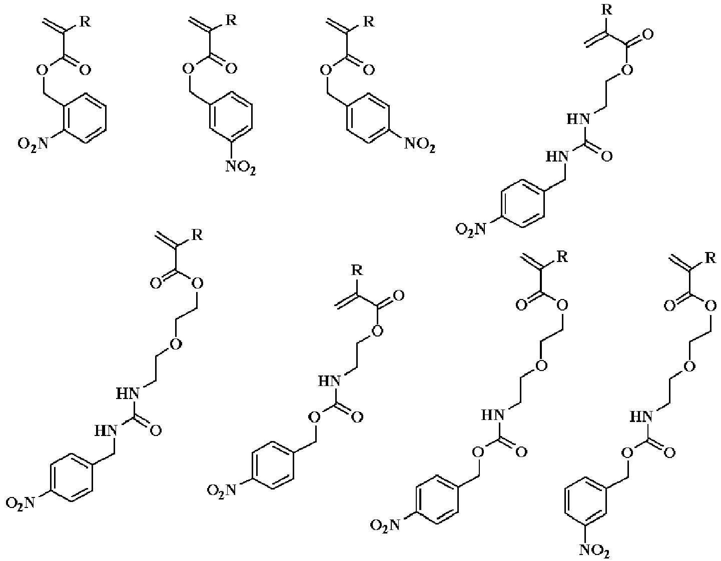 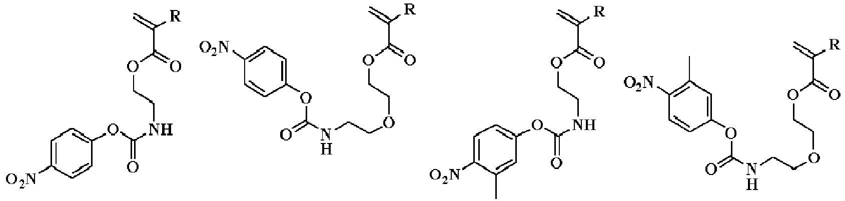 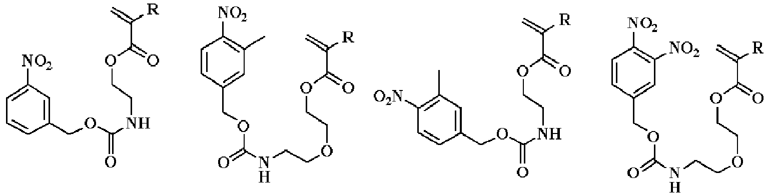 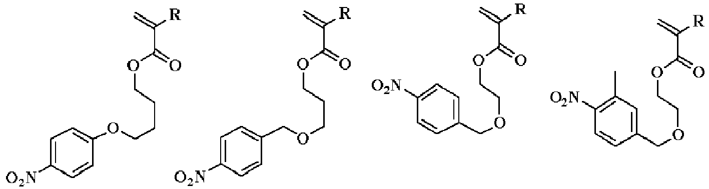 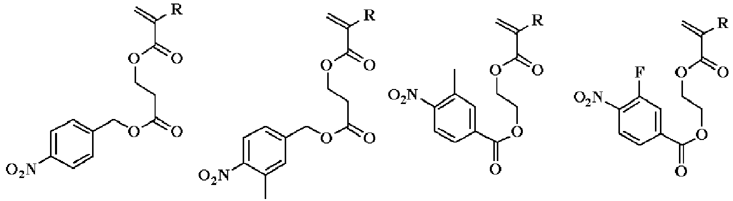 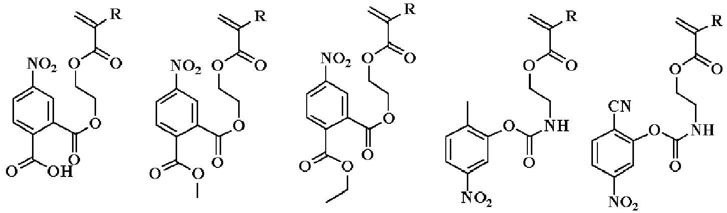 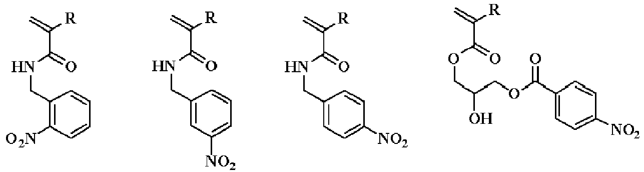 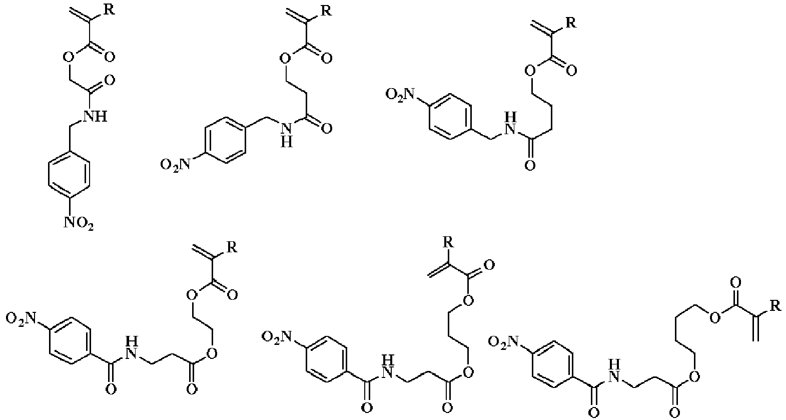 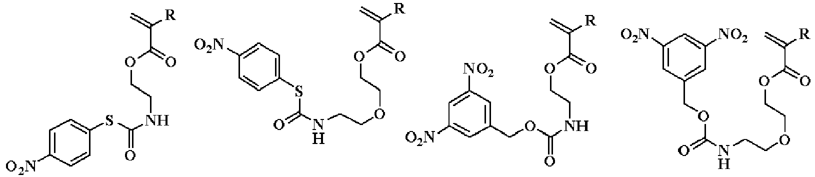 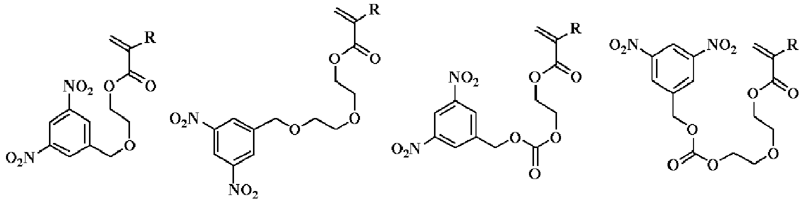 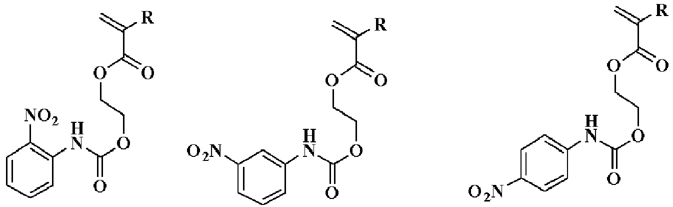 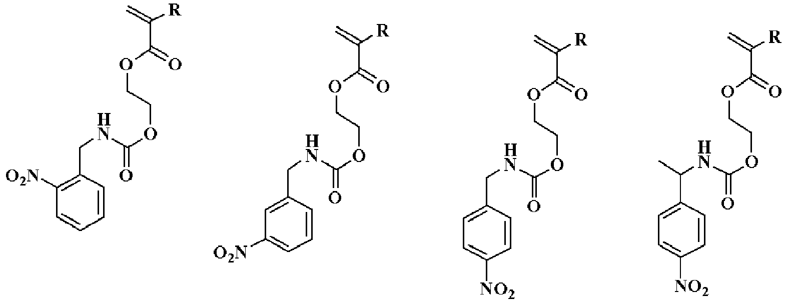 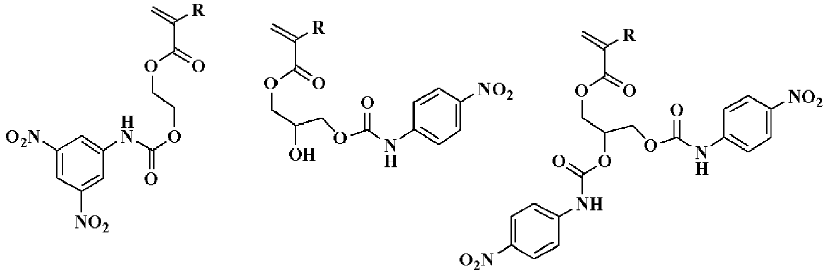 In the formulae, R represents a hydrogen atom or a methyl group.

### (Repeating Unit-f)

The component (A) may further have a repeating unit-f having a cyano group in addition to the repeating units-A1 to -A4, -b, -c, -d, and -e.

Specific examples of a monomer for obtaining the repeating unit-f having a cyano group include the following monomers.

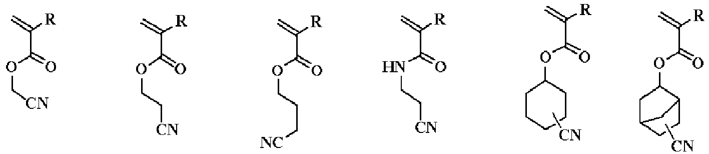

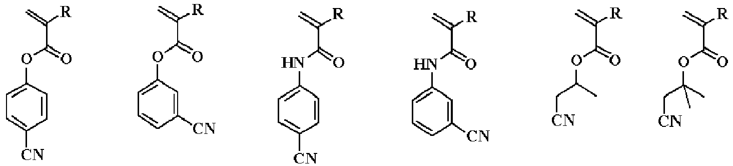

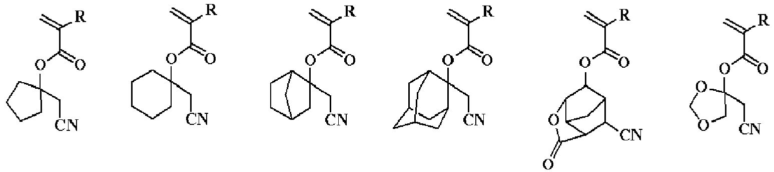

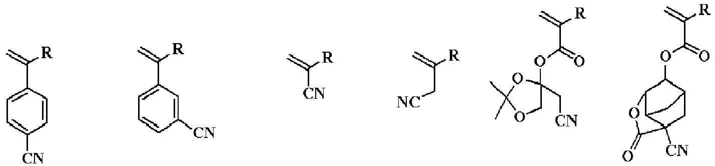

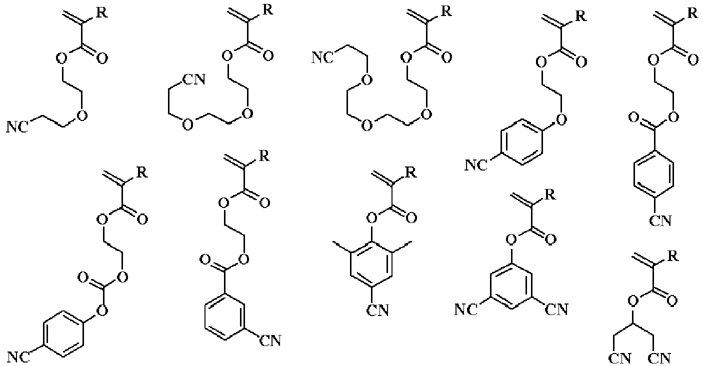

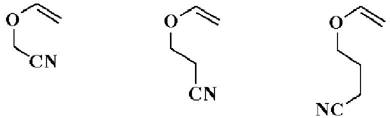

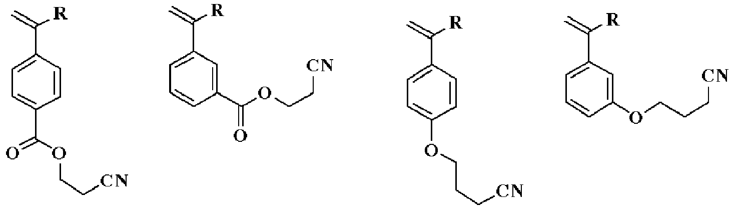

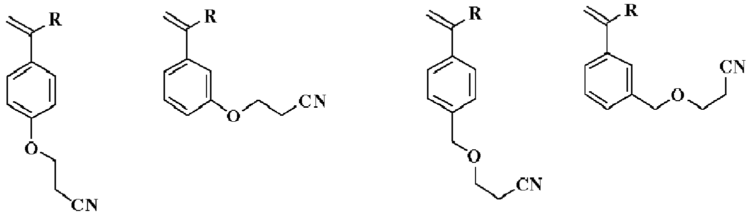

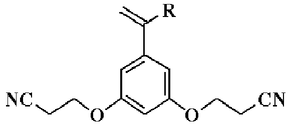

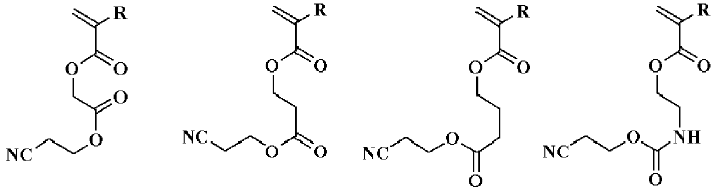

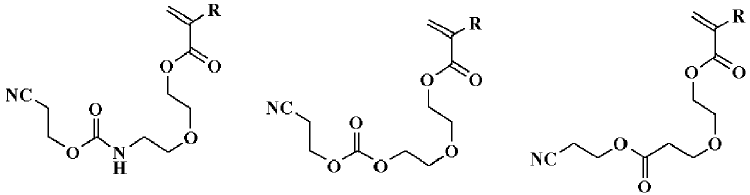

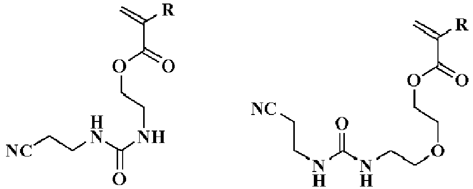

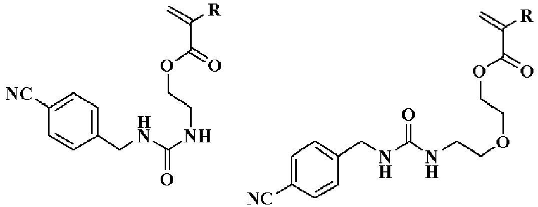

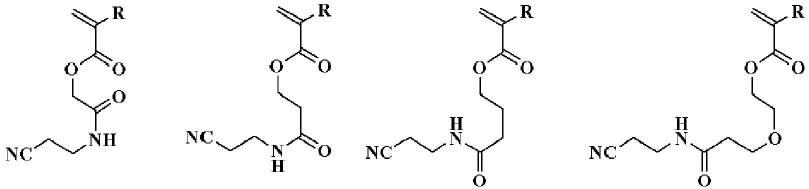

In the formulae, R represents a hydrogen atom or a methyl group.

### (Repeating Unit-g)

The component (A) may further have a repeating unit-g having an oxirane group or an oxetane group in addition to the repeating units-A1 to -A4, -b, -c, -d, - e, and -f.

Specific examples of a monomer for obtaining the repeating unit-g having an oxirane group or an oxetane group include the following monomers.

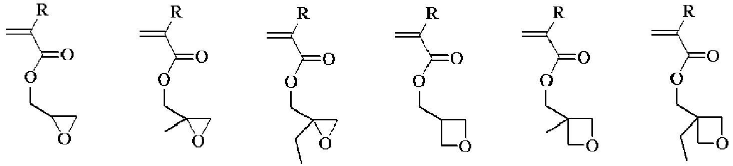

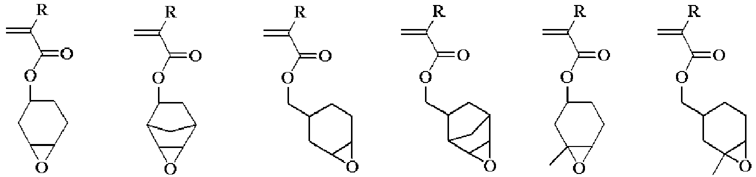

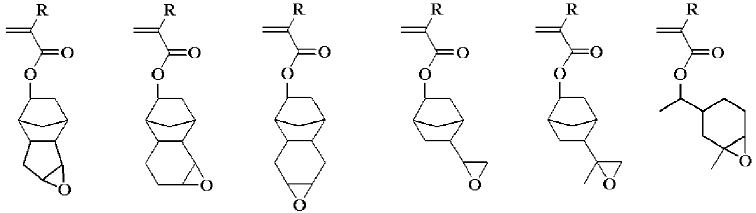

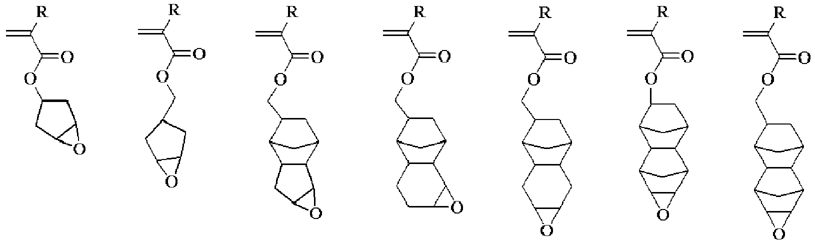

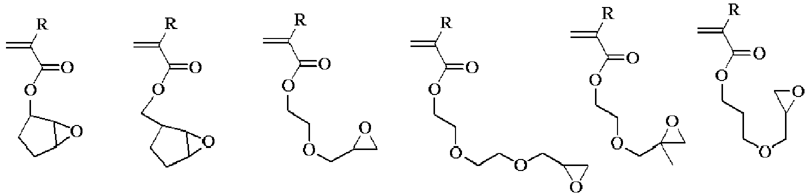

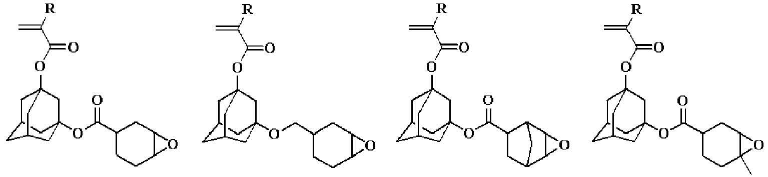

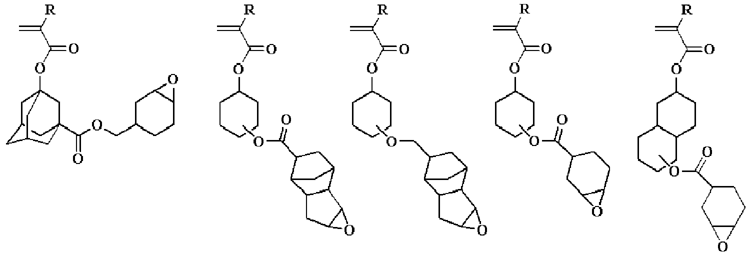

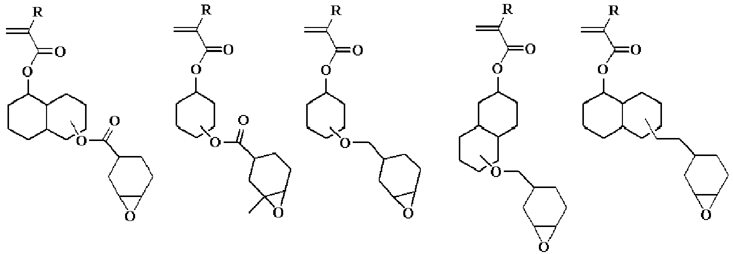

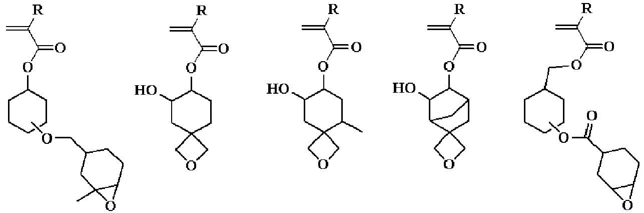

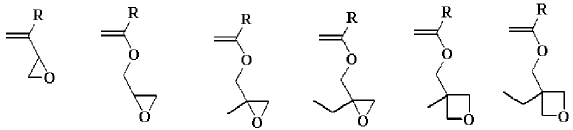

In the formulae, R represents a hydrogen atom or a methyl group.

### (Repeating Unit-h)

The component (A) may further have a repeating unit-h having an isocyanate group or a blocked isocyanate group in addition to the repeating units-A1 to -A4, -b, -c, -d, -e, -f, and -g.

Specific examples of a monomer for obtaining the repeating unit-h having an isocyanate group or a blocked isocyanate group include the following monomers.

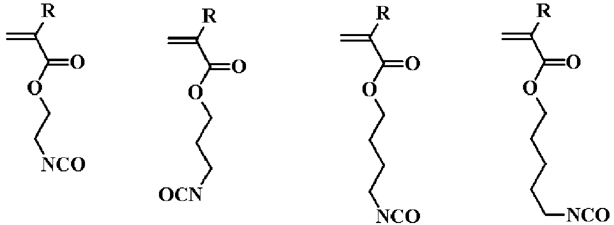

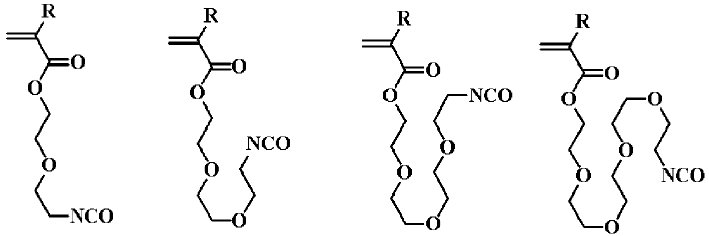

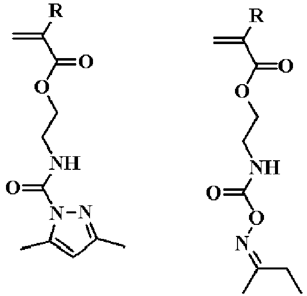

In the formulae, R represents a hydrogen atom or a methyl group.

As a method for synthesizing the ionic polymer (A) having the repeating units-A1 to -A4 described in the general formulae (2), for example, desired monomers among monomers to provide the aforementioned repeating units-A1 to -A4, -b, -c, -d, -e, -f, -g, and -h are subjected to heating polymerization in an organic solvent with adding a radical polymerization initiator to obtain the ionic polymer (A).

Examples of the organic solvent used in the polymerization include toluene, benzene, tetrahydrofuran, diethyl ether, dioxane, cyclohexane, cyclopentane, methyl ethyl ketone, and γ-butyrolactone.

Examples of the radical polymerization initiator include 2,2'-azobisisobutyronitrile (AIBN), 2,2'-azobis(2,4-dimethylvaleronitrile), dimethyl 2,2'-azobis(2-methylpropionate), benzoyl peroxide, and lauroyl peroxide.

The reaction temperature is preferably 50 to 80°C, and the reaction time is preferably 2 to 100 hours, and more preferably 5 to 20 hours.

In the ionic polymer (A), a number of monomers to provide the repeating units-A1 to -A4 may be one or two or more.

When two or more monomers to provide the repeating units-A1 to -A4 are used, each monomer may be randomly copolymerized, or block-copolymerized.

The monomers to provide the repeating units-A1 to - A4, -b, -c, -d, -e, -f, -g, and -h may be randomly copolymerized, or each of them may be block-copolymerized.

For a typical method for the random copolymerization with radical polymerization, the monomers to be copolymerized and the radical polymerization initiator are mixed and heated to perform polymerization. A case where a first monomer is started to be polymerized in the presence of the radical polymerization initiator and then a second monomer is added yields a structure in which the first monomer is polymerized on one side of the polymer molecule and the second monomer is polymerized on the other side. In this case, however, repeating units of the first monomer and the second monomer are mixed in the middle portion, which is different form from a block copolymer. To form a block copolymer with the radical polymerization, living-radical polymerization is preferably used.

In a living-radical polymerization method called reversible addition fragmentation chain transfer (RAFT) polymerization, a radical at the polymer terminal is always alive, and thereby a diblock copolymer of a block of a repeating unit of a first monomer and a block of a repeating unit of a second monomer can be formed by starting polymerization with the first monomer, and adding the second monomer at a stage when the first monomer has been consumed. In addition, when polymerization is started with a first monomer, a second monomer is added at a stage where the first monomer has been consumed, and a third monomer is subsequently added, a triblock polymer can be formed.

The RAFT polymerization has a feature of forming a narrow-dispersed polymer having a narrow molecular weight distribution (dispersion degree). Particularly, a case where monomers are added once to perform the RAFT polymerization can form a polymer having narrower molecular weight distribution.

For the RAFT polymerization, a chain-transfer agent is needed. Specific examples thereof include 2-cyano-2-propyl benzothioate, 4-cyano-4-phenylcarbonothioylthiopentanoic acid, 2-cyano-2-propyl dodecyl trithiocarbonate, 4-cyano-4-[(dodecylsulfanylthiocarbonyl)sulfanyl]pentanoic acid, 2-(dodecylthiocarbonothioylthio)-2-methylpropanoic acid, cyanomethyl dodecyl thiocarbonate, cyanomethyl methyl(phenyl)carbamothioate, bis(thiobenzoyl) disulfide, and bis(dodecylsulfanylthiocarbonyl) disulfide. Among these, 2-cyano-2-propyl benzothioate is particularly preferable.

The ionic polymer has a weight-average molecular weight within a range of 1,000 to 500,000, and preferably 2,000 to 200,000. If the weight-average molecular weight is less than 1,000, heat resistance is deteriorated, and a residue may be generated on the skin after the bio-electrode is contacted with and released from the skin. Meanwhile, if the weight-average molecular weight is more than 500,000, the viscosity increases to deteriorate operability, and solubility in an organic solvent decreases.

The weight-average molecular weight (Mw) is a measured value by gel permeation chromatography (GPC) in terms of polyethylene oxide, polyethylene glycol, or polystyrene using dimethylformamide (DMF) or tetrahydrofuran (THF) as a solvent.

As described in JP 2021-164630 A, a (meth)acrylate group and styrene group, which are radically reactive groups, can be introduced by a reaction with the hydroxy group, the carboxy group, the oxirane group, the oxetane group, the isocyanate group, etc. in the polymerized ionic polymer.

Here, proportions of the repeating units-A1 to -A4, -b, -c, -d, -e, -f, -g, and -h in the ionic polymer are 0≤a1≤1.0, 0≤a2≤1.0, 0≤a3≤1.0, 0≤a4≤1.0, 0<a1+a2+a3+a4≤1.0, 0≤b<1.0, 0≤c<1.0, 0≤d<1.0, 0≤e<1.0, 0≤f<1.0, 0≤g<1.0, and 0≤h<1.0, preferably 0≤a1≤1.0, 0≤a2≤1.0, 0≤a3≤1.0, 0≤a4≤1.0, 0.1≤a1+a2+a3+a4≤1.0, 0≤b≤0.8, 0≤c≤0.8, 0≤d≤0.8, 0≤e≤0.8, 0≤f≤0.8, 0≤g≤0.8, and 0≤h≤0.8, and more preferably 0≤a1≤1.0, 0≤a2≤1.0, 0≤a3≤1.0, 0≤a4≤1.0, 0.2≤a1+a2+a3+a4≤1.0, 0≤b≤0.7, 0≤c≤0.7, 0≤d≤0.7, 0≤e≤0.7, 0≤f≤0.7, 0≤g≤0.7, and 0≤h≤0.7. "b", "c", "d", "e", "f", "g", and "h" represent the proportion of the repeating units-b to -h, respectively.

### (Repeating Units-A5 to -A7)

The monomer for obtaining the repeating units-A5 to -A7 represented by the general formulae (4) has hydroxy groups at both the terminals, and can be reacted with an isocyanate compound for extending the chain length to form a polymer.

The repeating unit-A5 or -A6 can be obtained by a reaction between: a monomer having a dihydroxy group represented by the following general formula a5-1 or a6-1; and a compound having an isocyanate group.

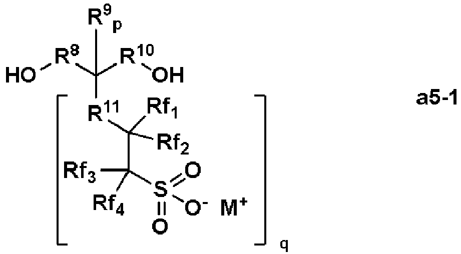

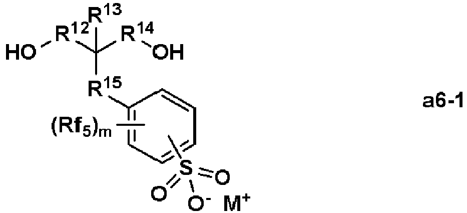

In the formulae, R⁸ to R¹⁵, Rf₁ to Rf₅, "p", "q", "m", and M⁺ are as above.

Specific examples of the monomer having a dihydroxy group represented by the general formula a5-1 include the following monomers.

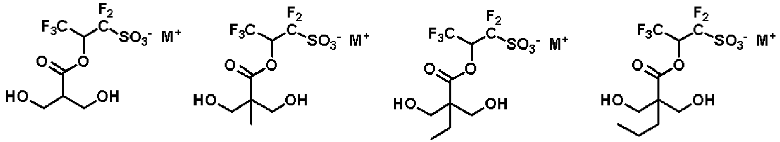

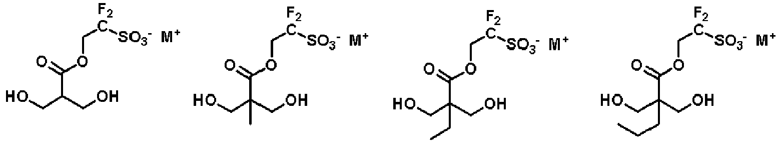

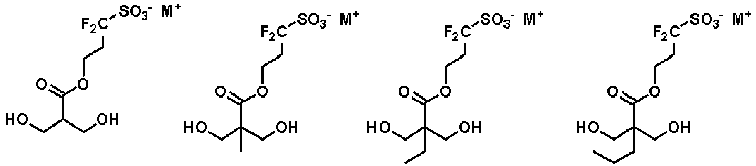

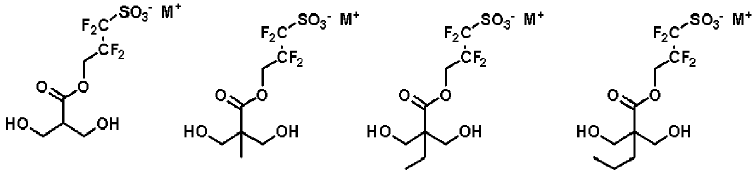

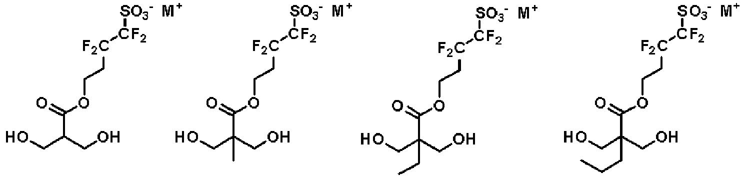

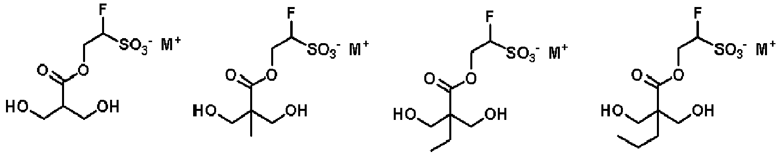

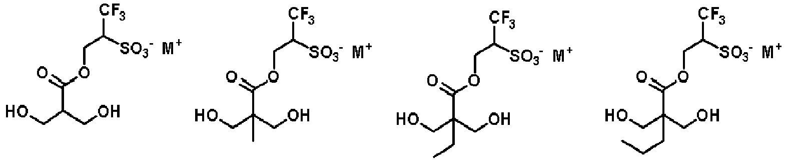

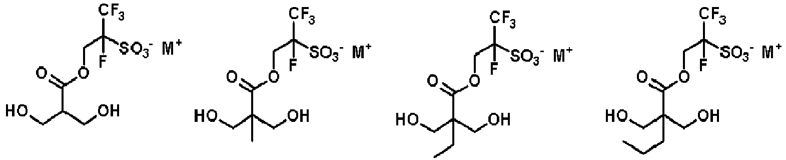

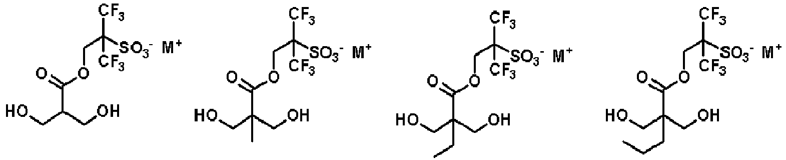

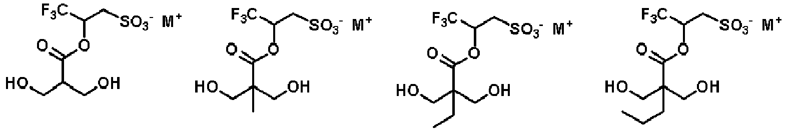

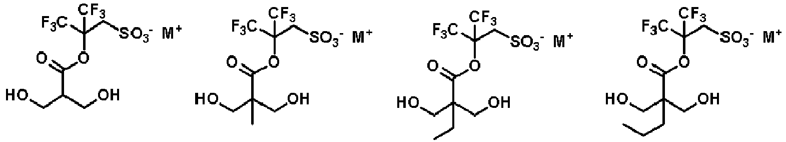

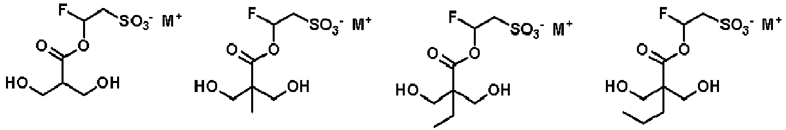

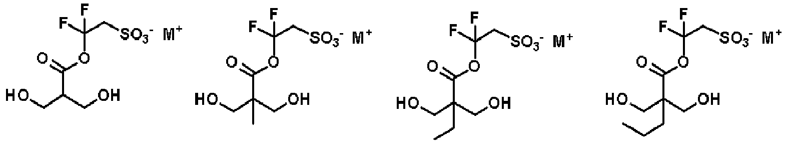

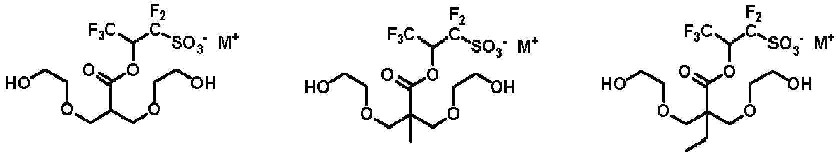

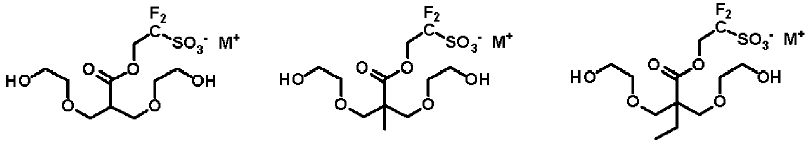

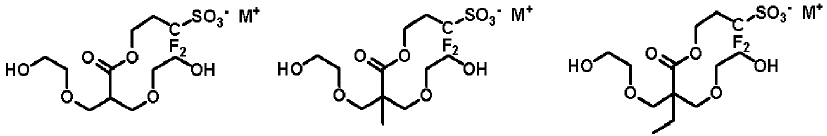

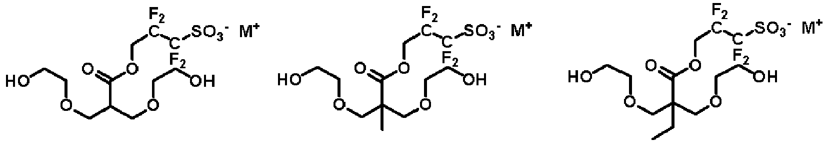

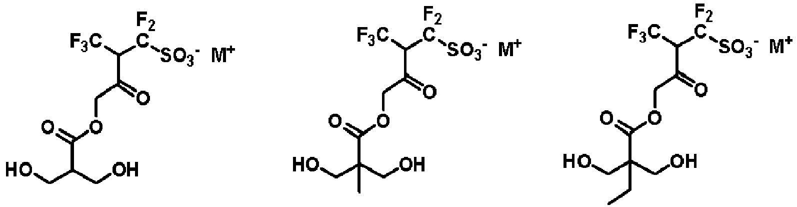

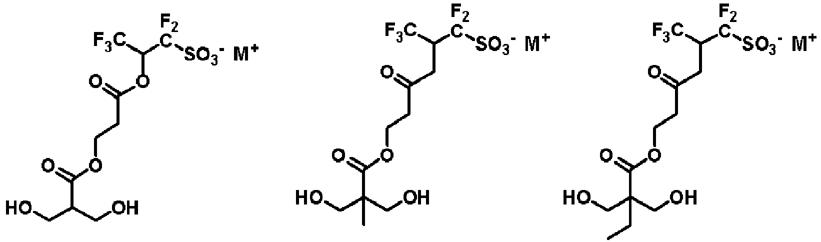

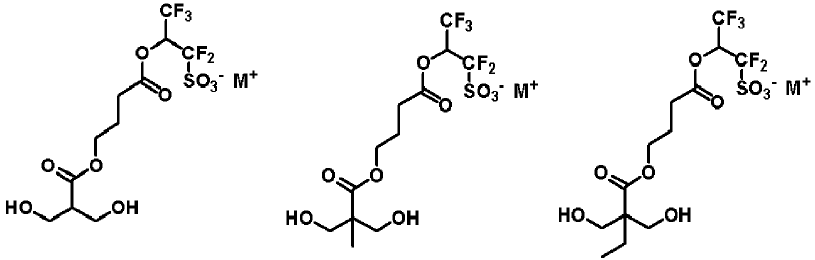

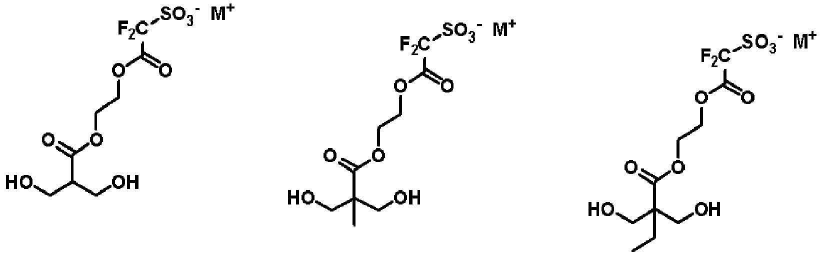

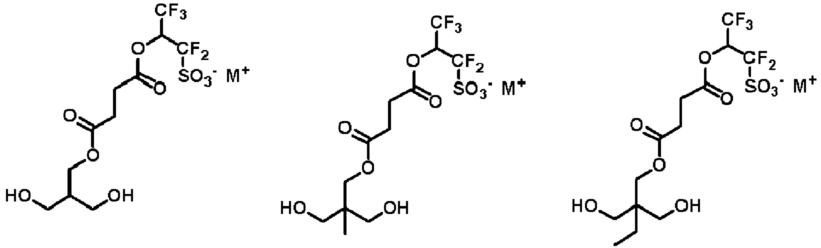

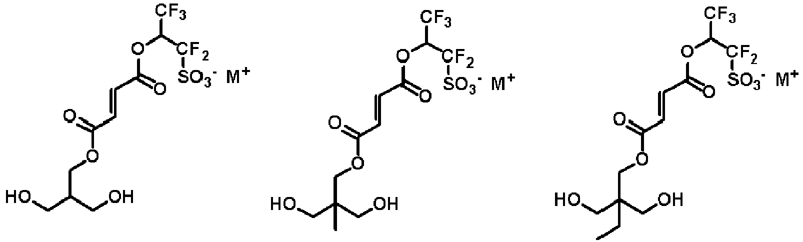

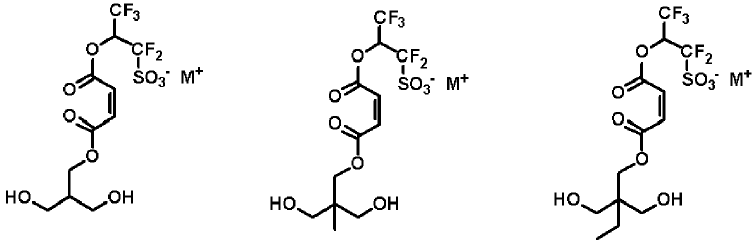

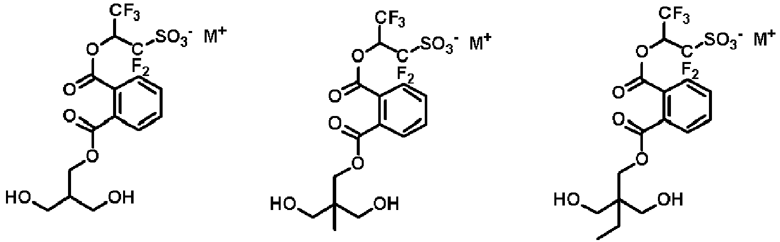

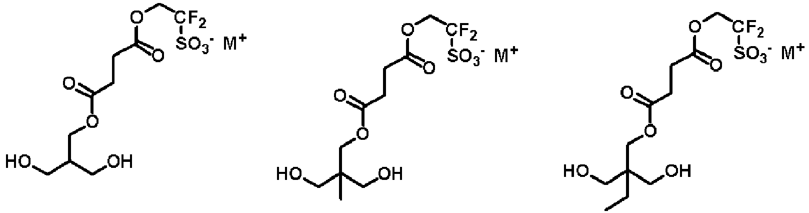

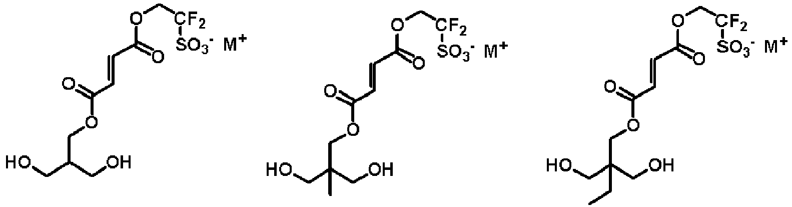

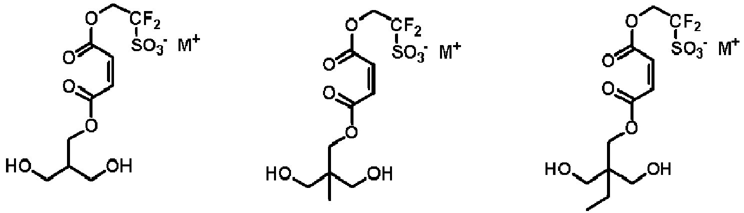

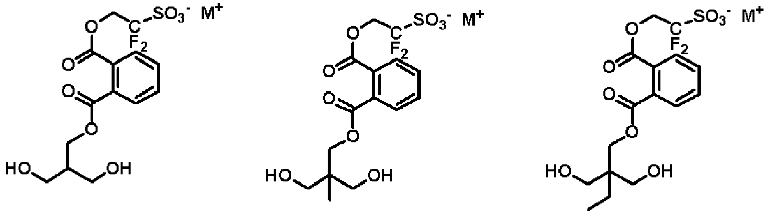

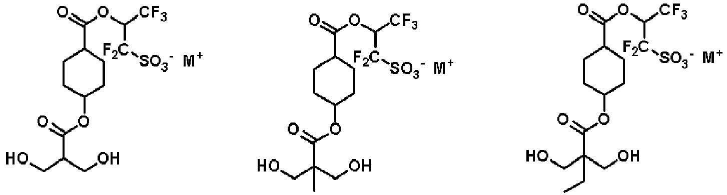

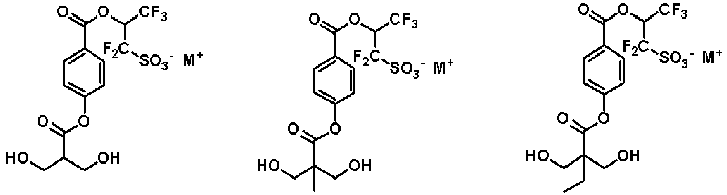

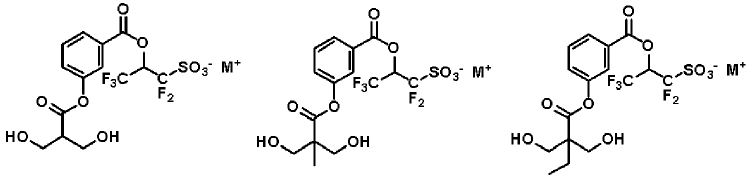

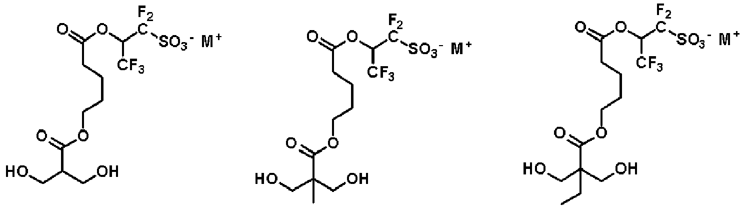

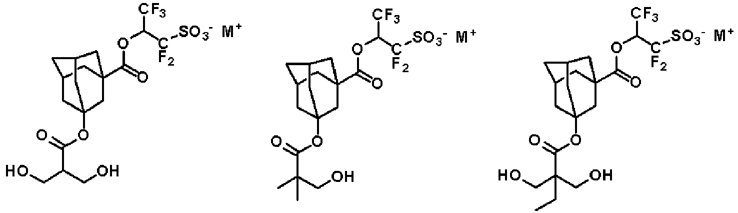

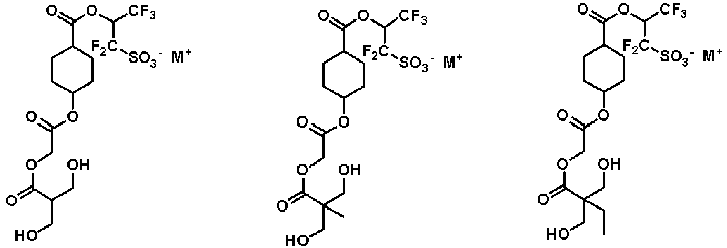

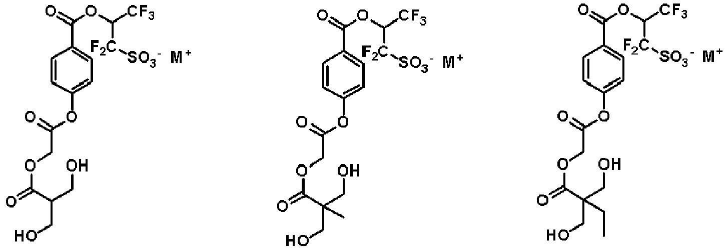

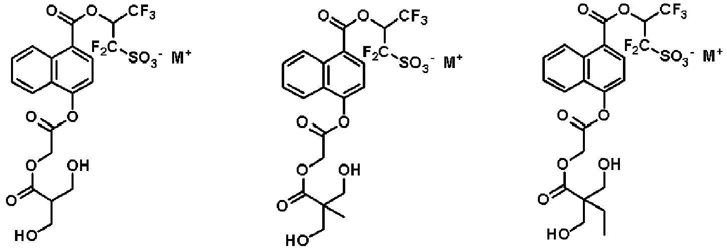

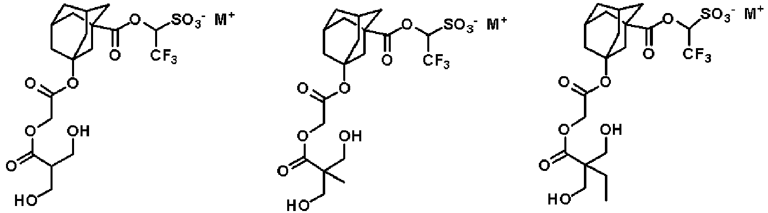

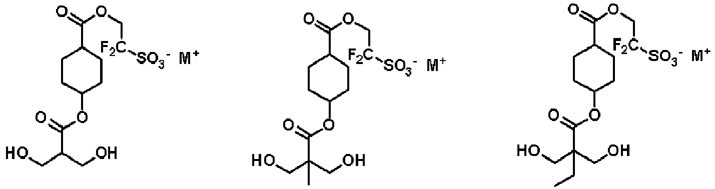

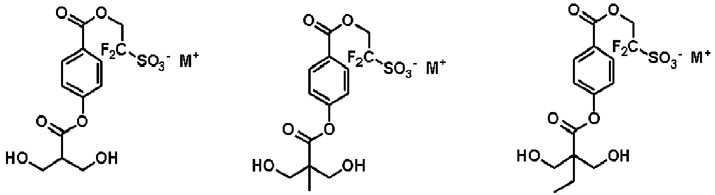

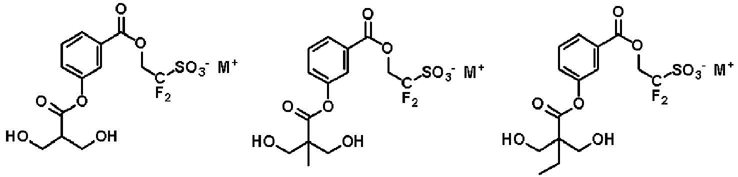

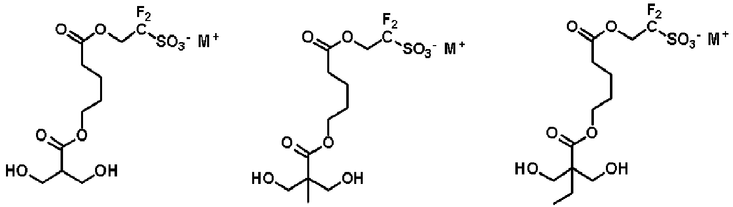

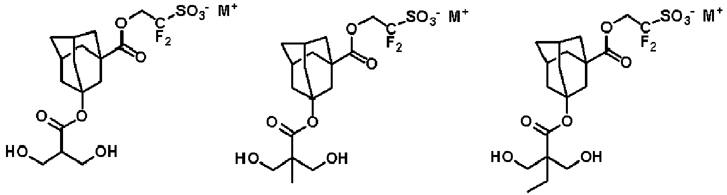

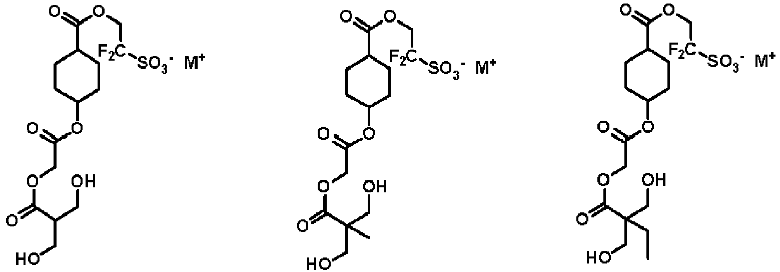

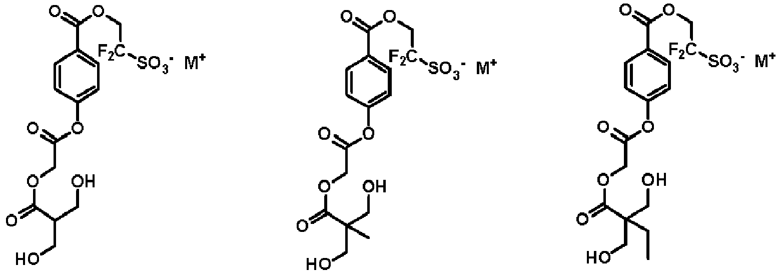

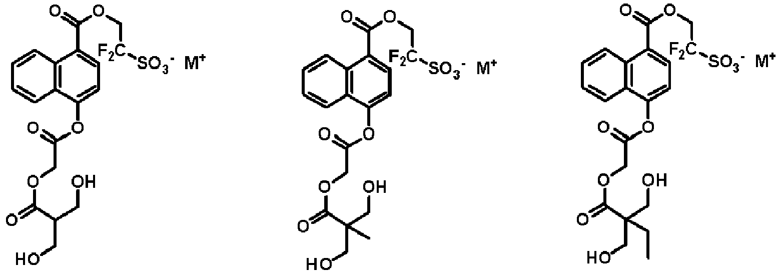

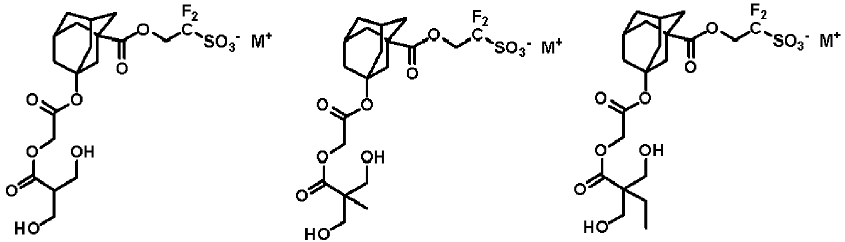

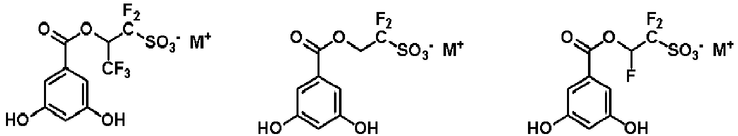

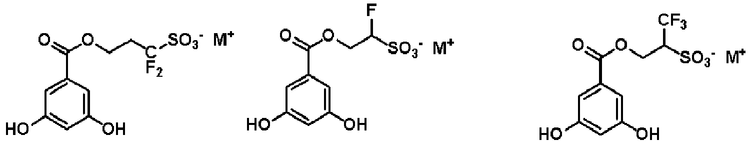

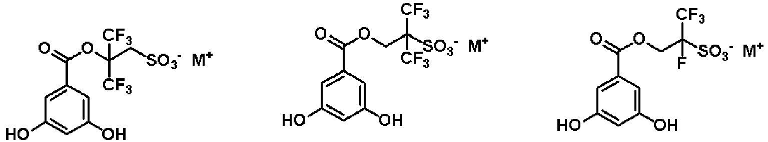

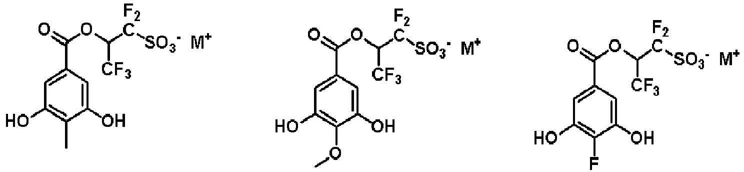

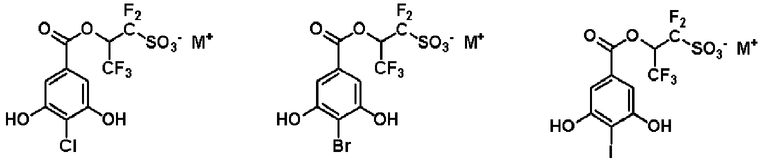

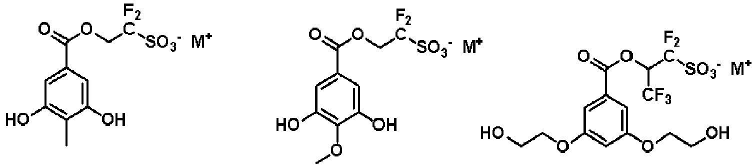

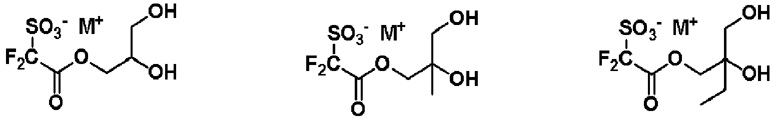

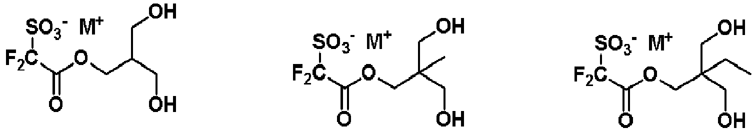

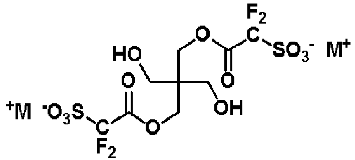

Specific examples of the monomer having a dihydroxy group represented by the general formula a6-1 include the following monomers.

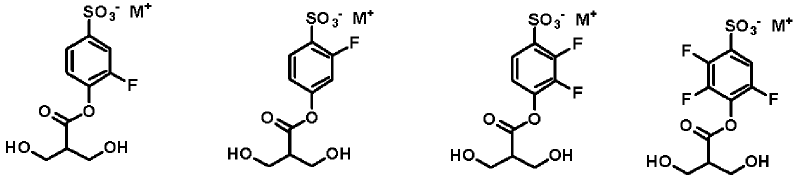

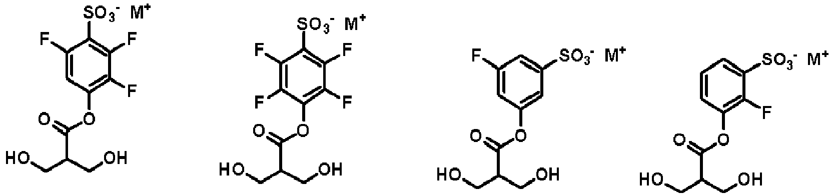

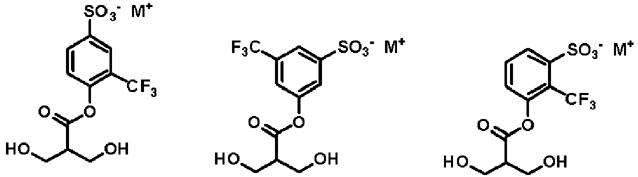

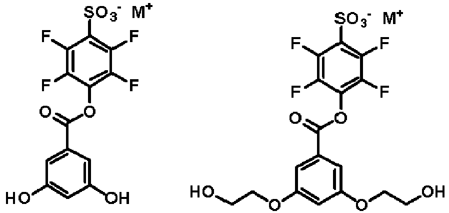

The repeating unit-A7 can be obtained by a reaction between: a monomer having a dihydroxy group represented by the following general formula a7-1; and a compound having an isocyanate group.

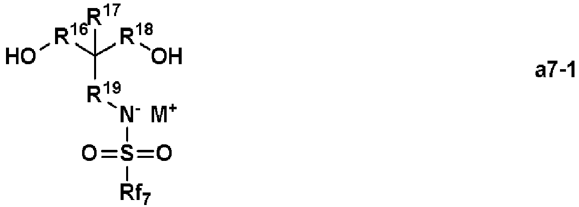

In the formula, R¹⁶ to R¹⁹, Rf₇, and M⁺ are as above.

Specific examples of the monomer having a dihydroxy group represented by the general formula a7-1 include the following monomers.

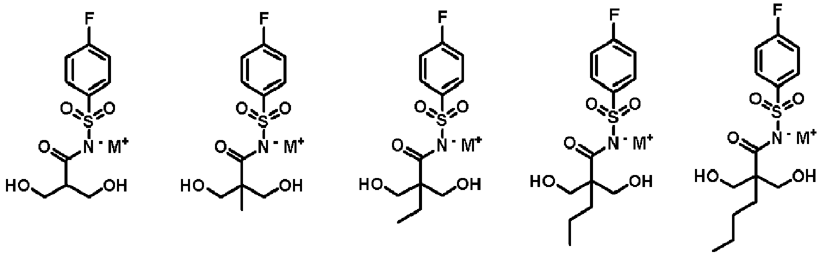

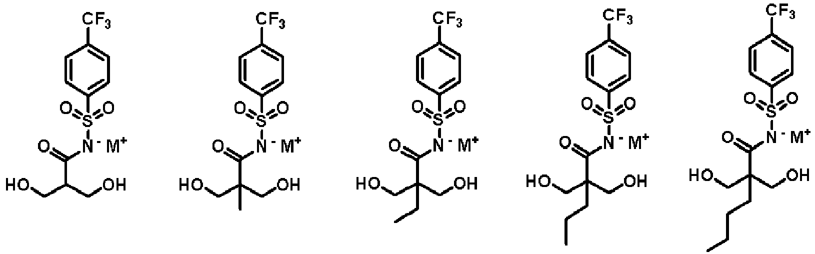

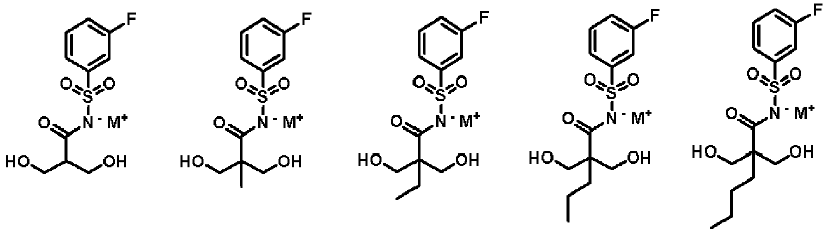

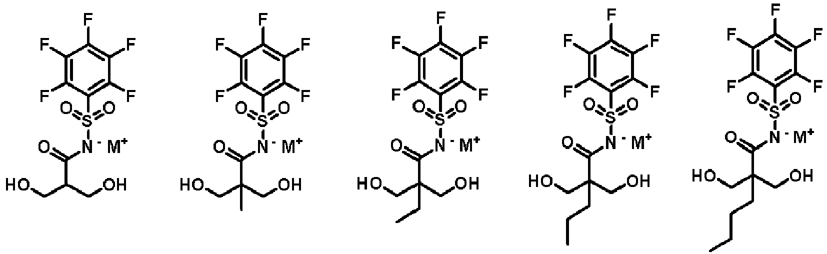

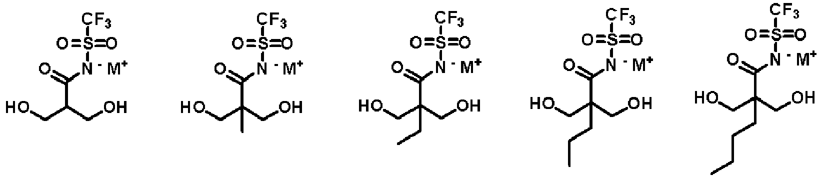

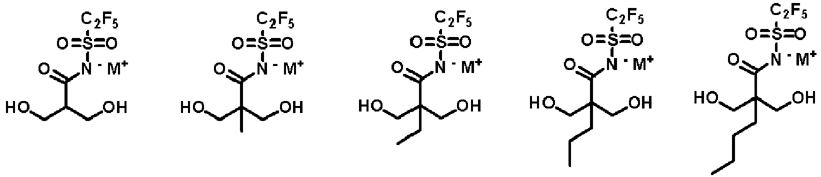

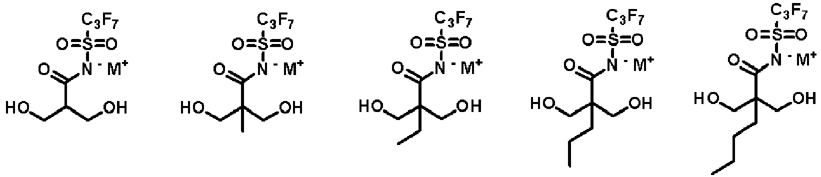

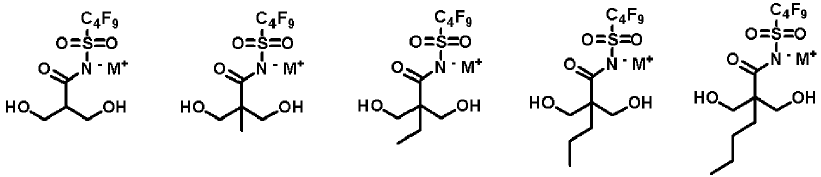

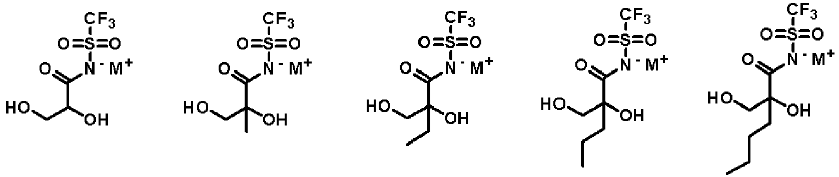

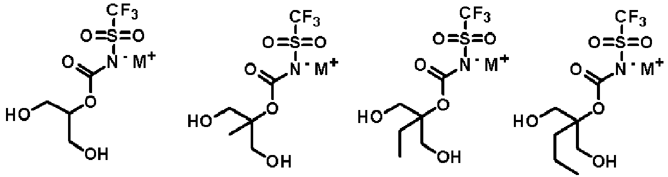

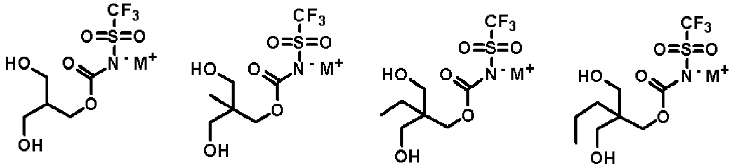

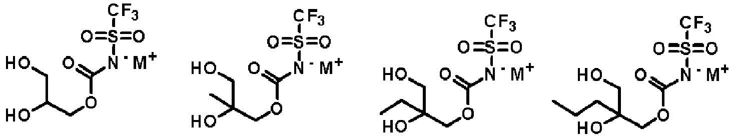

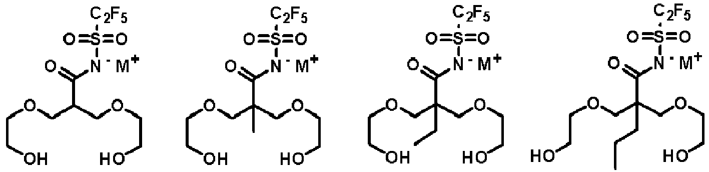

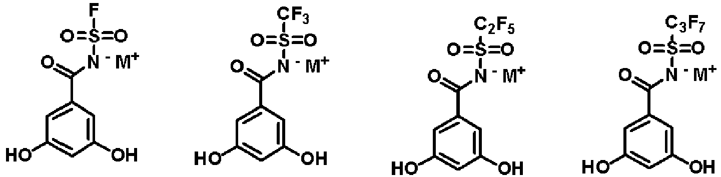

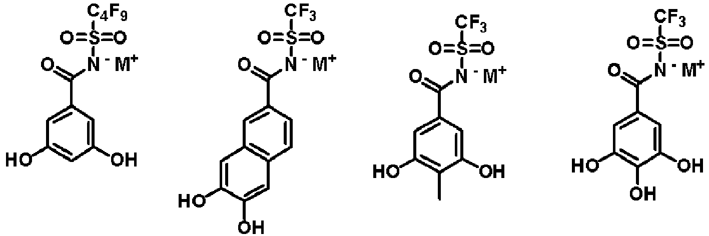

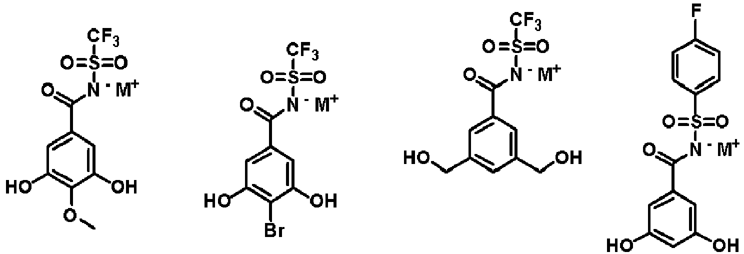

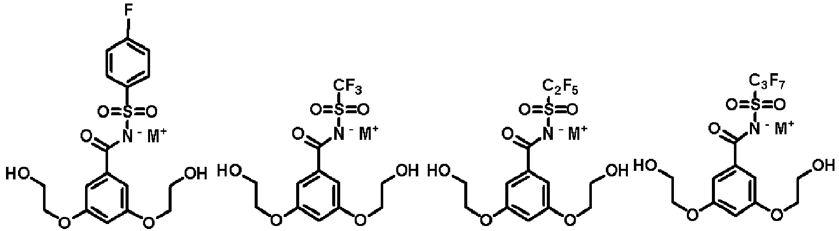

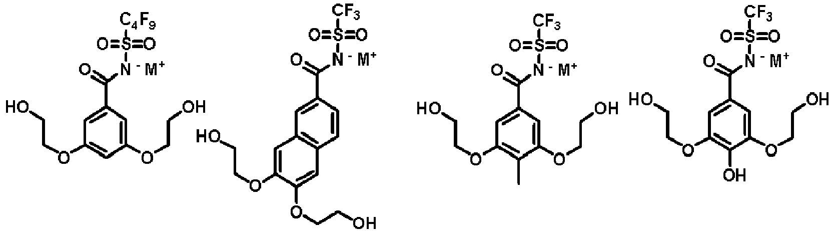

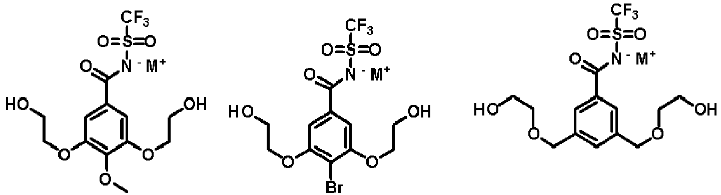

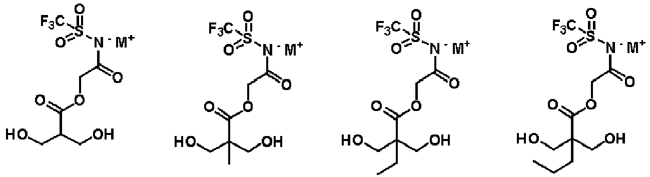

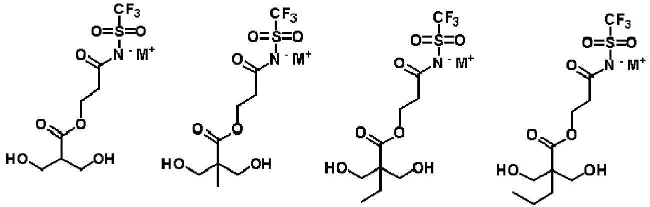

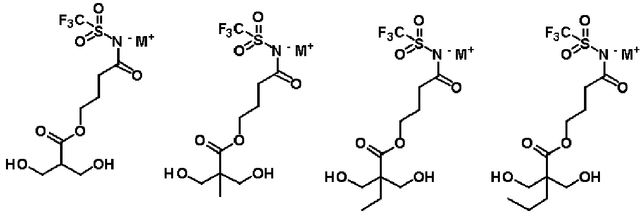

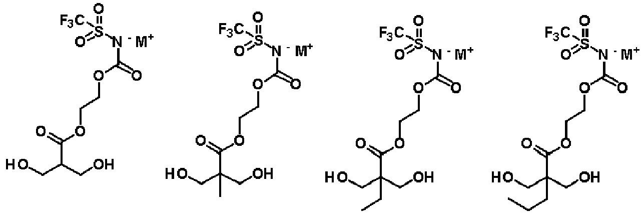

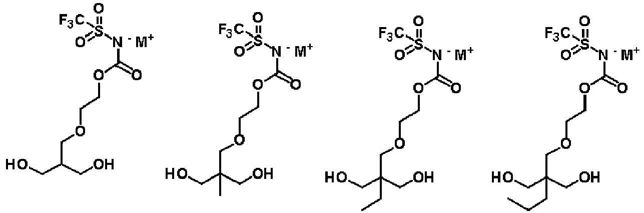

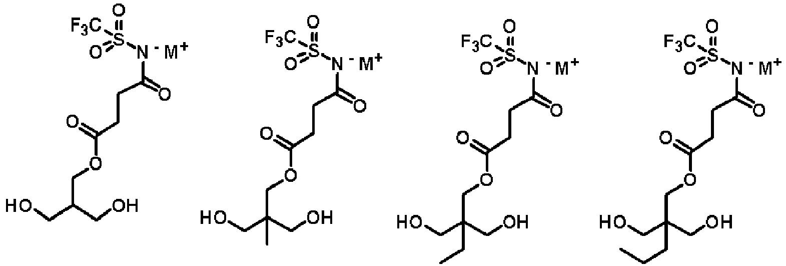

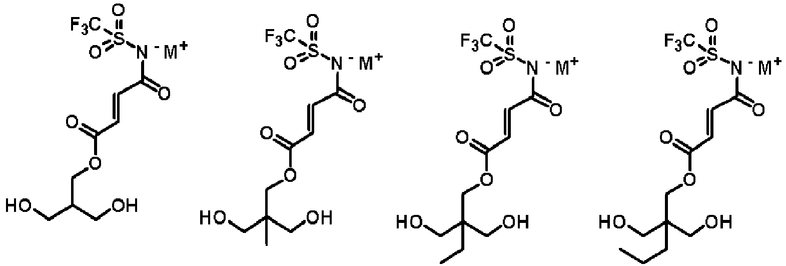

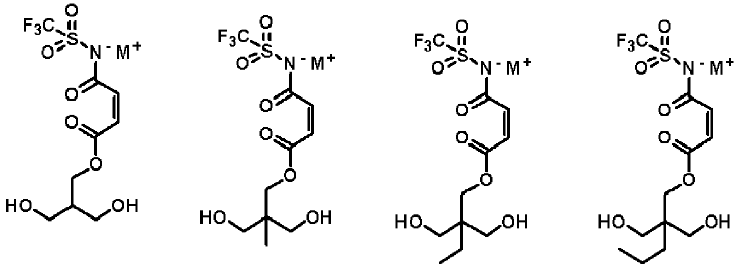

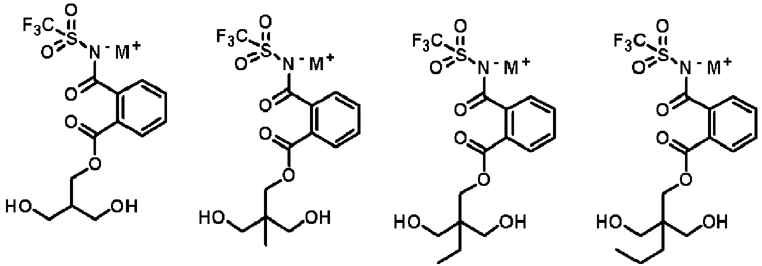

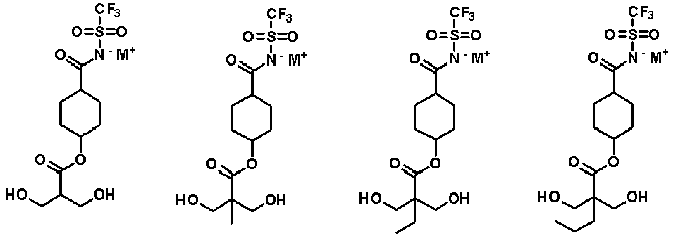

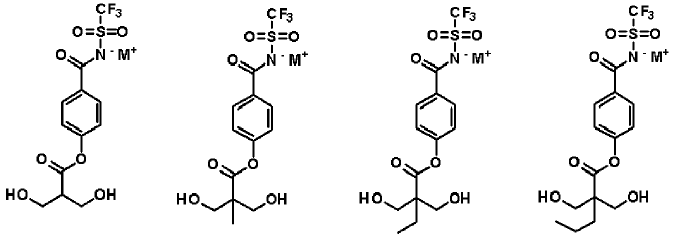

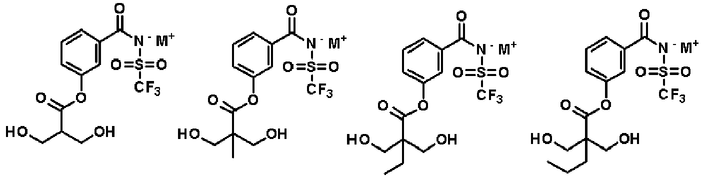

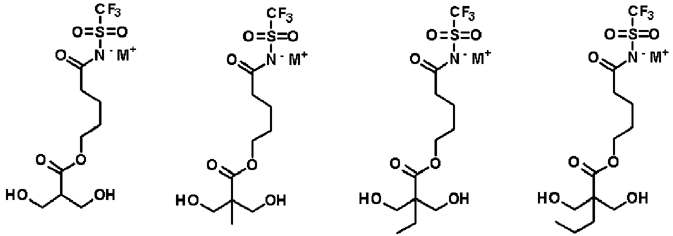

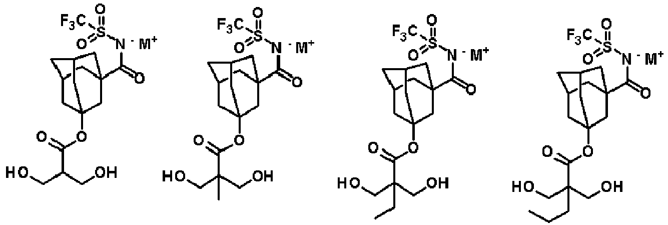

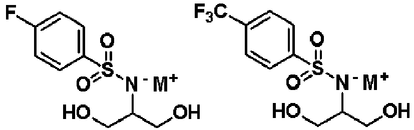

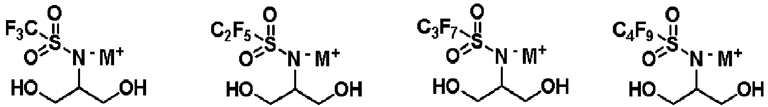

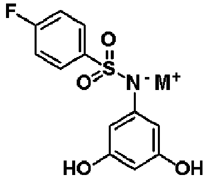

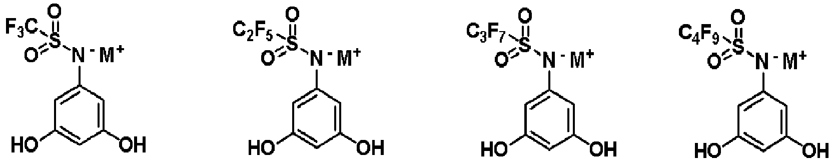

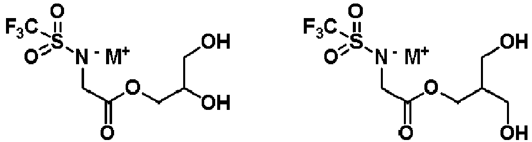

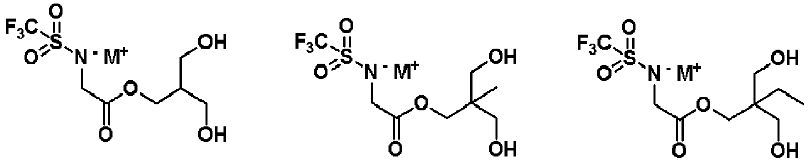

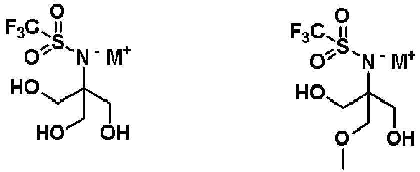

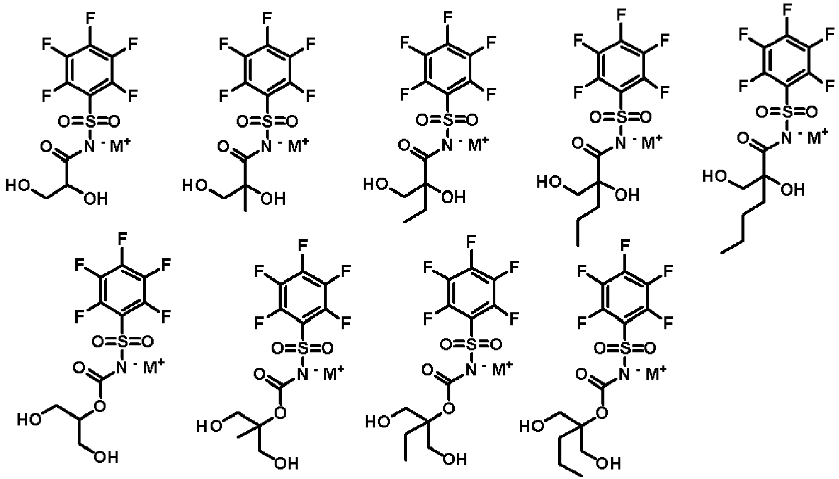

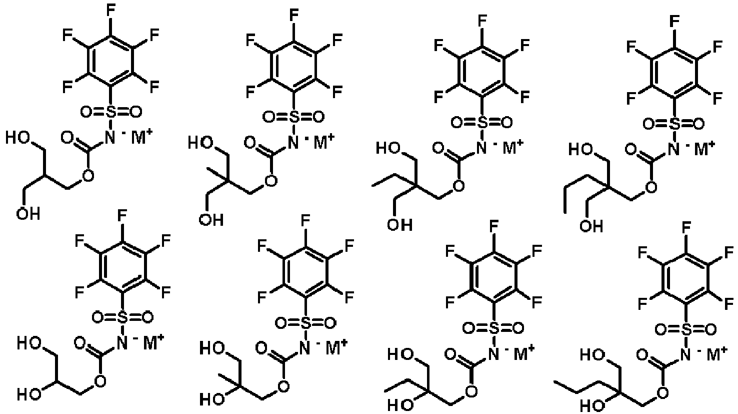

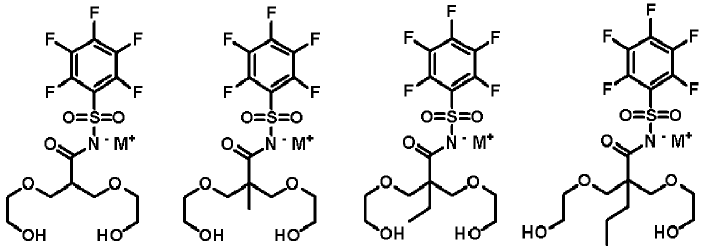

The ionic polymer (A) having the repeating unit-A5, -A6, or -A7 represented by the general formulae (4) can be obtained by a reaction between: the monomer having a dihydroxy group represented by the general formula a5-1, a6-1, or a7-1; and a compound having an isocyanate group. Examples of the compound having an isocyanate group include the following compounds.

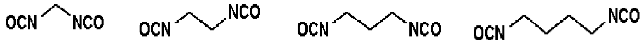

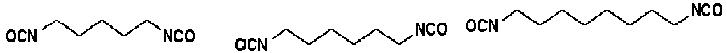

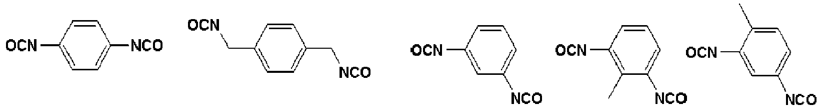

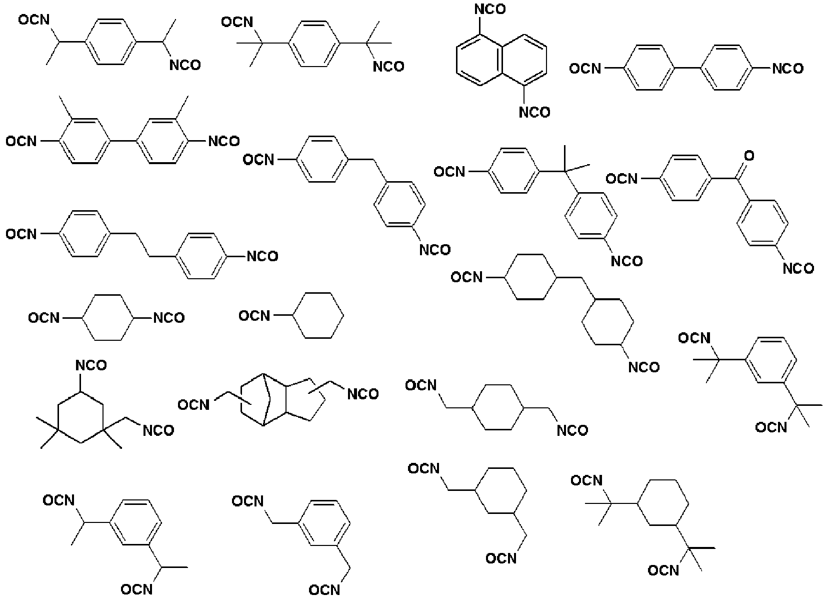

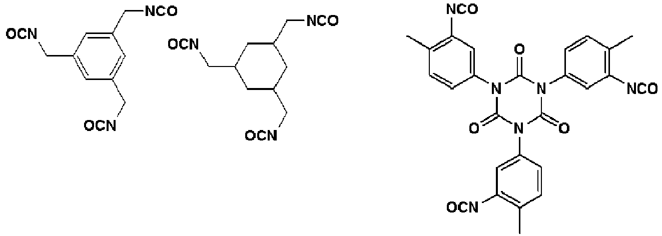

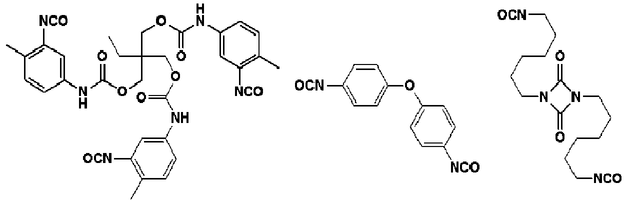

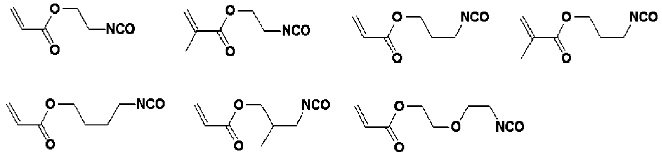

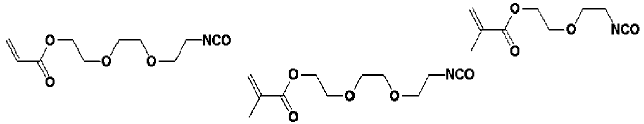

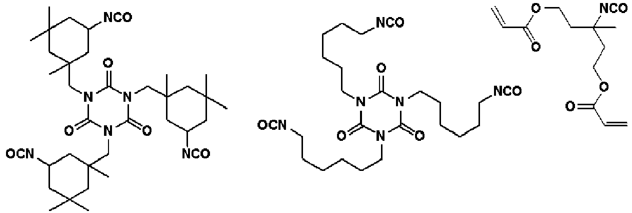

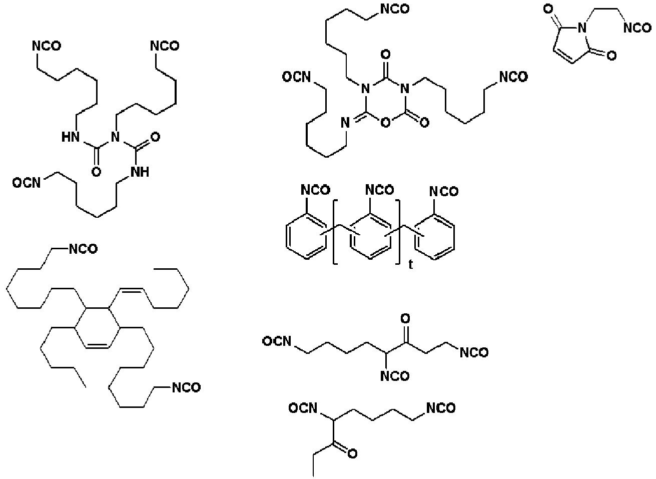

In the formula, "t" represents an integer of 1 or more.

Although the ionic polymer (A) having the repeating unit-A5, -A6, or -A7 represented by the general formulae (4) can be obtained by a reaction between: the monomer having a dihydroxy group; and the compound having an isocyanate group, the monomer may also be reacted with a compound having a hydroxy group other than those represented by the general formulae a5-1, a6-1, and a7-1.

Examples of the compound having a hydroxy group other than those represented by the general formulae a5-1, a6-1, and a7-1 include a polyether compound with a diol terminal represented by the following formulae.

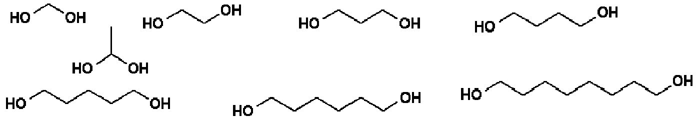

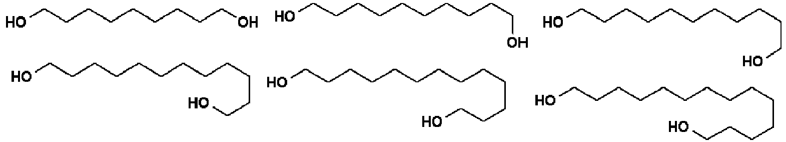

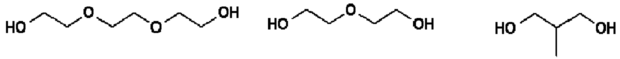

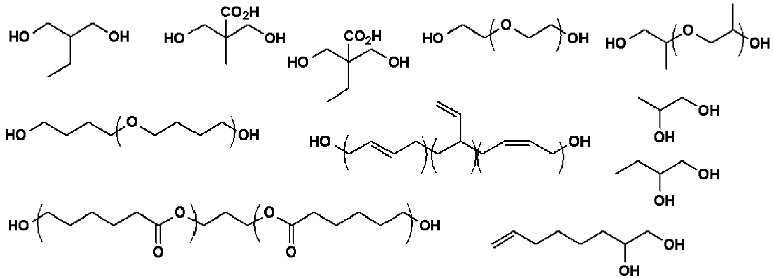

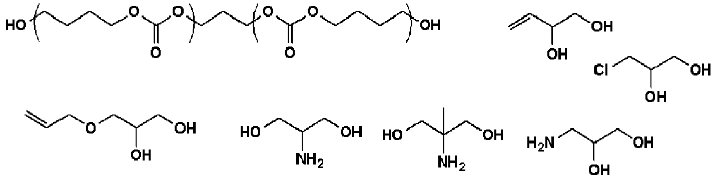

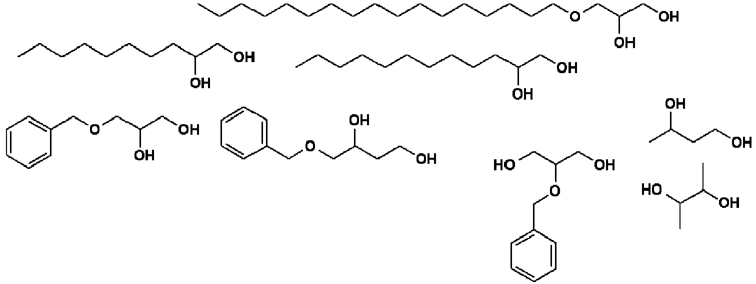

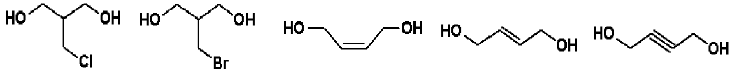

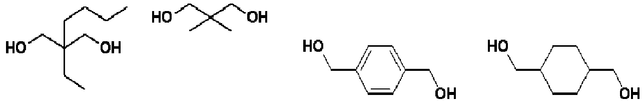

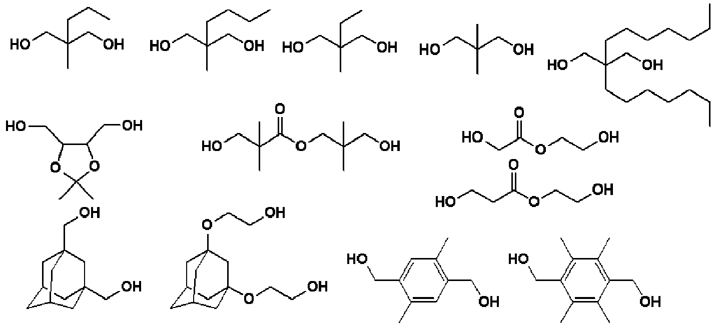

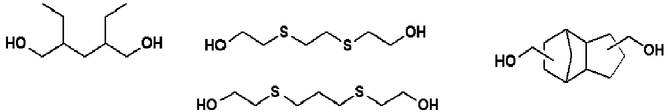

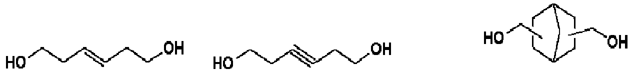

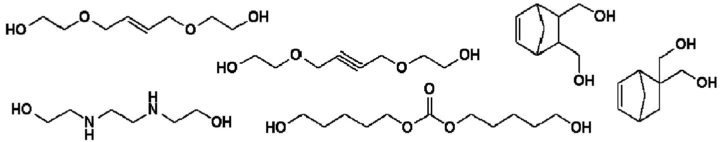

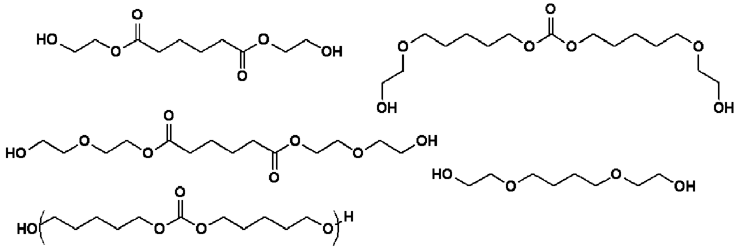

Here, the parenthesis represents a repeating unit.

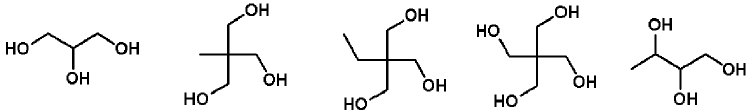

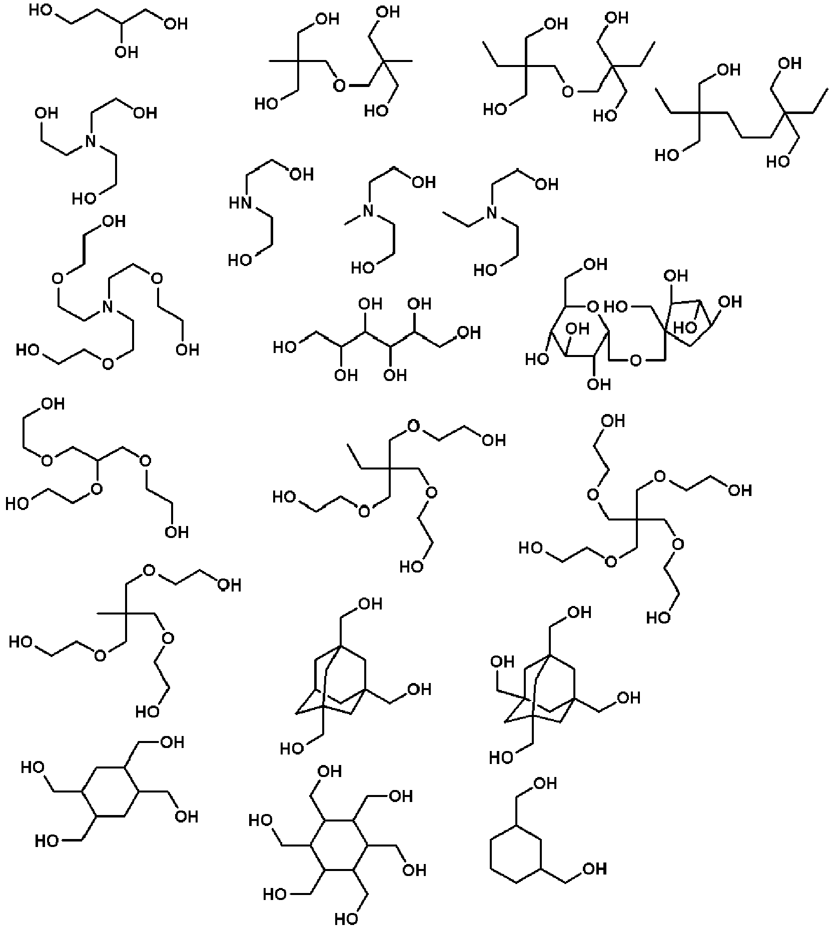

Examples of a copolymerizable diol compound for which a polyester compound with a diol terminal is a raw material include the following compounds.

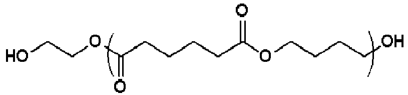

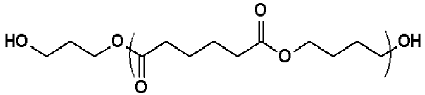

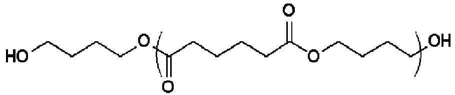

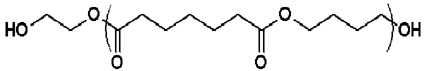

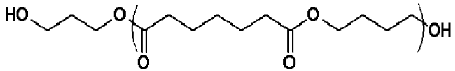

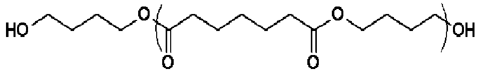

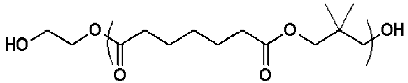

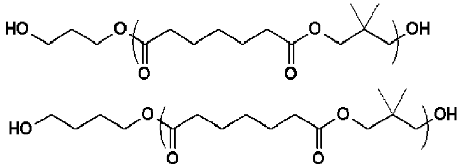

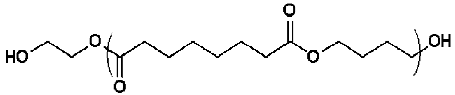

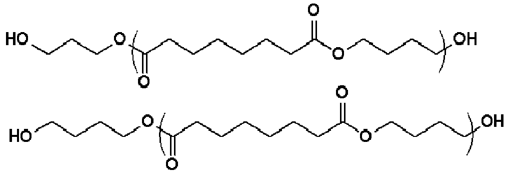

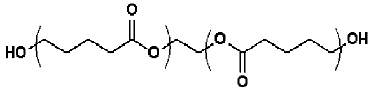

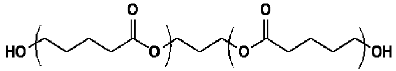

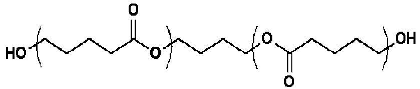

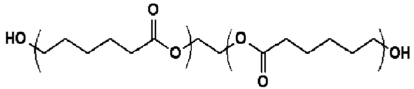

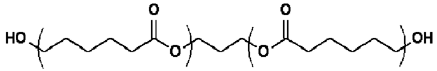

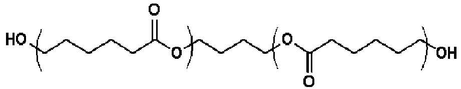

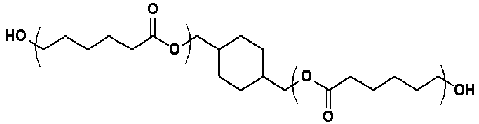

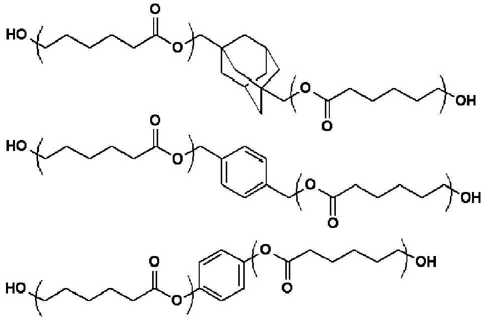

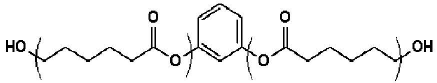

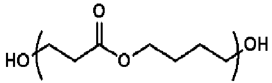

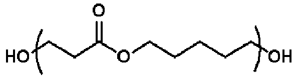

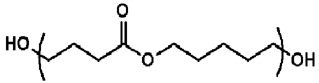

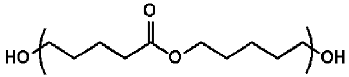

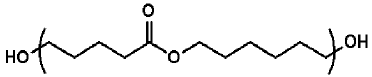

Here, the parenthesis represents a repeating unit.

The compound having an isocyanate group may also be copolymerized with a polycarbonate compound with a diol terminal as follows.

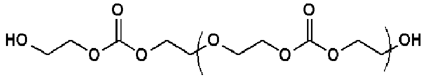

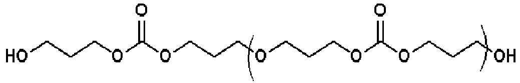

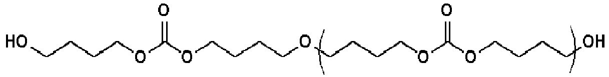

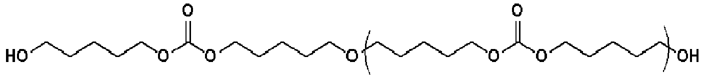

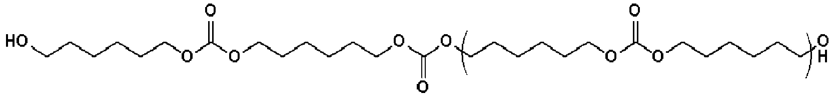

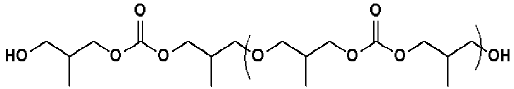

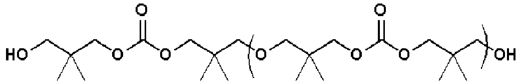

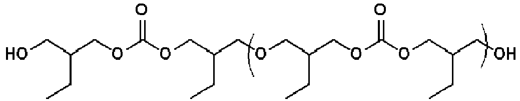

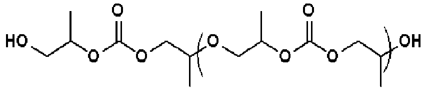

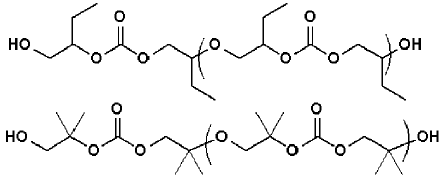

Here, the parenthesis represents a repeating unit.

Further, the compound having an isocyanate group may also be copolymerized with a diol compound having a fluoroalkyl group or a fluoroalkylene group as follows.

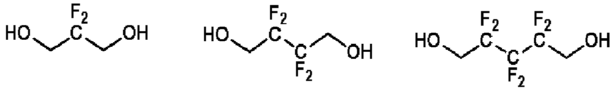

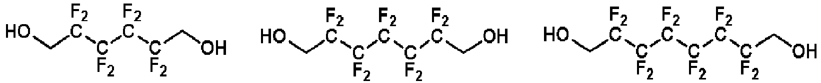

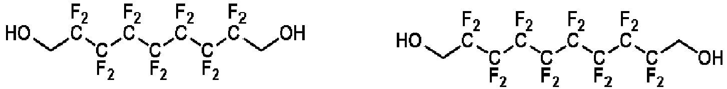

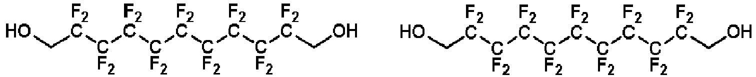

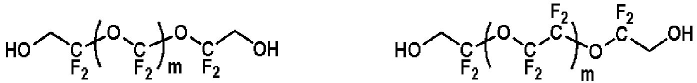

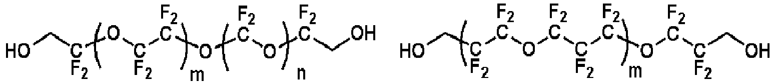

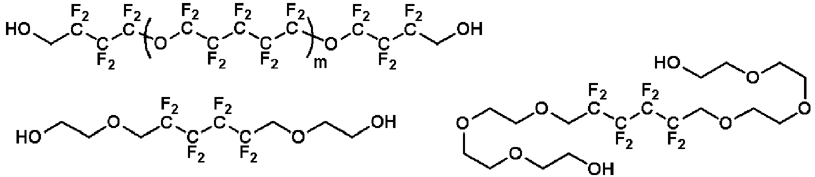

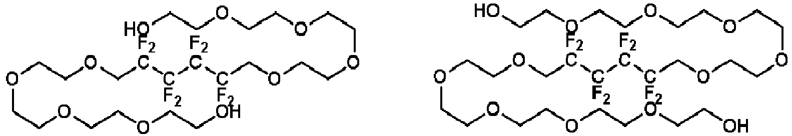

Here, "m" and "n" are within a range of 0 to 20.

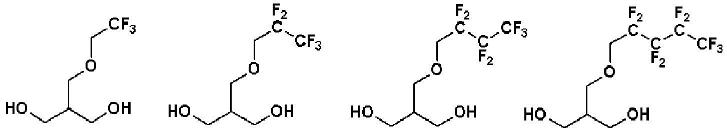

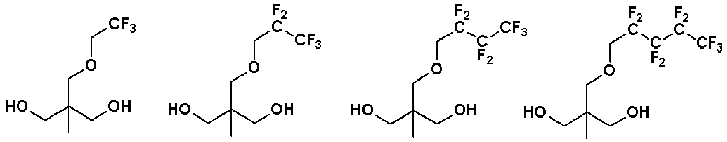

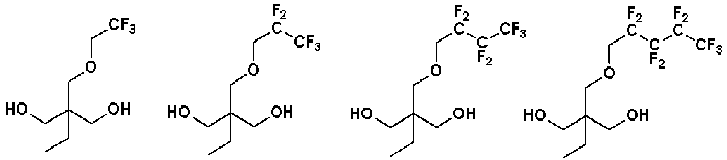

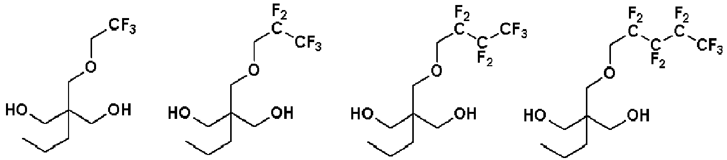

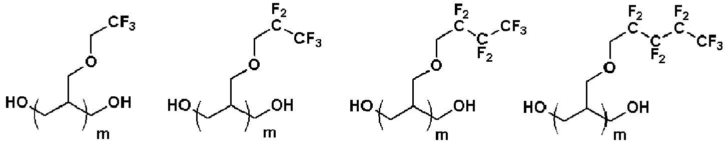

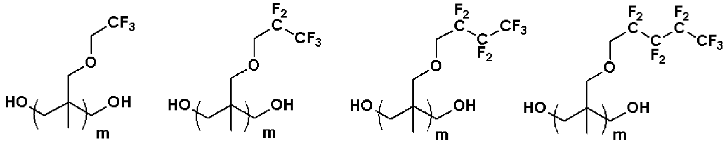

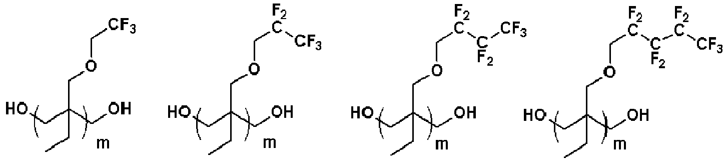

Here, "m" is within a range of 1 to 20.

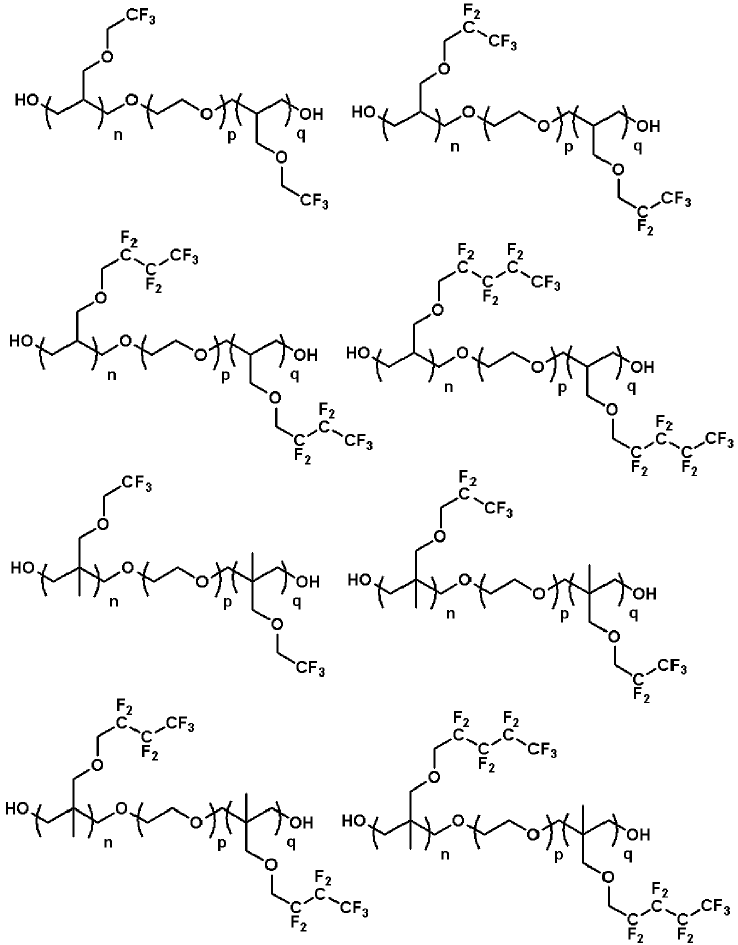

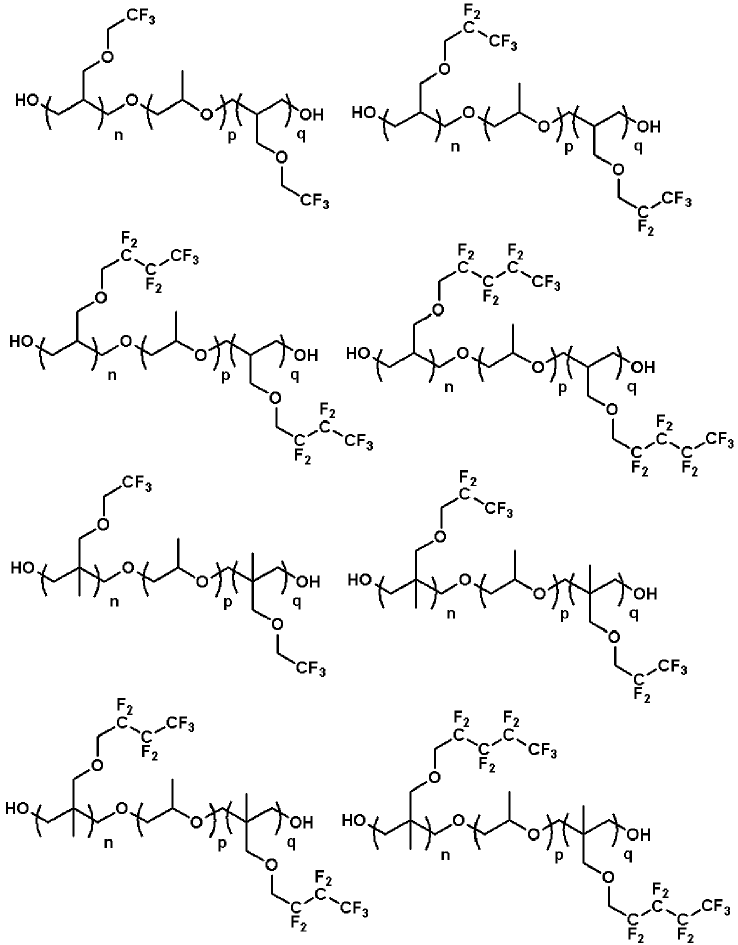

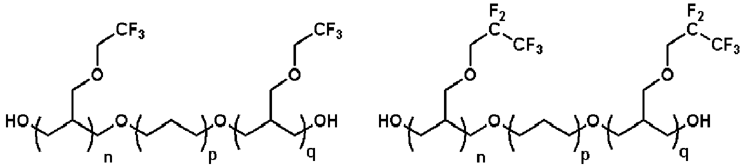

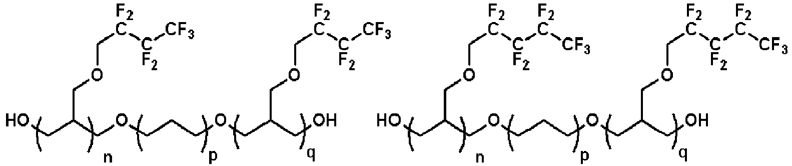

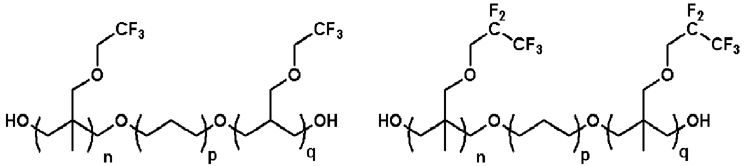

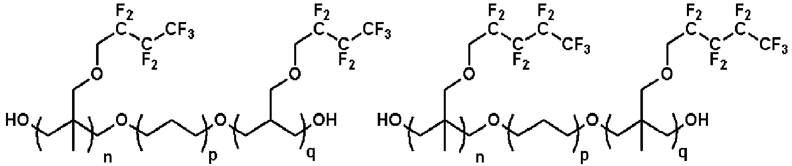

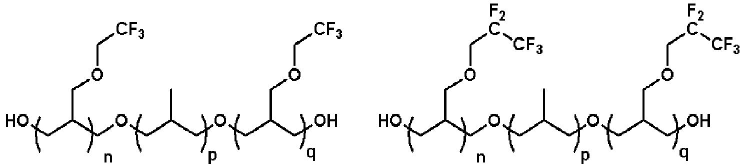

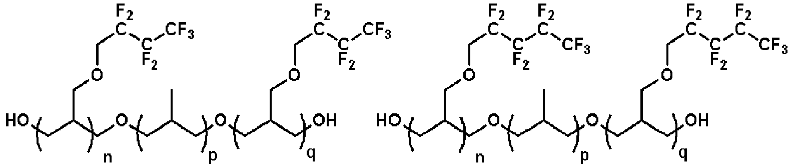

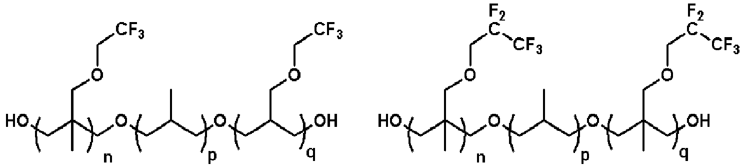

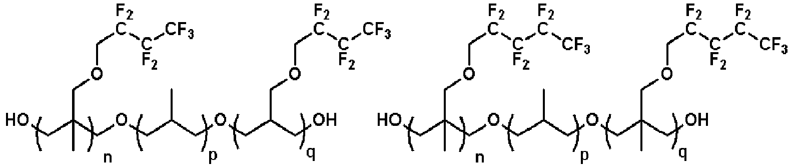

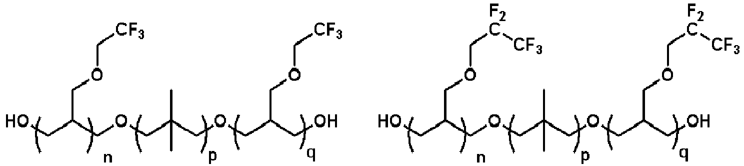

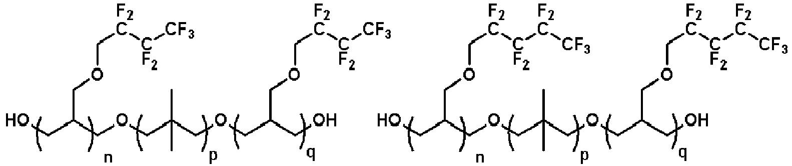

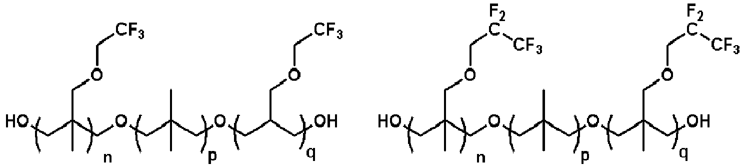

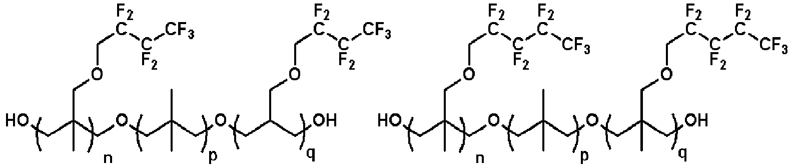

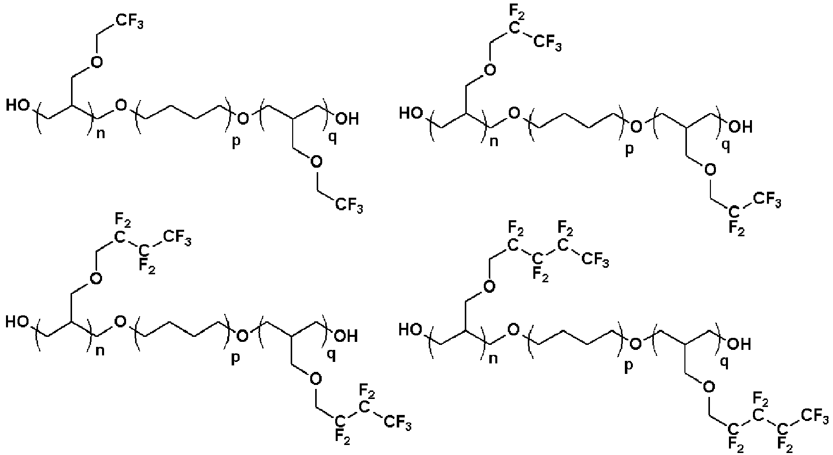

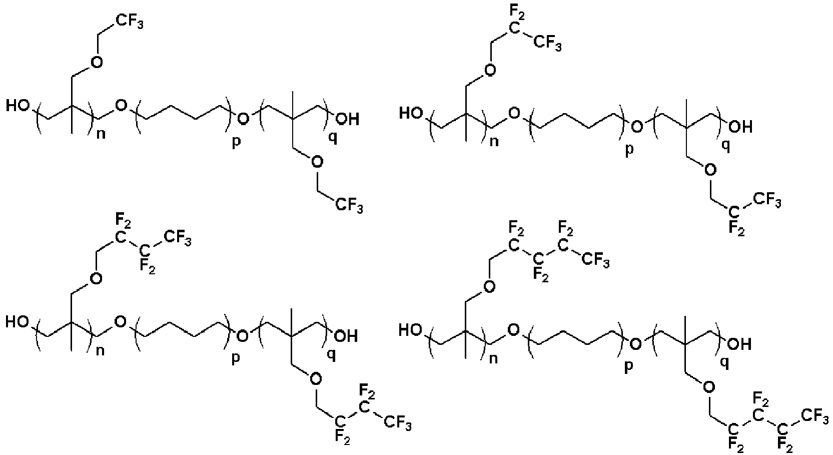

Here, "n" and "q" are within a range of 1 to 20, 2≤n+q≤21, and 1≤p≤40.

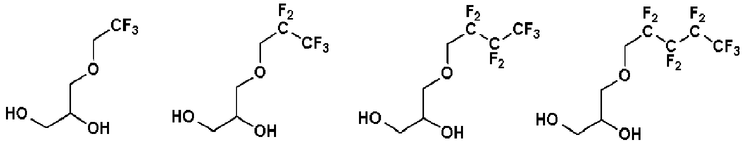

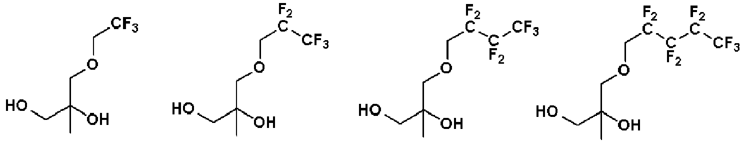

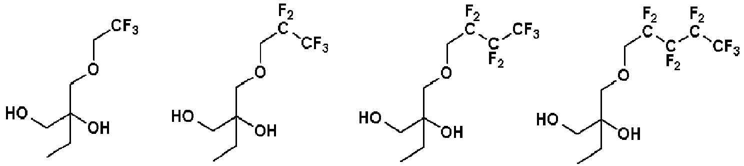

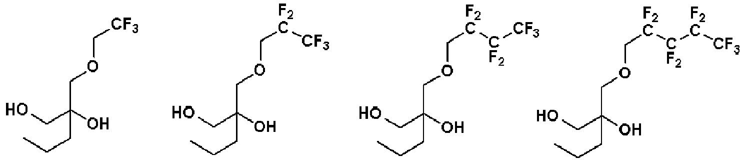

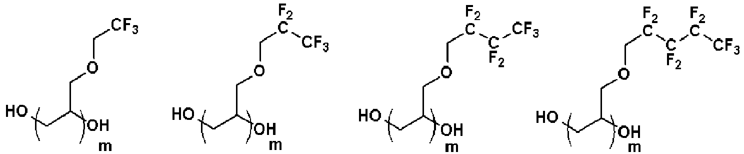

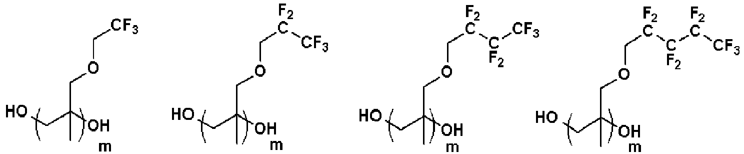

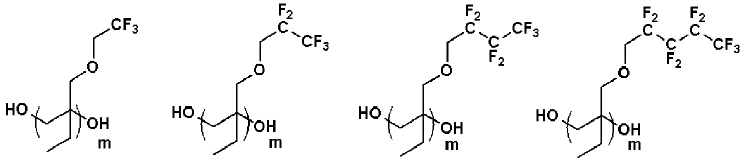

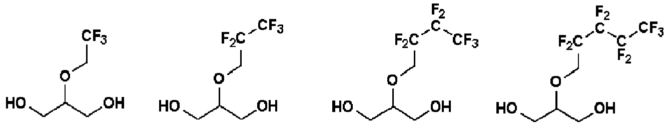

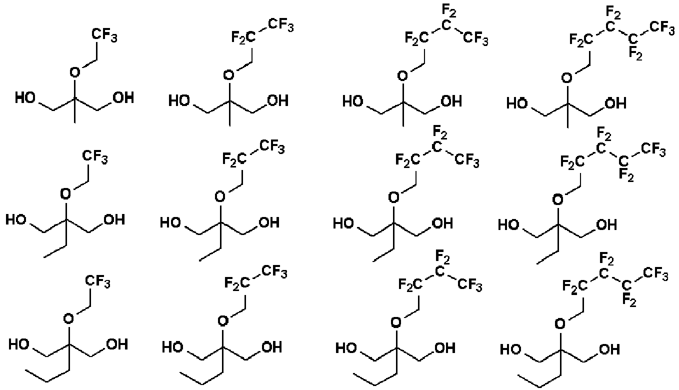

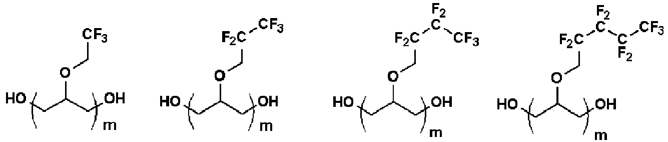

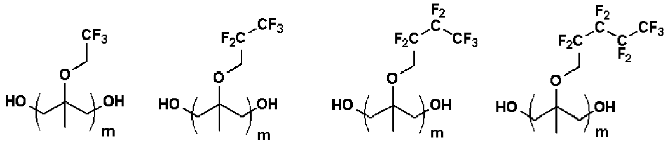

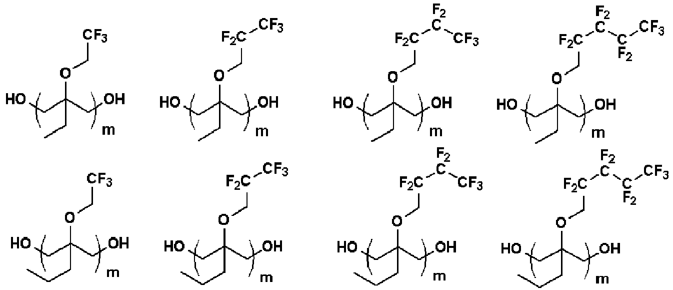

Here, "m" is within a range of 1 to 20.

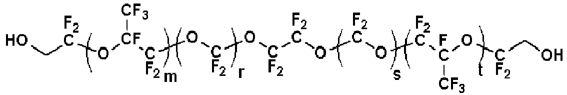

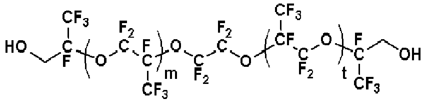

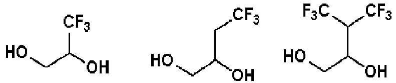

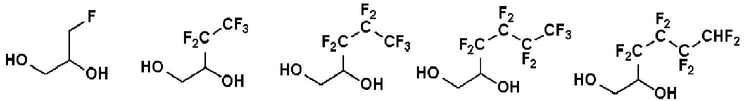

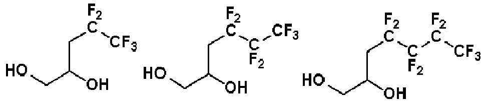

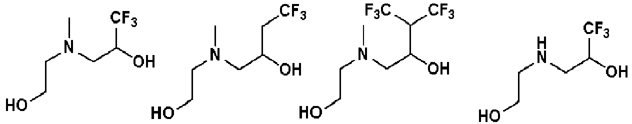

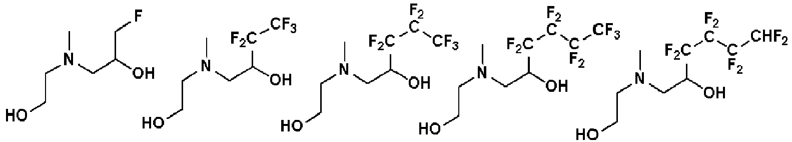

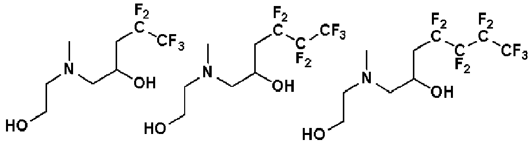

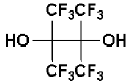

Here, "m", "r", "s", and "t" are within a range of 0 to 20.

The compound having an isocyanate group may also be copolymerized with a diol compound having a fluoroalcohol group or a sulfonamide group described in JP 2022-078861 A.

The compound having an isocyanate group may also be copolymerized with a diol compound having a silicon pendant as follows.

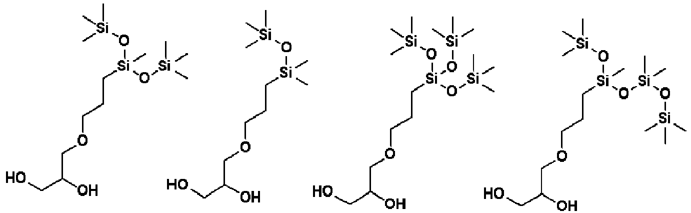

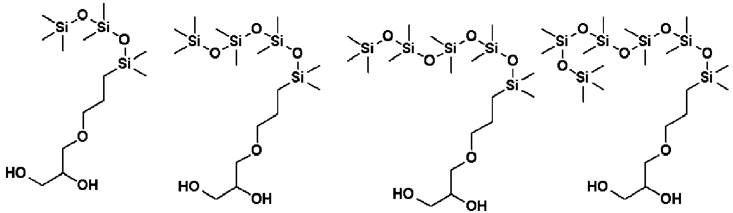

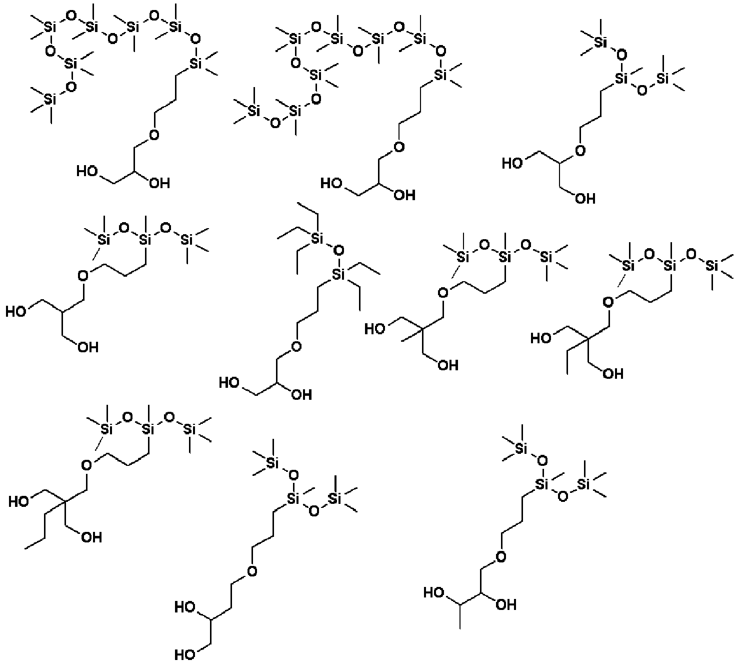

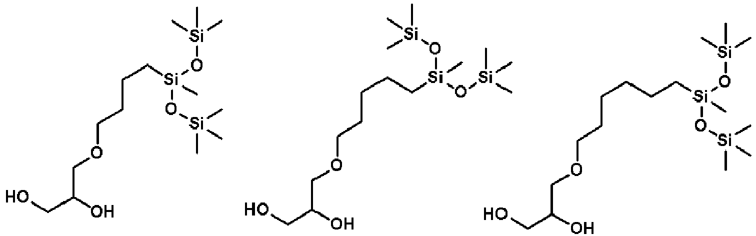

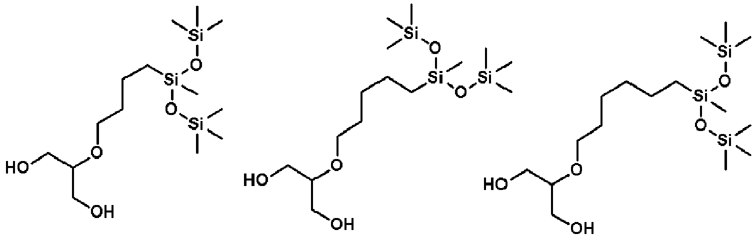

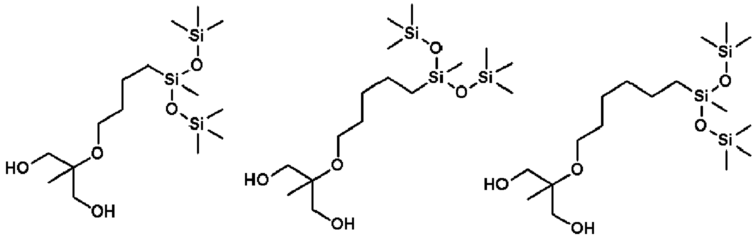

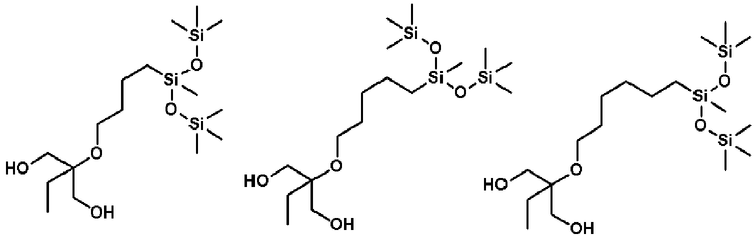

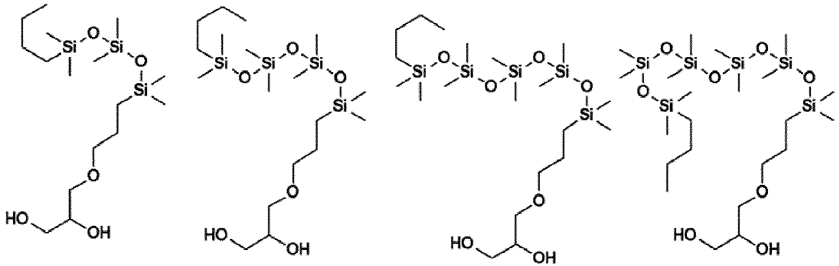

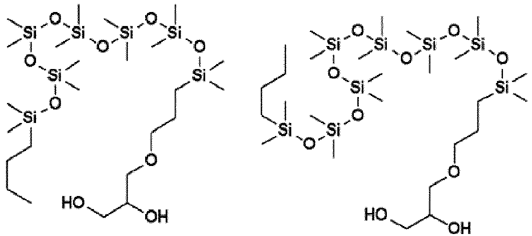

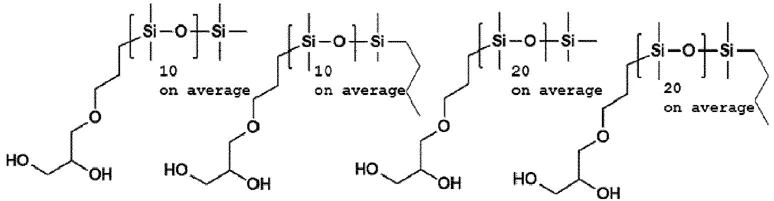

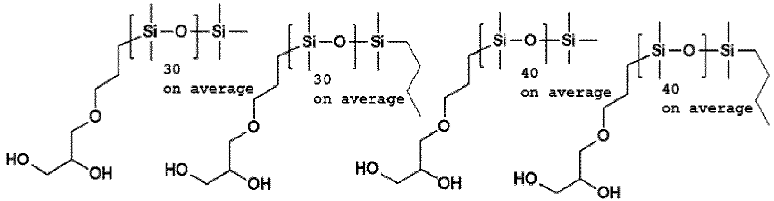

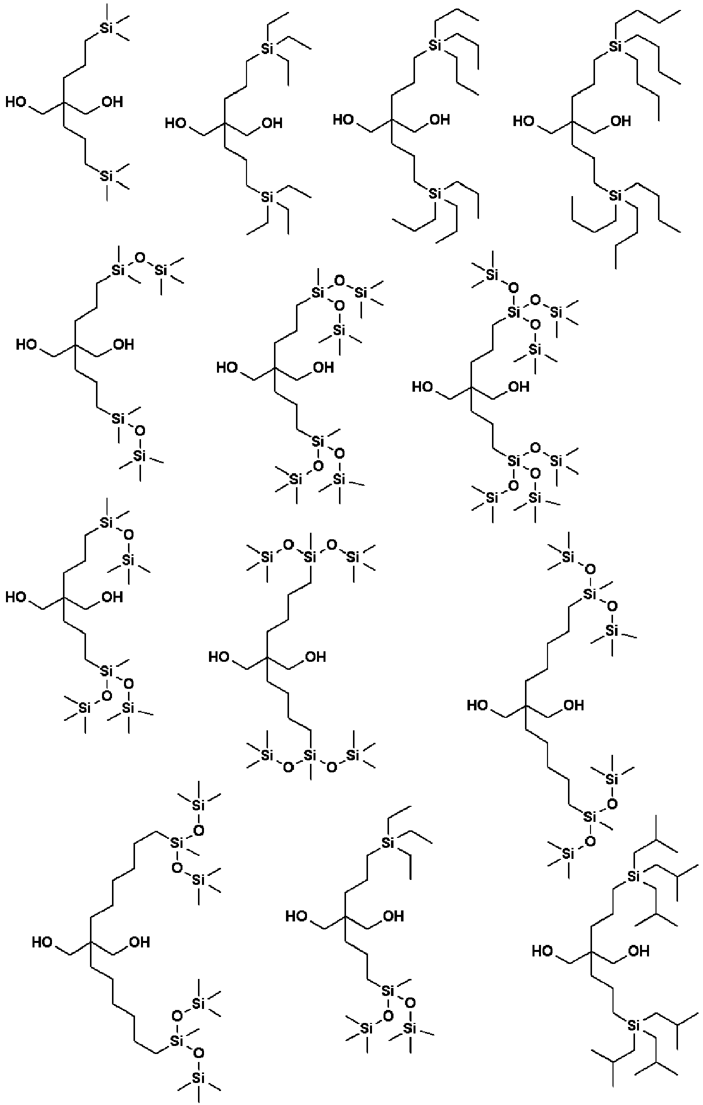

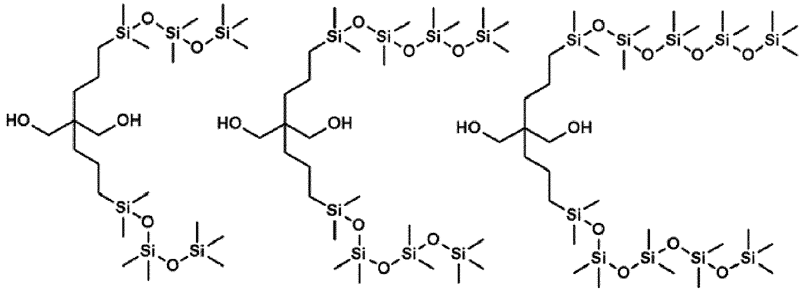

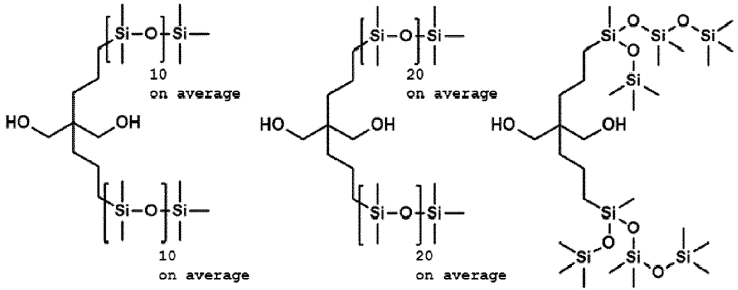

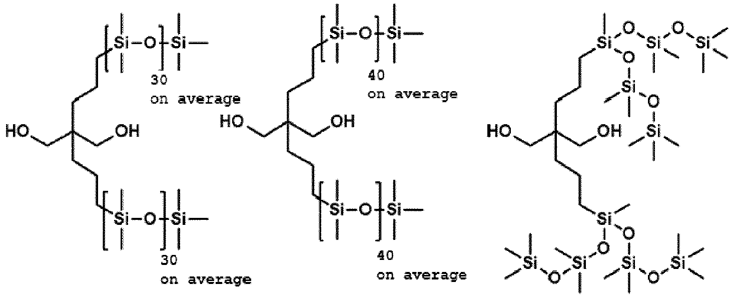

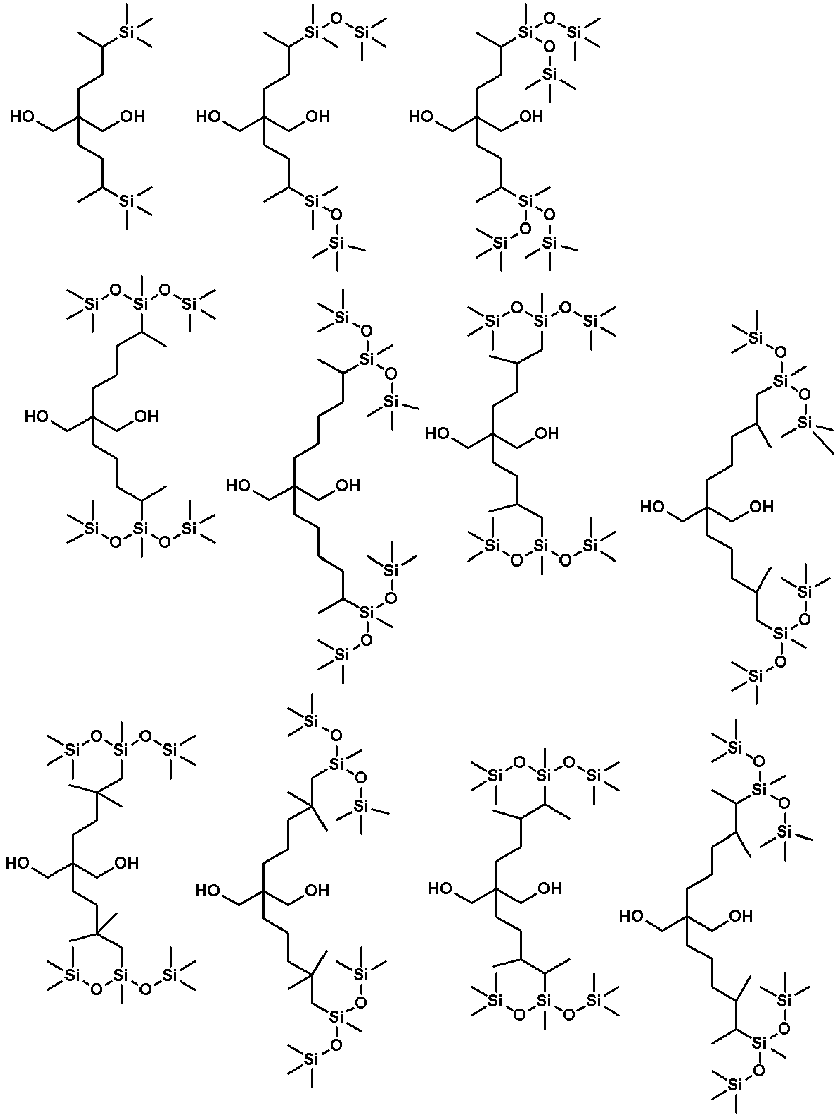

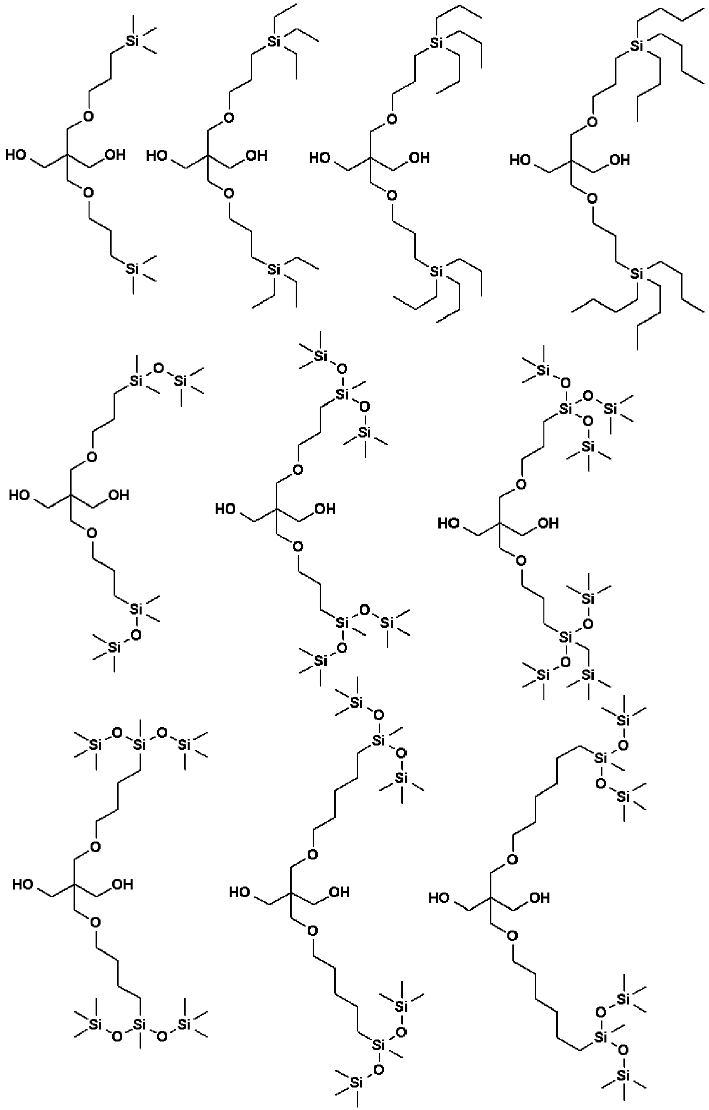

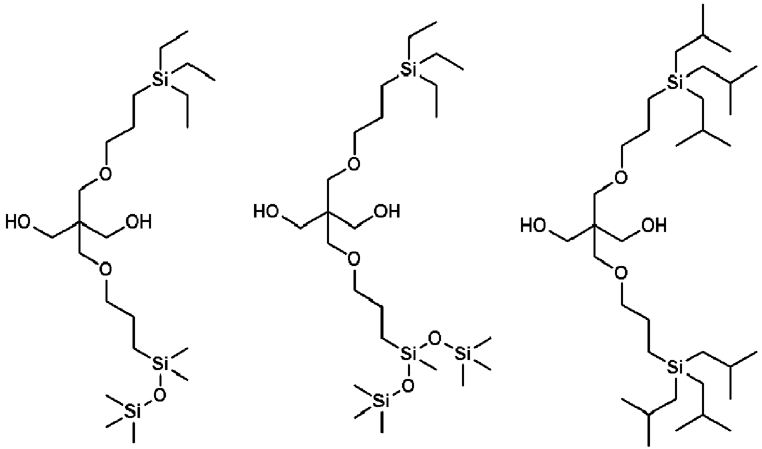

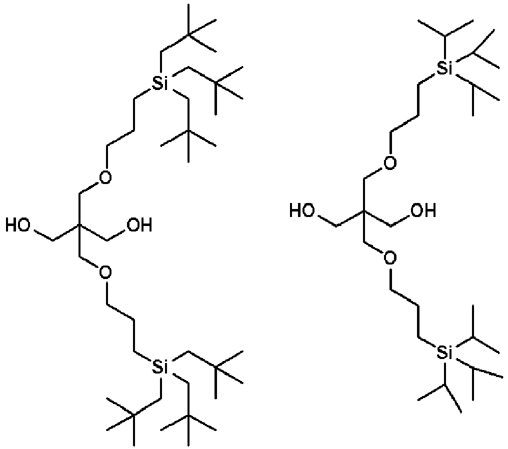

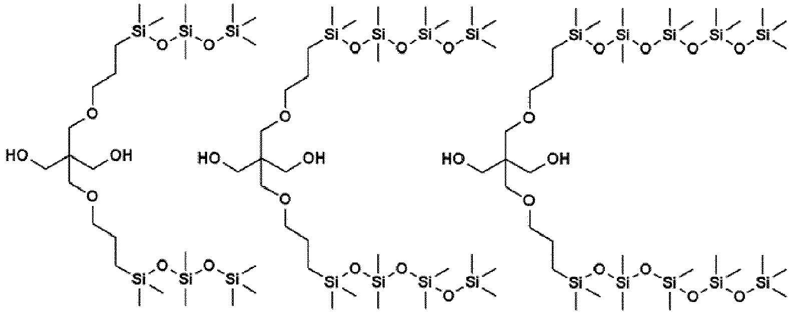

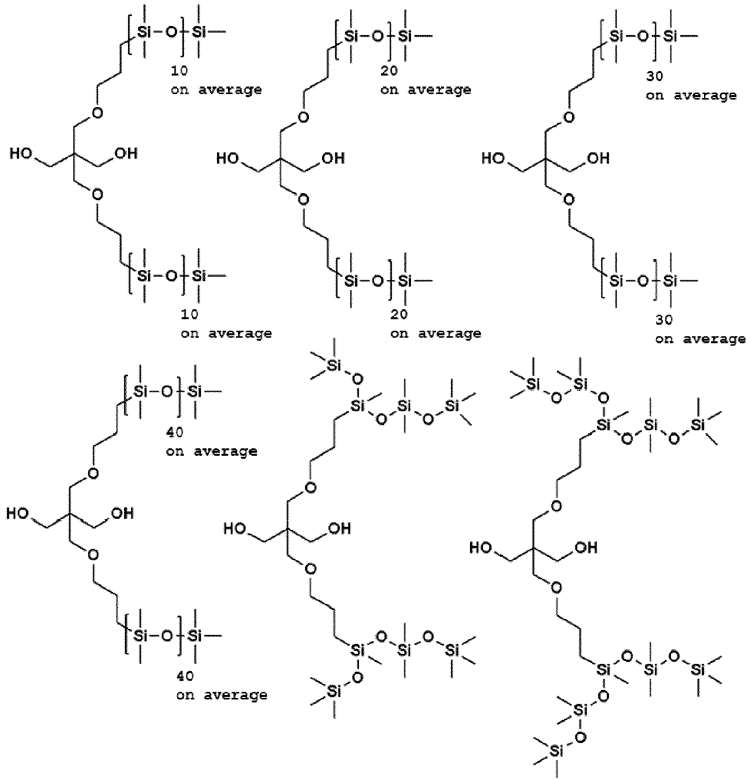

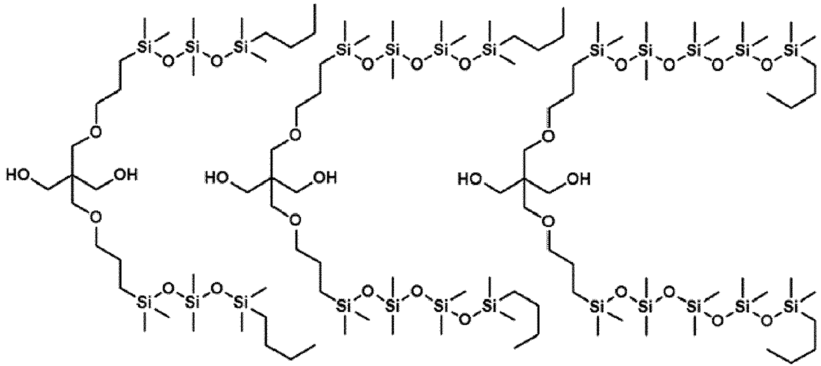

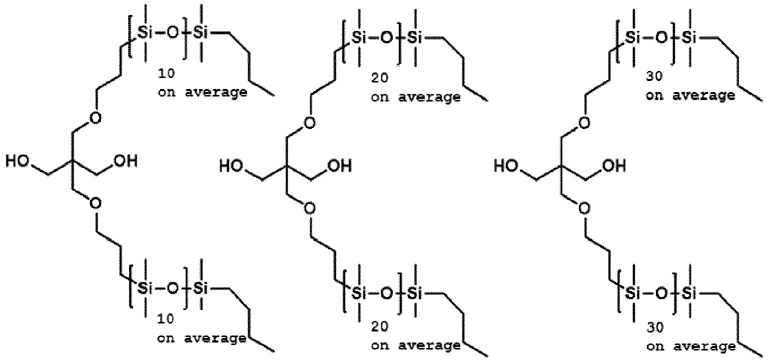

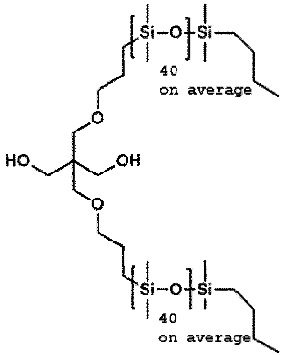

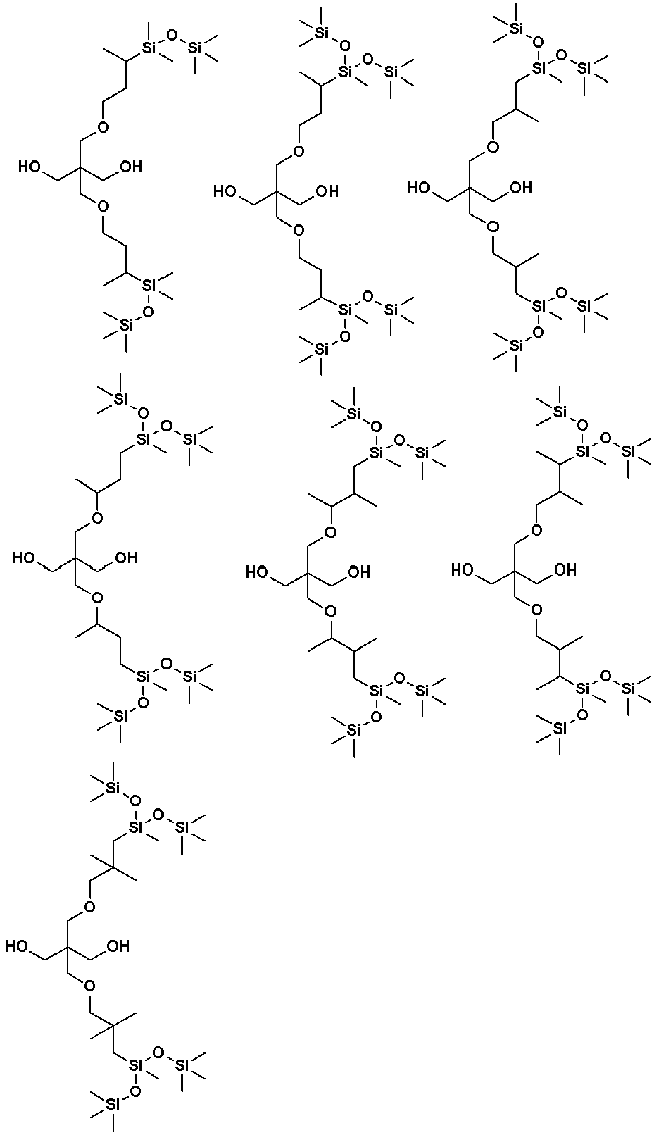

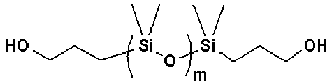

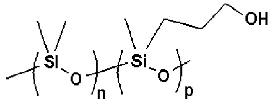

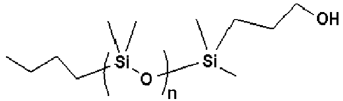

Here, "m", "n", and "p" are within a range of 1 to 20.

Among the above isocyanate compounds, in particular, the isocyanate compound having a (meth)acrylate group can be reacted with the compound represented by the general formula a5-1, a6-1, or a7-1 or with another hydroxy compound to obtain a compound having the (meth)acrylate group at a terminal. A hydroxy group having a (meth)acrylate group can also be reacted with an isocyanate compound to obtain the compound having the (meth)acrylate group at a terminal.

Among the above isocyanate compounds, in particular, the isocyanate compound having a maleimide group can be reacted with the compound represented by the general formula a5-1, a6-1, or a7-1 or with another hydroxy compound to obtain a compound having the maleimide group at a terminal. A hydroxy group having a maleimide group can also be reacted with an isocyanate compound to obtain the compound having the maleimide group at a terminal.

Since the isocyanate compound is highly reactive with the hydroxy-group-containing compound, it may be difficult to control the reactivity. In addition, since the isocyanate compound may be reacted with moisture in the atmosphere during storage to deactivate the isocyanate group, sufficient attention such as sufficient prevention from humidity is required for the storage. Thus, a compound having a block isocyanate group in which the isocyanate group is protected with a substituent may be used to prevent these phenomena.

The block isocyanate group is a group in which the block group is deprotected by heating to form the isocyanate group. Specific examples thereof include an isocyanate group substituted with an alcohol, phenol, a thioalcohol, an imine, a ketimine, an amine, a lactam, pyrazole, an oxime, a β-diketone, etc.

To lower a temperature for deprotecting the block isocyanate group, a catalyst may be added. Known as this catalyst are an organic tin such as dibutyltin dilaurate, a bismuth salt, a zinc carboxylate such as zinc 2-ethylhexanoate and zinc acetate, etc.

Specifically, JP 2012-152725 A describes that containing a carboxylic acid, a zinc α,β-unsaturated carboxylate as the catalyst for dissociating the block isocyanate can lower the temperature for the deprotection reaction.

As the ammonium ion constituting the ammonium salt, the repeating unit-a preferably contains an ammonium ion (ammonium cation) represented by the following general formula (3).

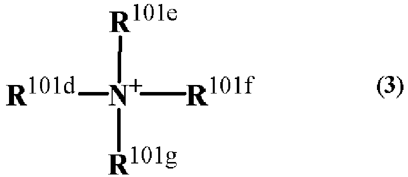

In the general formula (3), R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, optionally having one or more selected from an ether group, a carbonyl group, an ester group, a hydroxy group, a carboxyl group, an amino group, a nitro group, a sulfonyl group, a sulfinyl group, a halogen atom, and a sulfur atom; and R^{101d} and R^{101e}, or R^{101d}, R^{101e}, and R^{101f} optionally form a ring together with a nitrogen atom to which these groups are bonded, and when forming the ring, R^{101d} and R^{101e}, or R^{101d}, R^{101e}, and R^{101f} form an alkylene group having 3 to 10 carbon atoms or a heteroaromatic ring having the nitrogen atom in the general formula (3) in the ring.

Specific examples of the ammonium salt represented by the general formula (3) include the following ions.

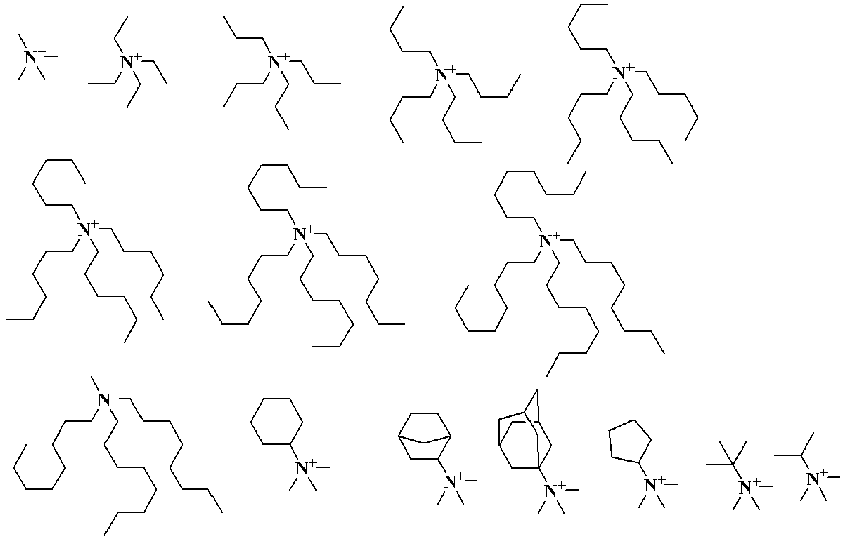

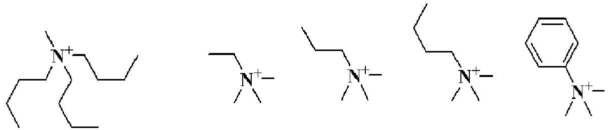

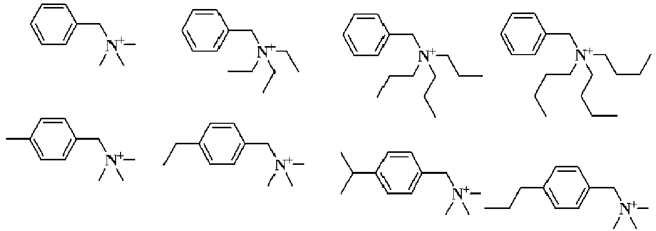

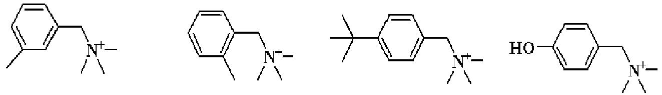

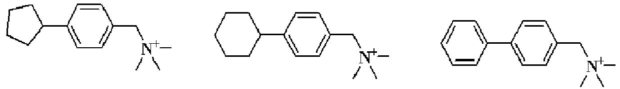

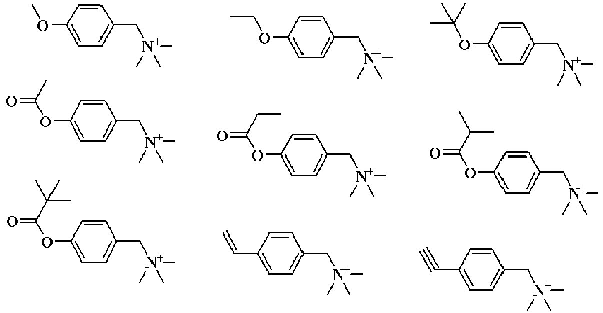

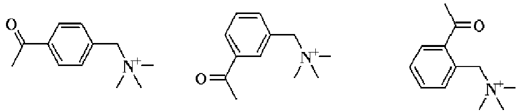

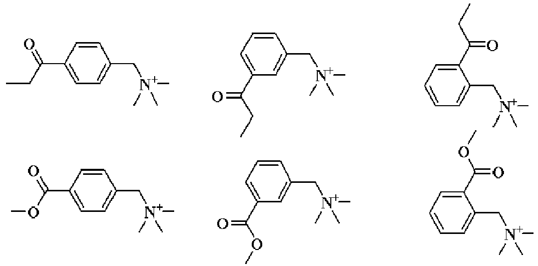

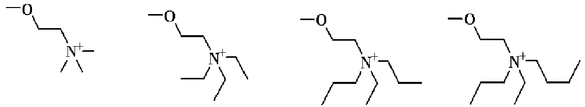

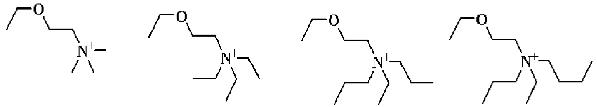

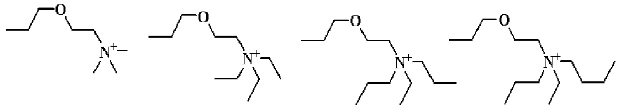

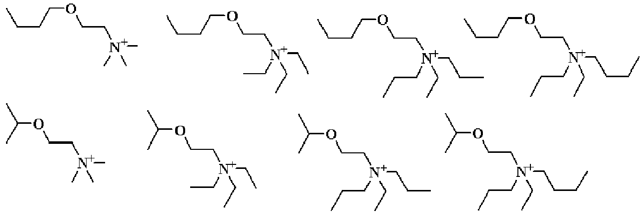

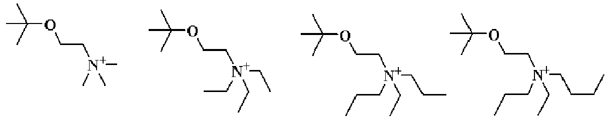

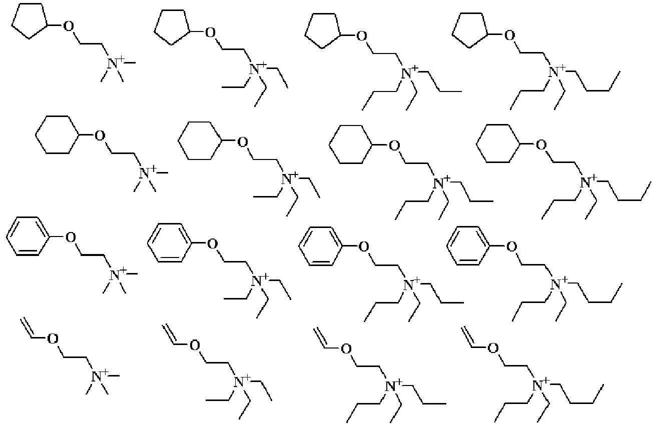

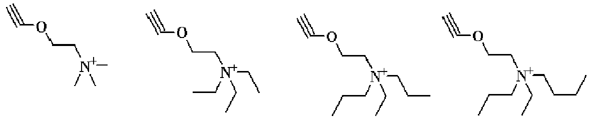

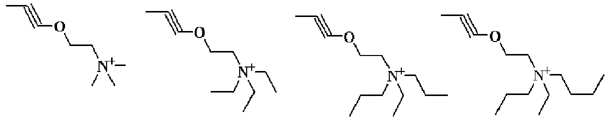

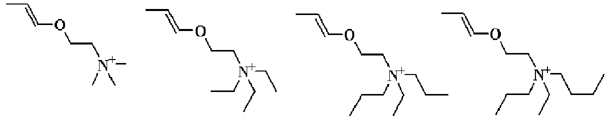

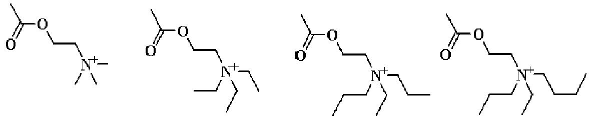

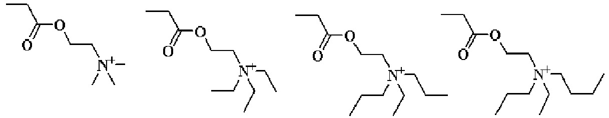

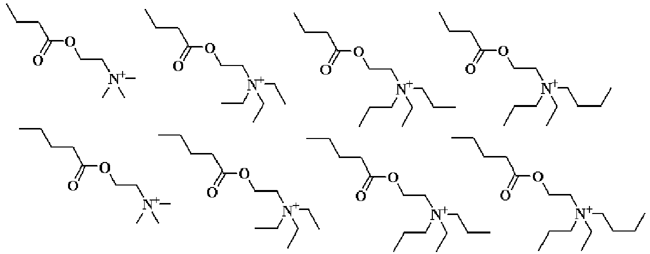

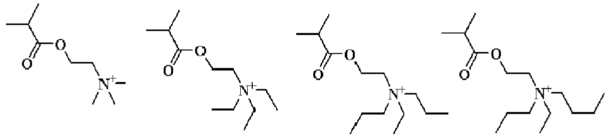

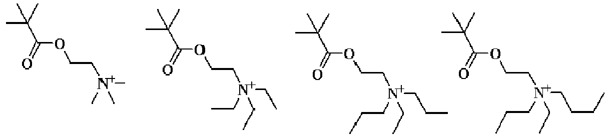

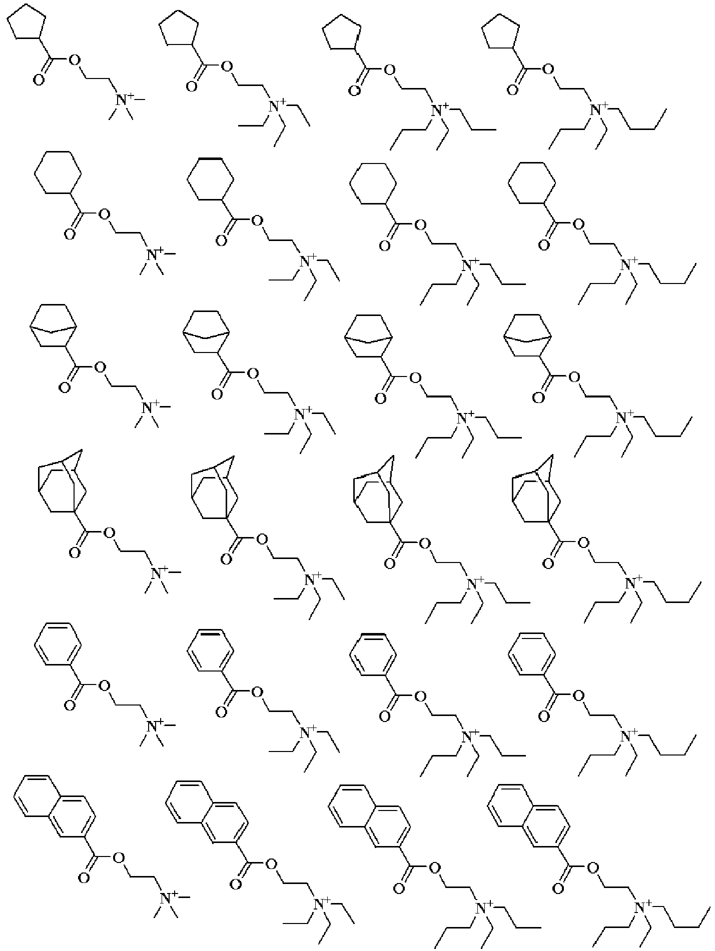

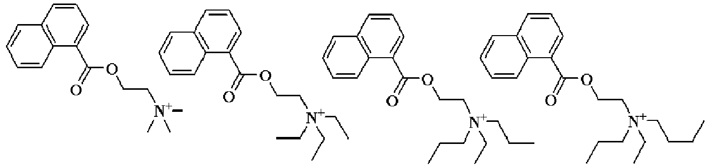

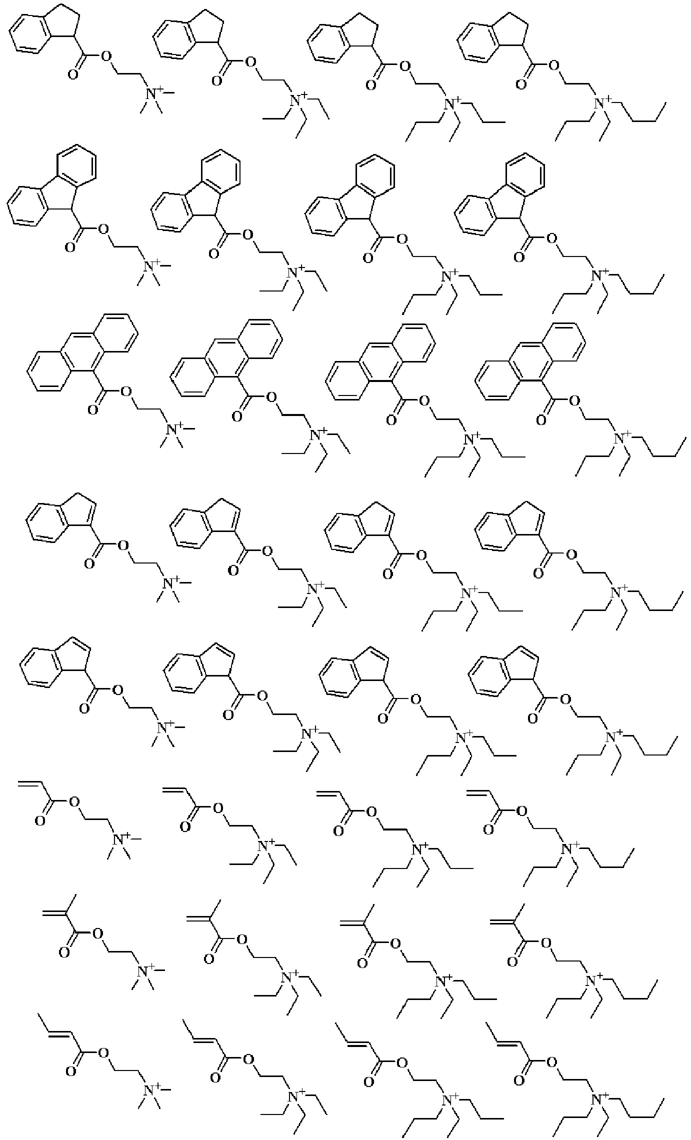

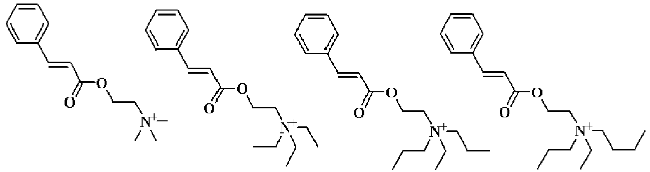

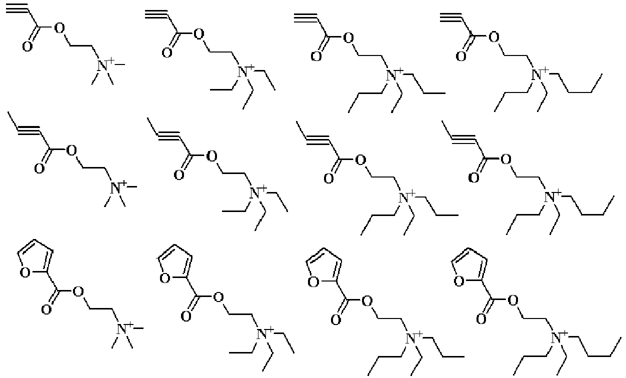

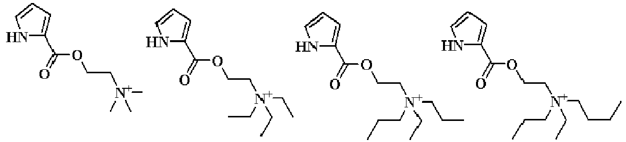

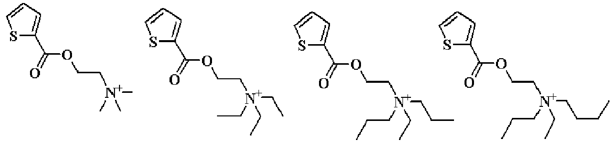

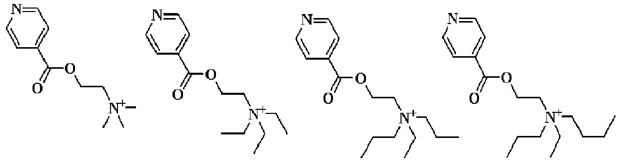

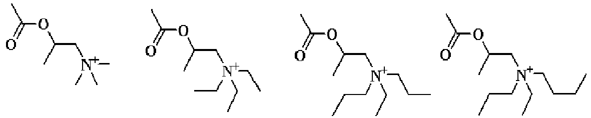

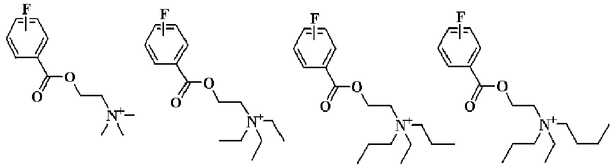

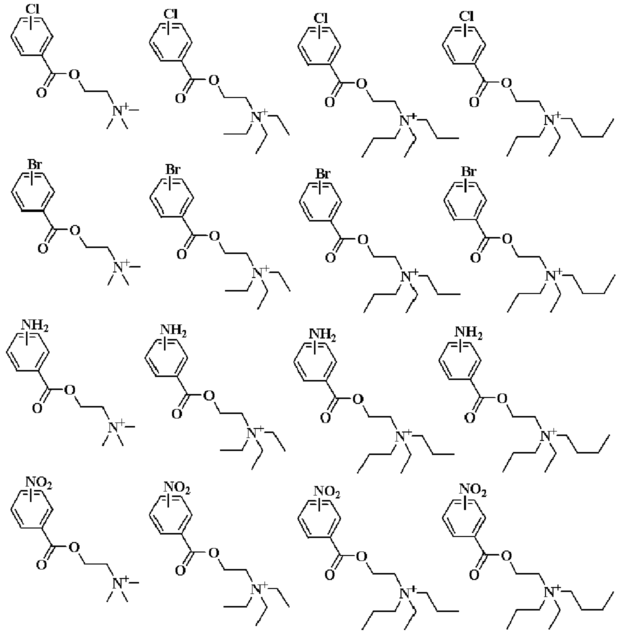

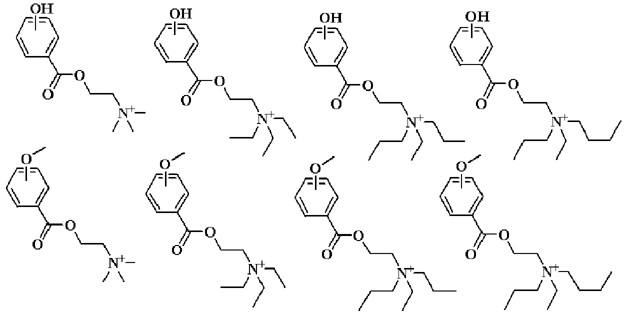

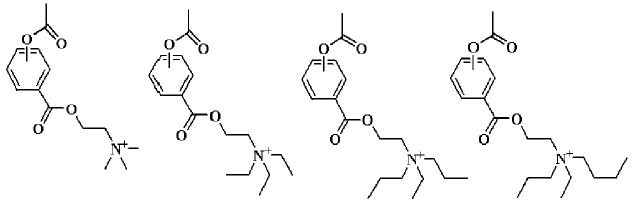

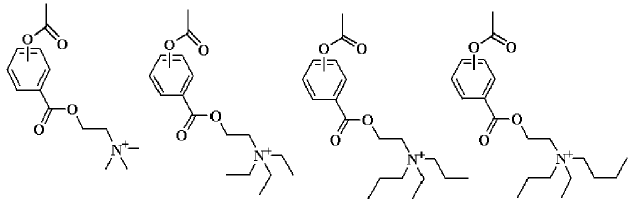

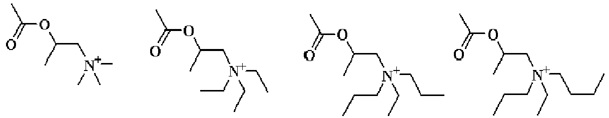

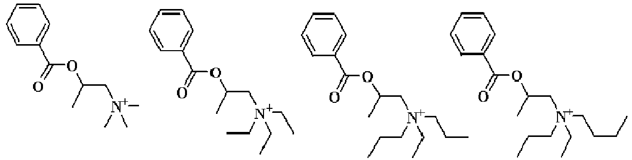

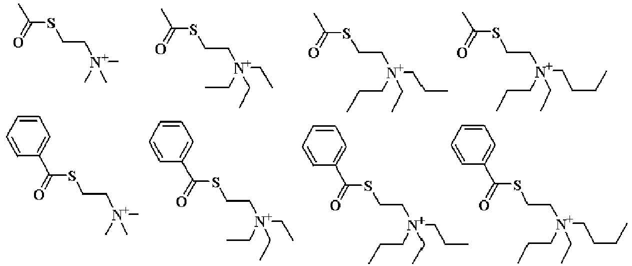

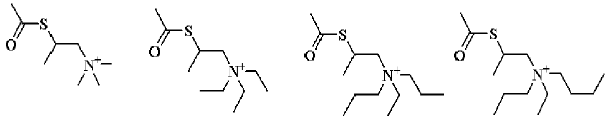

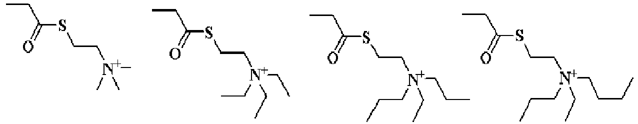

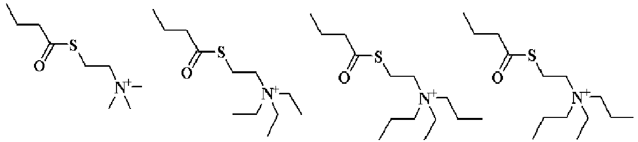

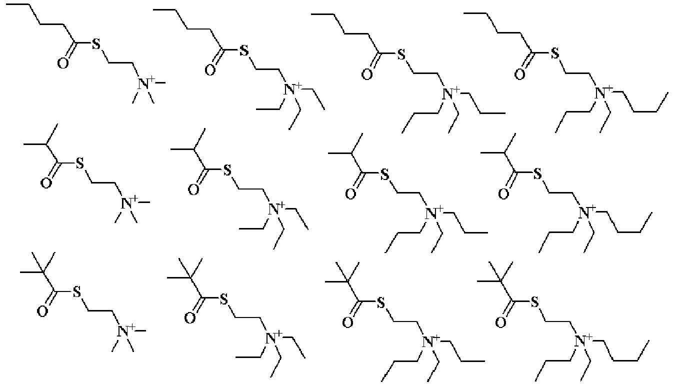

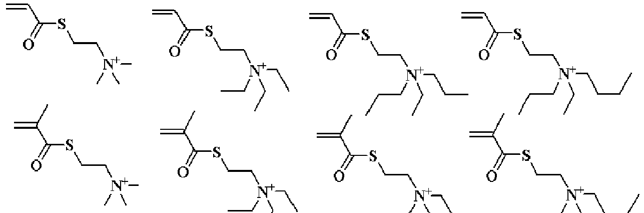

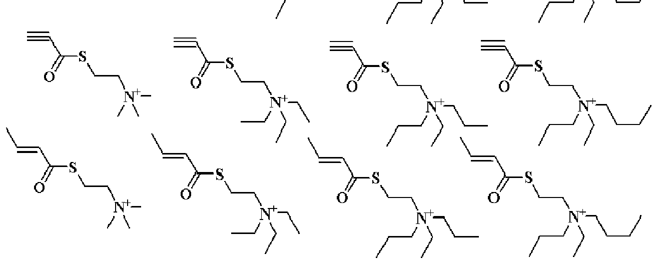

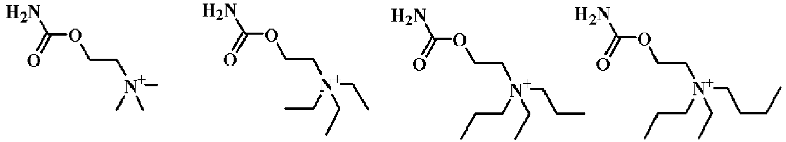

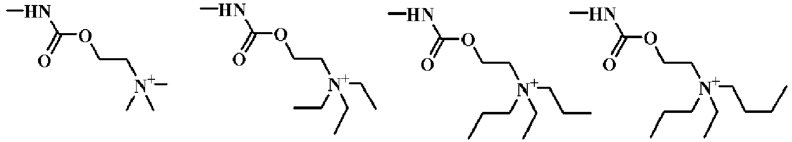

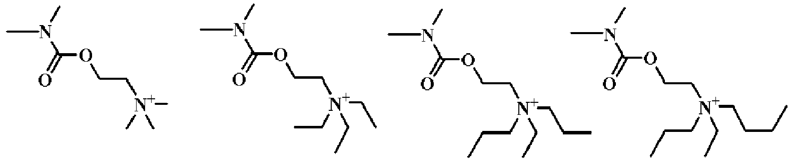

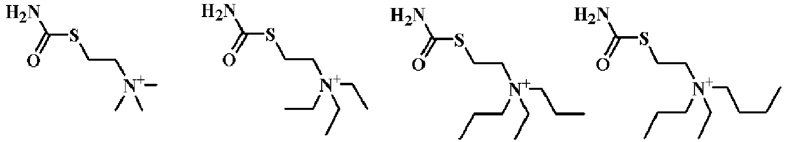

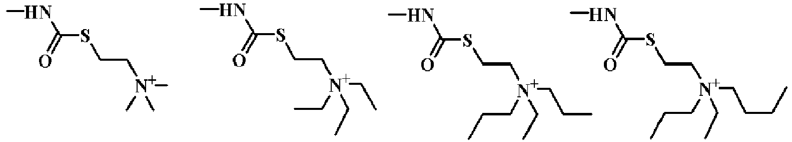

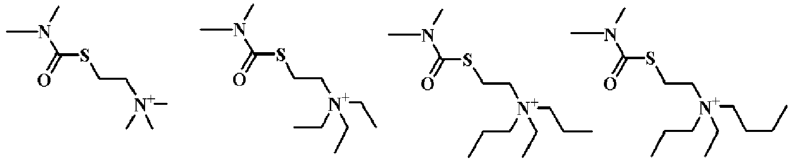

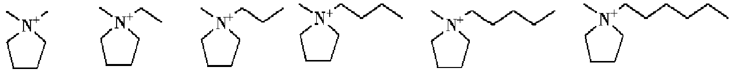

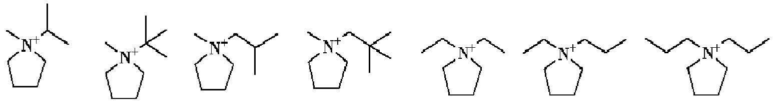

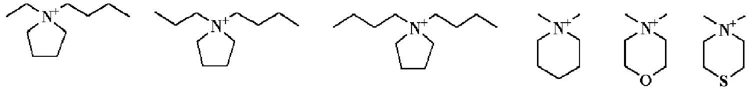

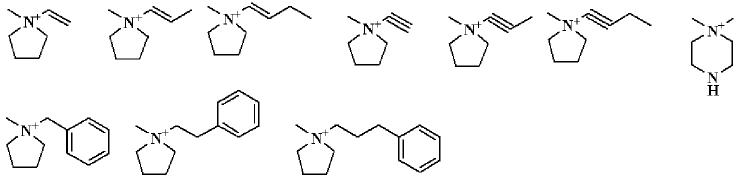

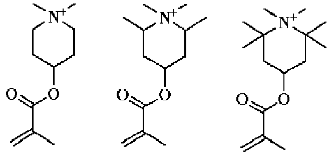

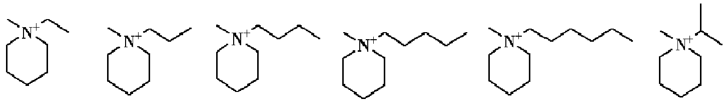

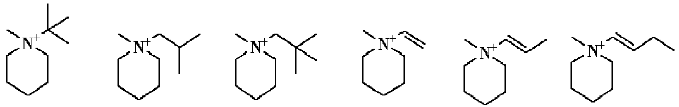

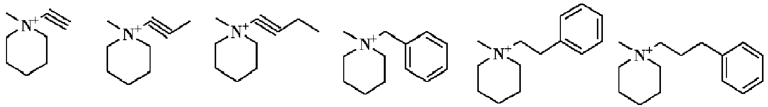

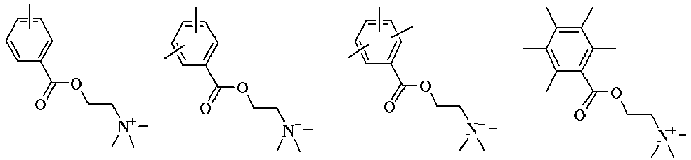

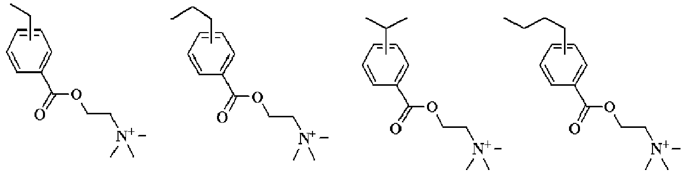

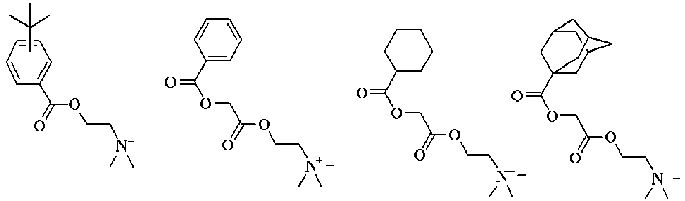

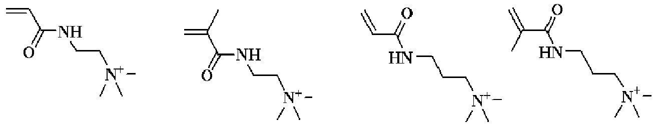

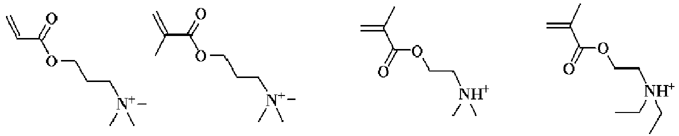

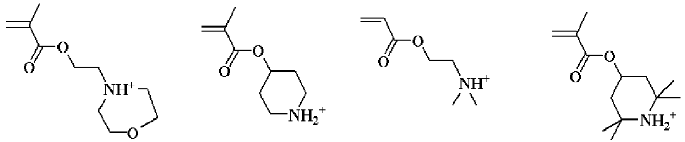

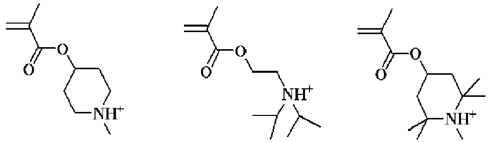

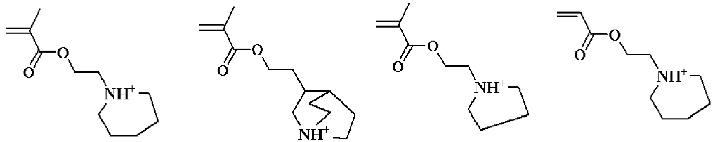

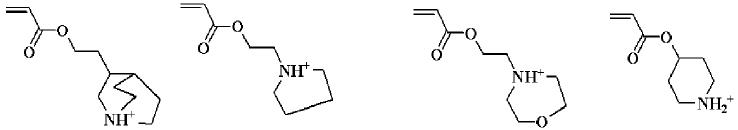

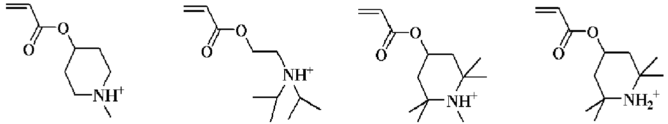

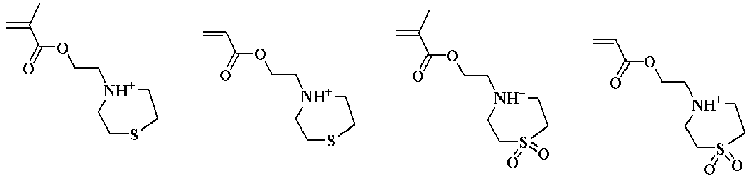

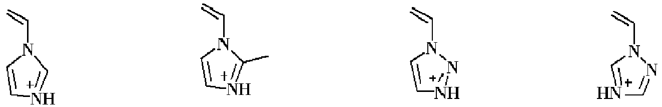

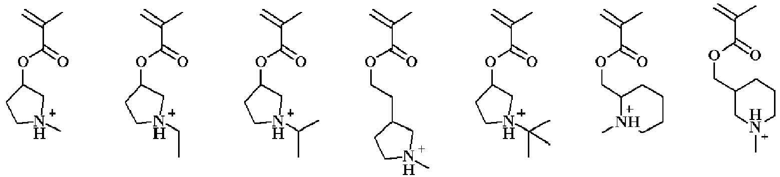

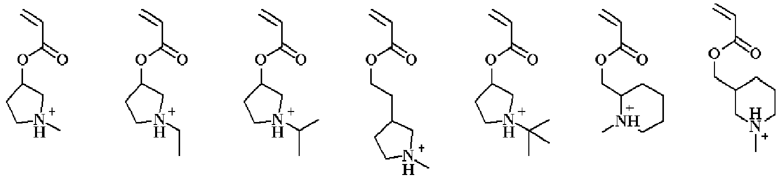

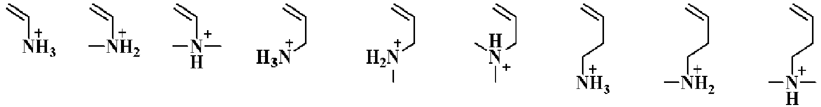

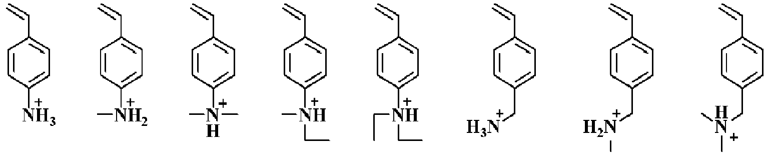

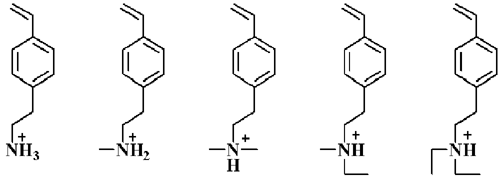

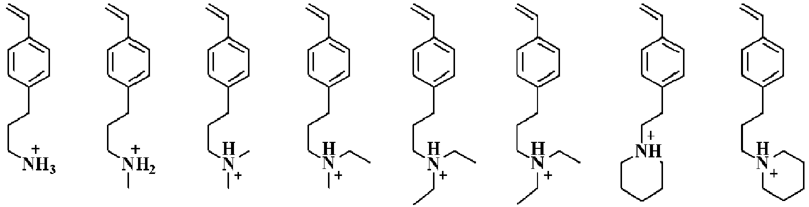

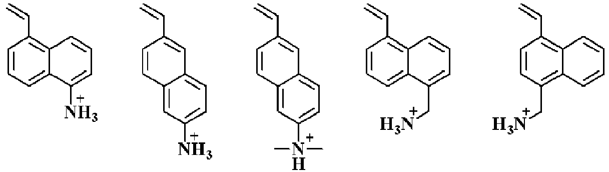

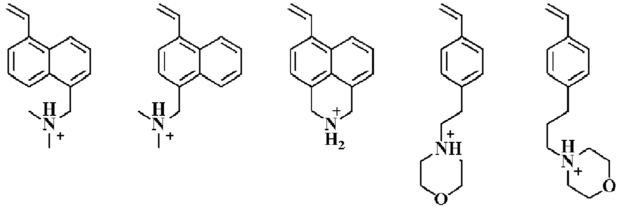

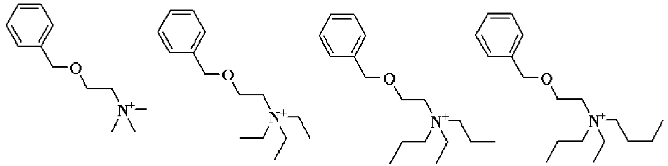

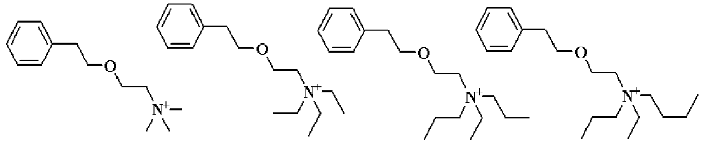

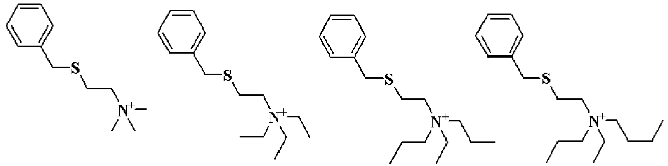

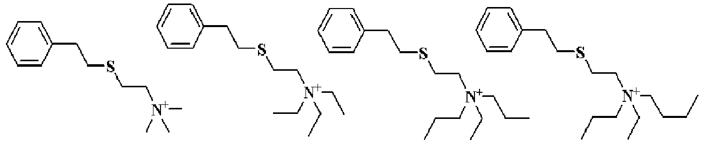

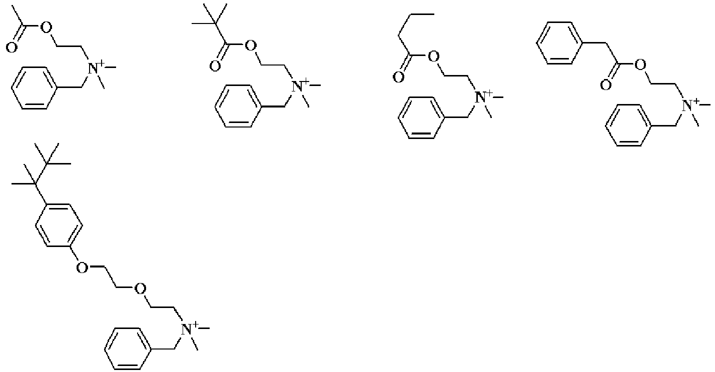

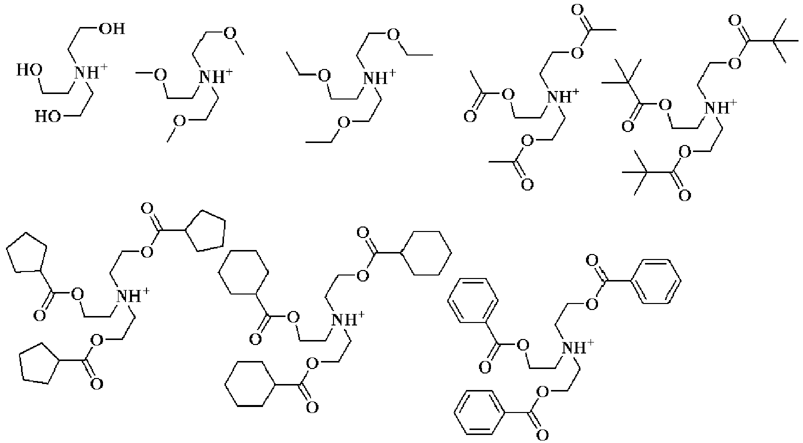

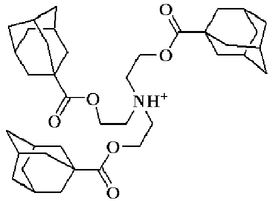

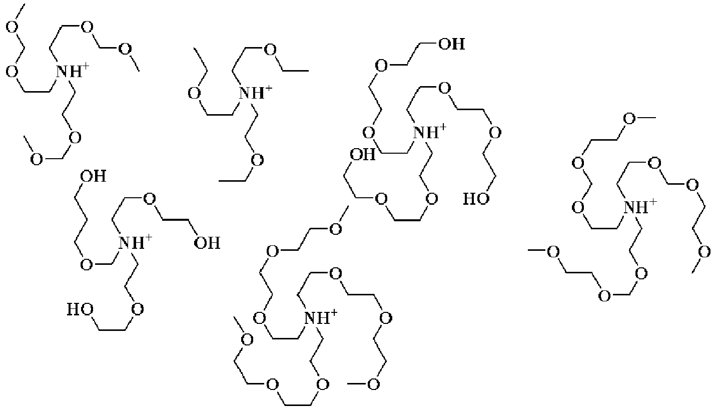

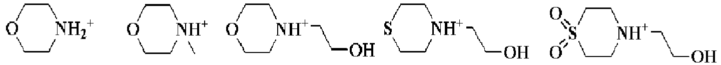

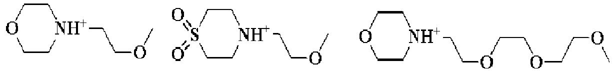

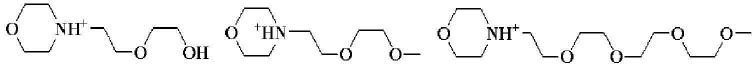

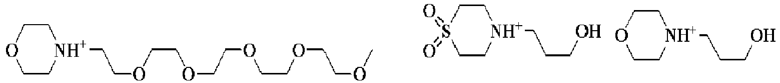

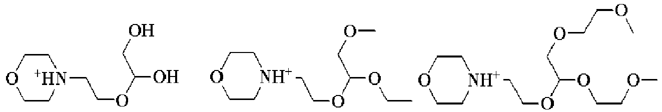

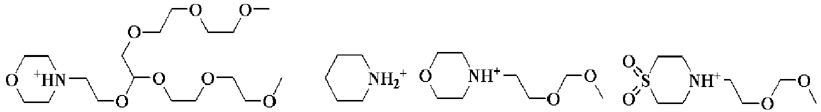

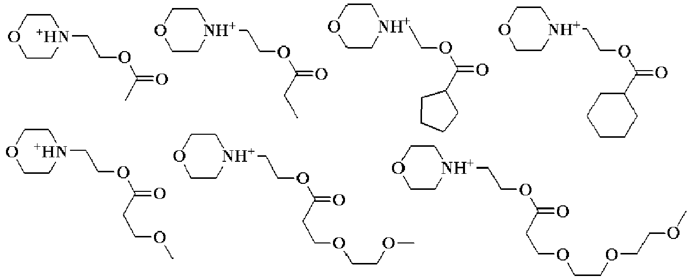

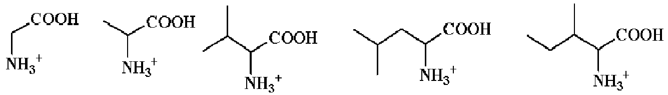

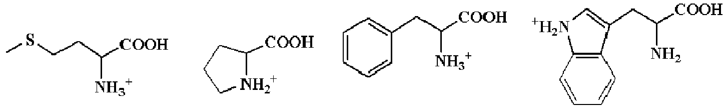

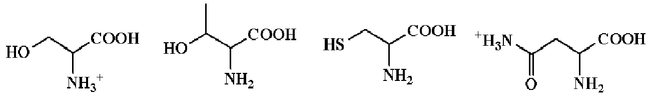

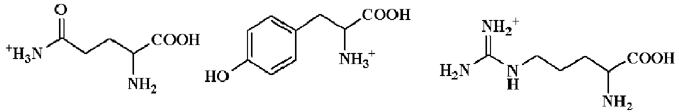

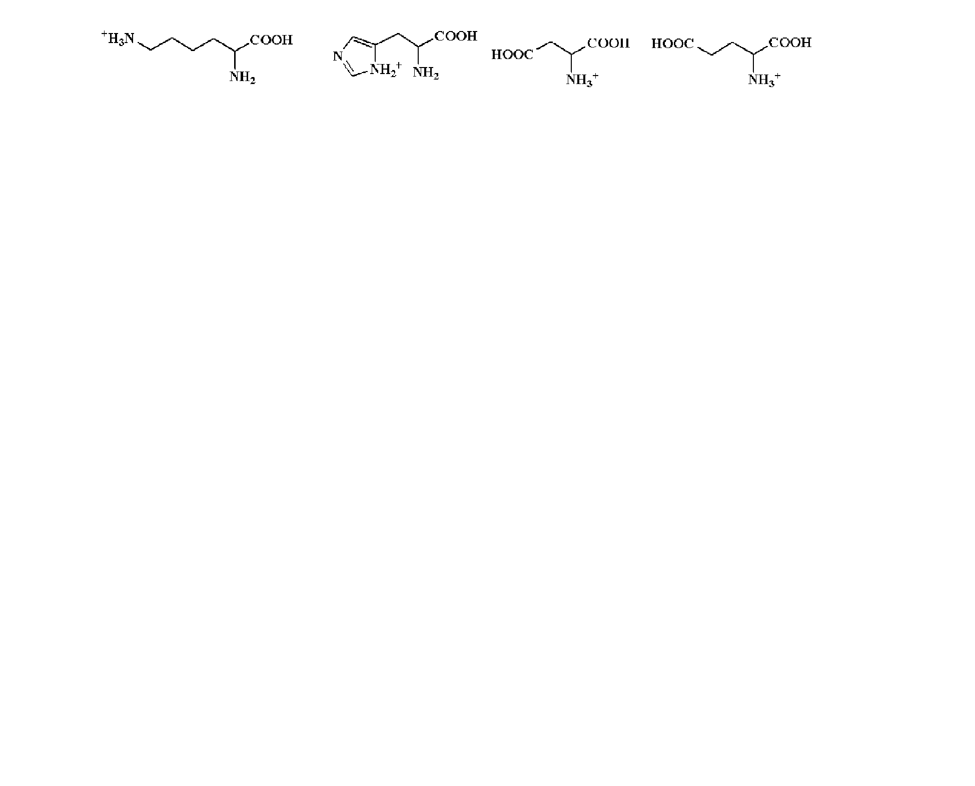

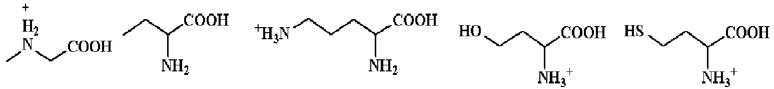

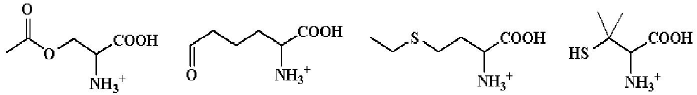

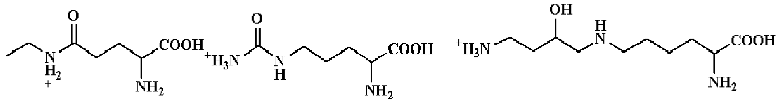

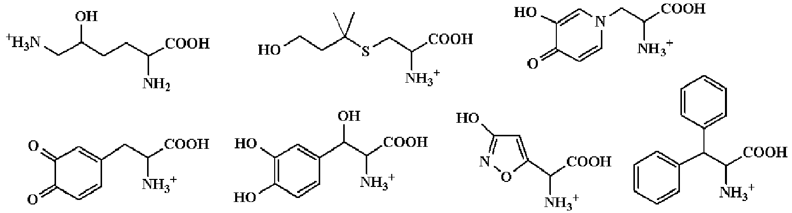

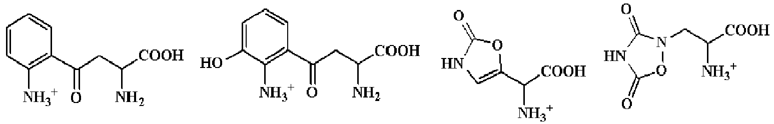

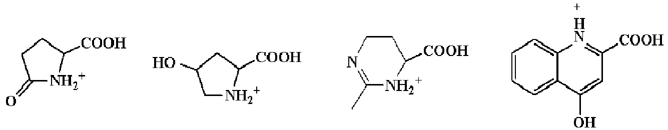

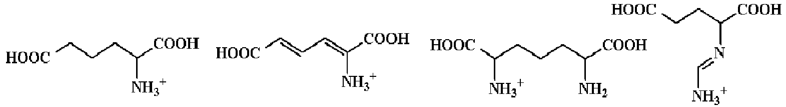

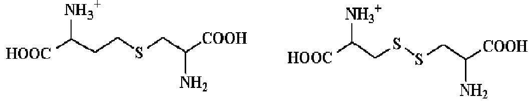

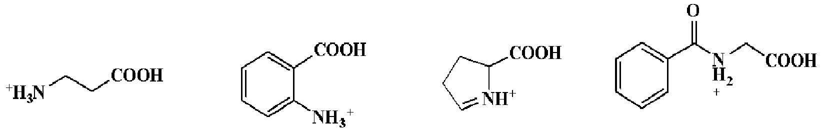

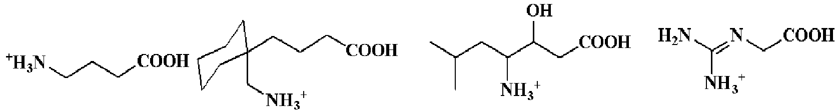

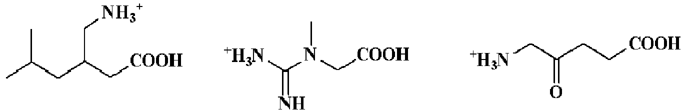

### Other Components

### (Surfactant)

Into the material for forming the living-body contact layer contained in the bio-electrode of the present invention, a surfactant may be added in order to improve wettability on a mold or on a body to be processed such as a substrate such as a conductive layer. Examples of such a surfactant include nonionic, cationic, and anionic surfactants. Specific examples include: nonionic surfactants such as a polyoxyethylene alkyl ether, a polyoxyethylene alkyl phenyl ether, a polyoxyethylene carboxylate ester, a sorbate ester, and a polyoxyethylene sorbate ester; cationic surfactants such as an alkyltrimethylammonium chloride and an alkylbenzylammonium chloride; anionic surfactants such as an alkyl or alkylallyl sulfate salt, an alkyl or alkylallyl sulfonate salt, and a dialkylsulfosuccinate salt; and zwitterion surfactants such as an amino-acid type and a betain-type surfactant. A blending amount of the surfactant is preferably 0.01 to 0.1 part by mass relative to 100 parts by mass of the ionic polymer (A).

### (B) Resin

As the component (B) contained in the material for the living-body contact layer, a resin other than the component (A) may be contained. The resin (B) is a component to prevent elution of the ionic polymer (A) and to retain adhesiveness with the substrate. The component (B) is preferably one or more resins selected from a silicone resin, a (meth)acrylate resin, and a urethane resin. A blending amount of the component (B) is preferably 1 to 10,000 parts by mass relative to 100 parts by mass of the ionic polymer (A).

### Optional Components

The material for forming the living-body contact layer contained in the bio-electrode of the present invention may contain optional components such as an ionic additive and an organic solvent.

### Ionic Additive

Into the material for forming the living-body contact layer contained in the bio-electrode of the present invention, the ionic additive may be added in order to increase the ion conductivity. With considering the bio-compatibility, examples thereof include sodium chloride, potassium chloride, calcium chloride, saccharine, potassium acesulfame, and salts described in JP 2018-044147 A, JP 2018-059050 A, JP 2018-059052 A, and JP 2018-130534 A. A blending amount of the ionic additive is preferably 0.1 to 100 parts by mass relative to 100 parts by mass of the ionic polymer (A) .

Ammonium salts of a fluorosulfonic acid, a fluoroimide acid, and a fluoromethide acid are known as ionic liquid. Specifically, ionic liquids described in the following literature may be added: Trulove C, Mantz R. 2003. Ionic Liquids in Synthesis, Chapter 3.6: Electrochemical Properties of Ionic Liquids.

### Silicone Compound having Polyglycerol Structure

Into the material for forming the living-body contact layer contained in the bio-electrode of the present invention, a silicone compound having a polyglycerol structure may be added in order to improve moisture retainability of the film to improve sensibility and ion conductivity released from the skin. A blending amount of the silicone compound having a polyglycerol structure is preferably 0.01 to 100 parts by mass, and more preferably 0.5 to 60 parts by mass relative to 100 parts by mass of the total of the component (A) and the component (B). The silicone compound having a polyglycerol structure may be used singly, or two or more thereof may be mixed for use.

Examples of the silicone compound having a polyglycerol structure include compounds described in JP 2021-115458 A.

The material containing such a silicone compound having a polyglycerol structure can exhibit more excellent moisture retainability, and is consequently a material that can form a living-body contact layer exhibiting more excellent sensitivity to the ions released from the skin.

### Metal Powder

Into the material for forming the living-body contact layer contained in the bio-electrode of the present invention, a metal powder selected from gold, silver, platinum, copper, tin, titanium, nickel, aluminum, tungsten, molybdenum, ruthenium, chromium, and indium may be added in order to improve the electron conductivity. An amount of the added metal powder and a carbon content is preferably within a range of 1 to 50 parts by mass relative to 100 parts by mass of the resin being the total of the component (A) and the component (B) .

The type of the metal powder is preferably gold, silver, or platinum from the viewpoint of conductivity, and preferably silver, copper, tin, titanium, nickel, aluminum, tungsten, molybdenum, ruthenium, or chromium from the viewpoint of price. The type of the metal powder is preferably the noble metals from the viewpoint of the bio-compatibility, and most preferably silver in terms of these collective viewpoints.

Examples of the shape of the metal powder include sphere, disk, flake, and needle, and the flake powder is preferably added because of the highest conductivity.

The metal powder is preferably a flake having a relatively low density and a large specific surface area, and having a size of 100 um or less, a tap density of 5 g/cm³ or less, and a specific surface area of 0.5 m²/g or more.

### Carbon Material

A carbon material may be added as a conductivity improver. Examples of the carbon material include carbon black, graphite, carbon nanotube, carbon fiber, and graphene. The carbon nanotube may be any of a single layer and a multilayer, and a surface thereof may be modified with an organic group. An amount of the added carbon material is preferably within a range of 1 to 50 parts by mass relative to 100 parts by mass of the resin being the total of the component (A) and the component (B).

### Silicon Powder

Into the material for forming the living-body contact layer contained in the bio-electrode of the present invention, a silicon powder may be added in order to improve sensitivity to ion receiving. Examples of the silicon powder include powders made of silicon, silicon monoxide, and silicon carbide. A particle diameter of the powder is preferably less than 100 um, and more preferably 1 um or less. Since finer particles have a larger surface area, the finer particles can receive a larger amount of ions to achieve the bio-electrode with high sensitivity. An amount of the added silicon powder is preferably within a range of 1 to 50 parts by mass relative to 100 parts by mass of the resin being the total of the component (A) and the component (B) .

### Lithium Titanate Powder

Into the material for forming the living-body contact layer contained in the bio-electrode of the present invention, a lithium titanate powder may be added in order to improve sensitivity to ion receiving. Examples of a molecular formula of the lithium titanate powder include Li₂TiO₃, LiTiO₂, and Li₄Ti₅O₁₂ with a spinel structure, and the powder preferably has the spinel structure. Alternatively, a lithium titanate powder forming a composite with carbon may also be used. A particle diameter of the lithium titanate powder is preferably less than 100 um, and more preferably 1 um or less. Since finer particles have a larger surface area, the finer particles can receive a larger amount of ions to achieve the bio-electrode with high sensitivity. These may be a composite powder with carbon. An amount of the added lithium titanate powder is preferably within a range of 1 to 50 parts by mass relative to 100 parts by mass of the resin being the total of the component (A) and the component (B).

### Organic Solvent

Specific examples of the organic solvent include: ketone solvents, such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, and methyl n-pentyl ketone; alcohol solvents, such as 3-methoxybutanol, 3-methyl-3-methoxybutanol, 1-methoxy-2-propanol, and 1-ethoxy-2-propanol; ether solvents, such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, di-sec-pentyl ether, di-tert-amyl ether, di-n-hexyl ether, and anisole; ester solvents, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactone solvents, such as γ-butyrolactone.

An amount of the added organic solvent is preferably within a range of 10 to 50,000 parts by mass relative to 100 parts by mass of the resin being the total of the component (A) and the component (B).

When the ionic polymer has the oxirane group or the oxetane group in the repeating unit, an amine-based crosslinking agent and an imidazole-based crosslinking catalyst may be added.

To reduce a viscosity of a solution containing the ionic polymer or to achieve crosslinking, a (meth)acrylate monomer may be added. To achieve crosslinking, a monomer having a plurality of (meth)acrylates in one molecule is preferable.

To allow the (meth)acrylate to react, a radical generator may be added. The radical generator is a compound to generate radicals by heat or light, and specifically, compounds exemplified in JP 2020-097214 A may be used.

### <Method for Manufacturing Bio-Electrode>

The present invention provides a method for manufacturing the aforementioned bio-electrode, the method including a step of manufacturing the micropillar by inserting a resin containing the component (A) into a mold to be a template, and releasing the resin from the mold.

In addition, the present invention provides a method for manufacturing the aforementioned bio-electrode, the method including a step of manufacturing the micropillar by inserting a resin containing the component (A) into a pattern of a resin to be a template, and dissolving a pattern of the resin with water or an alkaline aqueous solution to release the resin containing the component (A).

Further, the present invention provides a method for manufacturing a bio-electrode, the method including steps of: forming a conductive layer on a substrate; and forming a living-body contact layer having a micropillar containing an ionic polymer on a side to be contacted with skin on the conductive layer.

The substrate preferably has flexibility, stretchability, and transparency. A thin glass has flexibility and high transparency, but damages the skin if breaking in a state of adhering to the skin. Thus, a substrate made of resin, which does not break, is preferable. Examples of the resin usable as the substrate include poly(meth)acrylate (PMMA), polyurethane, silicone, PEEK, polyimide, polyolefin, styrene-butadiene rubber, polycarbonate, PET, PEN, vinyl chloride, polystyrene, polyethylene, polypropylene, polymethylpentene, and Teflon (Trademark).

Examples of a method for forming the conductive layer on the substrate include: a method of printing a conductive pattern by using a conductive paste containing conductive particles, a resin, and a solvent; and a method of laminating a metal thin film.

A method for applying the material for forming the living-body contact layer on the conductive layer is not particularly limited, and examples thereof include a method of direct printing and a method of transferring after the applying on another substrate. For both the methods, methods such as dip coating, spray coating, spin coating, roll coating, flow coating, doctor coating, screen printing, flexographic printing, a gravure printing, and ink-jet printing are suitable, for example.

For a method for forming the conductive layer, a method similar to the method for applying the material for forming the living-body contact layer may be used.

After applying the conductive layer or after applying the material for forming the living-body contact layer, heating is performed for a purpose of evaporating the solvent to solidify the layer.

A temperature for heating the material for forming the living-body contact layer after the applying is not particularly limited, and preferably approximately 50 to 250°C, for example.

FIGS. 1 to 23 illustrate a method for forming the living-body contact layer into the micropillars by using the mold.

FIGS. 24 to 28 illustrate a method of forming a pattern to be a template by using lithography, forming the living-body contact layer thereon, and dissolving and removing the template pattern.

FIGS. 29 to 31 illustrate a method for forming the living-body contact layer on a surface of the conductive pillars connected to the conductive layer.

Adhering waterdrops or spraying water vapor or mist on the surface of the living-body contact layer after forming the micropillars improves compatibility with the skin to quickly obtain the bio-signal. To reduce the size the water vapor, mist, or waterdrops, water mixed with an alcohol may also be used. The layer surface or the skin surface may be wetted by contacting with water-containing cotton or cloth.

The water for wetting the surface of the living-body contact layer after the micropillar formation may contain a salt. The water-soluble salt to be mixed with water is preferably selected from sodium salts, potassium salts, calcium salts, magnesium salts, and betaines.

The water-soluble salt may be specifically a salt selected from sodium chloride, potassium chloride, calcium chloride, magnesium chloride, sodium saccharine salt, potassium acesulfame, a sodium carboxylate, a potassium carboxylate, a calcium carboxylate, a sodium sulfonate, a potassium sulfonate, a calcium sulfonate, sodium phosphate, potassium phosphate, calcium phosphate, magnesium phosphate, and betaines. The aforementioned ionic polymer (A) is not included in the water-soluble salt.

More specific examples additionally include sodium acetate, sodium propionate, sodium pivalate, sodium glycolate, sodium butyrate, sodium valerate, sodium caproate, sodium enanthate, sodium caprylate, sodium pelargonate, sodium caprate, sodium undecanoate, sodium laurate, sodium tridecanoate, sodium myristate, sodium pentadecanoate, sodium palmitate, sodium margarate, sodium stearate, sodium benzoate, disodium adipate, disodium maleate, disodium phthalate, sodium 2-hydroxybutyrate, sodium 3-hydroxybutyrate, sodium 2-oxobutyrate, sodium gluconate, sodium methanesulfonate, sodium 1-nonanesulfonate, sodium 1-decanesulfonate, sodium 1-dodecanesulfonate, sodium 1-undecanesulfonate, sodium cocoyl isethionate, sodium lauroylmethylalanine, sodium cocoyl methyltaurine, sodium cocoyl glutamate, sodium cocoyl sarcocinate, sodium lauroylmetyltaurine, lauramidopropyl betaine, potassium isobutyrate, potassium propionate, potassium pivalate, potassium glycolate, potassium gluconate, potassium methanesulfonate, calcium stearate, calcium glycolate, calcium gluconate, calcium 3-methyl-2-oxobutyrate, and calcium methanesulfonate. The betaine is a general name of an intramolecular salt, and specifically a compound in which three methyl groups are added on an amino group of an amino acid. More specific examples thereof include trimethylglycine, carnitine, and proline betaine.

The water-soluble salt may further contain a monohydric alcohol or polyhydric alcohol having 1 to 4 carbon atoms. The alcohol is preferably selected from ethanol, isopropyl alcohol, ethylene glycol, diethylene glycol, triethylene glycol, glycerol, polyethylene glycol, polypropylene glycol, polyglycerol, diglycerol, and a silicone compound having a polyglycerol structure.

As the pretreating method with the salt-containing aqueous solution, the living-body contact layer after the micropillar formation is wetted by a spraying method, a waterdrop dispensing method, etc. The living-body contact layer may also be wetted in a high-temperature high-humidity state like a sauna. After the wetting, the living-body contact layer may be covered with a sheet in order to prevent dryness. Since the sheet needs to be released immediately before attached to the skin, a fluororesin film that is coated with a releasing agent or that is releasable is used. The dry electrode covered with the release sheet is sealed with a bag covered with aluminum etc. for storage in a long term. To prevent dry in the bag covered with aluminum, moisture is preferably sealed therein.

It is effective for wetting the skin surface to obtain a highly sensitive and highly accurate bio-signal in a short time that the skin on a side to which the bio-electrode is to be attached is wiped with cloth containing water or an alcohol, such as ethanol and glycerol, containing water immediately before attachment or coating by spraying. Wiping with the water-containing cloth has effects of not only wetting the skin but also removing fat on the skin surface, which also improve the sensitivity to the bio-signal.

As above, by the method for manufacturing a bio-electrode of the present invention, the bio-electrode of the present invention that has excellent conductivity and bio-compatibility, that is lightweight, and that does not considerably deteriorate the conductivity even when being wetted or dried can be easily manufactured at a low cost.

### EXAMPLE

Hereinafter, the present invention will be specifically described by using Examples and Comparative Examples, but the present invention is not limited thereto.

Ionic polymers 1 to 11 that were blended as an ionic material (conductive material) with a bio-electrode composition solution were synthesized as follows.

A 30-mass% cyclopentanone solution of each monomer was added in a reaction vessel, and mixed. The reaction vessel was cooled under a nitrogen atmosphere to -70°C, and degassing under a reduced pressure and nitrogen blowing were repeated three times. After the temperature was raised to room temperature, azobisisobutyronitrile (AIBN) as a polymerization initiator was added at 0.01 mol relative to 1 mol of an entirety of the monomers, the temperature was raised to 60°C, and then the mixture was allowed to react for 15 hours. A composition of the obtained polymer was determined by ¹H-NMR after evaporating the solvent. A molecular weight (Mw) and a dispersion degree (Mw/Mn) of the obtained polymer were determined by gel permeation chromatography (GPC) using tetrahydrofuran (THF) as a solvent. The ionic polymers 1 to 11 synthesized as above are shown below.

### Ionic polymer 1

Mw=39,600
Mw/Mn=1.94

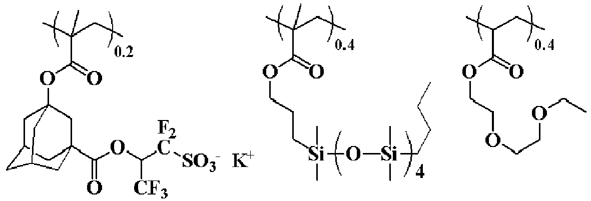

The repetition numbers in the formula represent average values.

### Ionic polymer 2

Mw=46,800
Mw/Mn=1.92

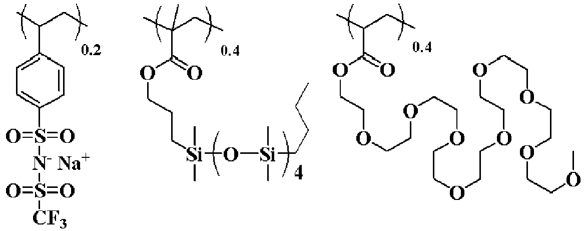

The repetition numbers in the formula represent average values.

### Ionic polymer 3

Mw=39,600
Mw/Mn=1.82

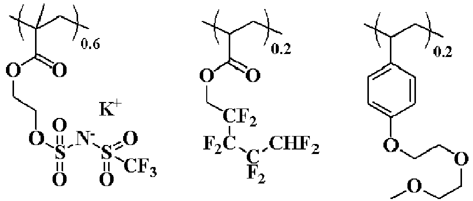

### Ionic polymer 4

Mw=44,100
Mw/Mn=1.91

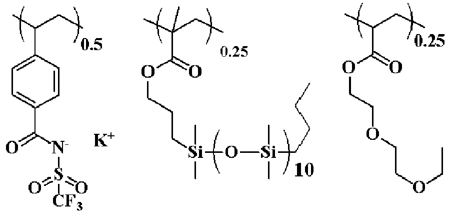

The repetition numbers in the formula represent average values.

### Ionic polymer 5

Mw=49,200
Mw/Mn=2.22

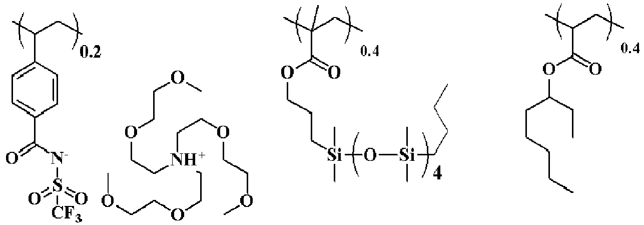

The repetition numbers in the formula represent average values.

### Ionic polymer 6

Mw=46,000
Mw/Mn=2.31

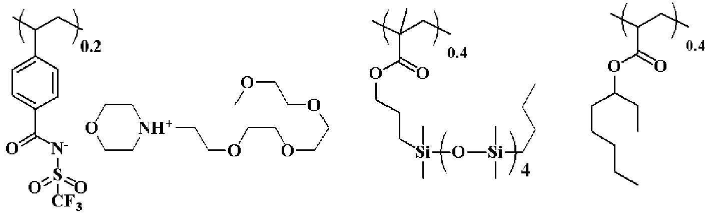

The repetition numbers in the formula represent average values.

### Ionic polymer 7

Mw=4 5, 700
Mw/Mn=2.01

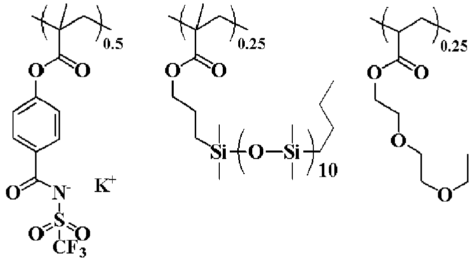

The repetition numbers in the formula represent average values.

### Ionic polymer 8

Mw=52,300
Mw/Mn=2.21

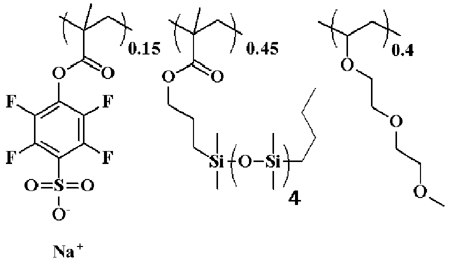

The repetition numbers in the formula represent average values.

### Ionic polymer 9

Mw=32,700
Mw/Mn=1.81

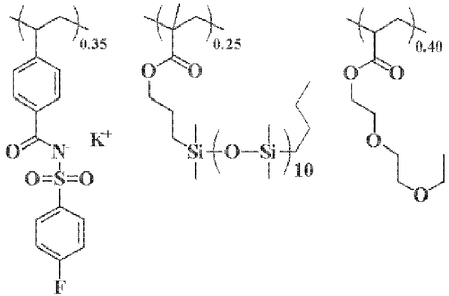

The repetition numbers in the formula represent average values.

### Ionic polymer 10

Mw=46,400
Mw/Mn=2.13

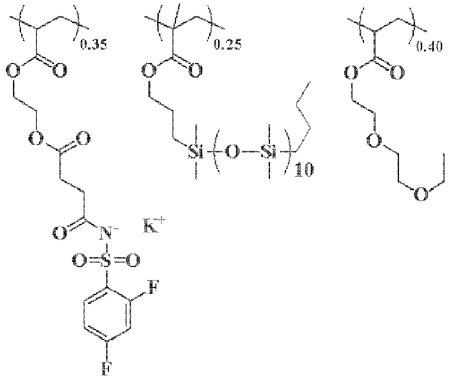

The repetition numbers in the formula represent average values.

### Ionic polymer 11

Mw=36,400
Mw/Mn=2.03

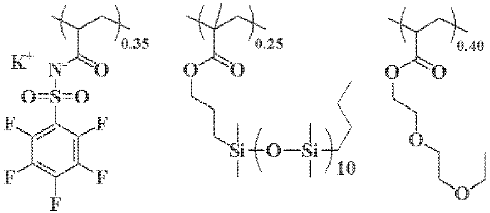

The repetition numbers in the formula represent average values.

Ionic polymers 12 to 19 were synthesized as follows. By using a dihydroxy-group-containing fluorinated alkyl or aryl sulfonate salt compound or by using a dihydroxy-group-containing fluorosulfonamide compound, an isocyanate compound, a dihydroxy compound for extending the chain length in some cases, a monohydroxy compound or monoisocyanate compound for blocking the terminals, propylene glycol monomethyl ether acetate solvent at twice mass of the raw material compounds, and a catalyst XK-640 (manufactured by King Industries, Inc.) at 0.02 mass% relative to 100 parts by mass of the raw material compounds were mixed and reacted at 90°C for 9 hours to synthesize the ionic polymers 12 to 19.

### Ionic polymer 12

Mw=22,700
Mw/Mn=2.73

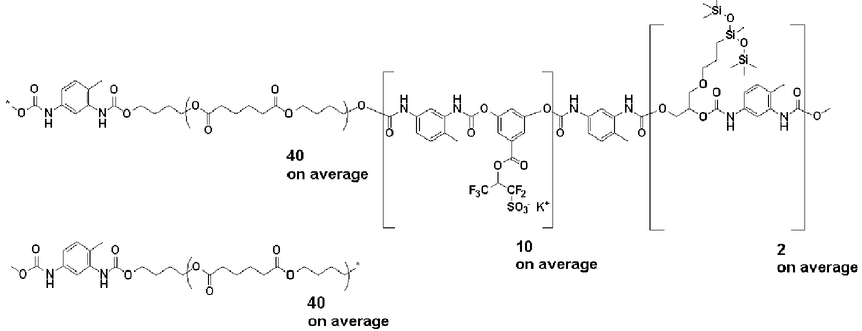

In the formula, "*" represents a bonding point.

### Ionic polymer 13

Mw=24,000
Mw/Mn=2.95

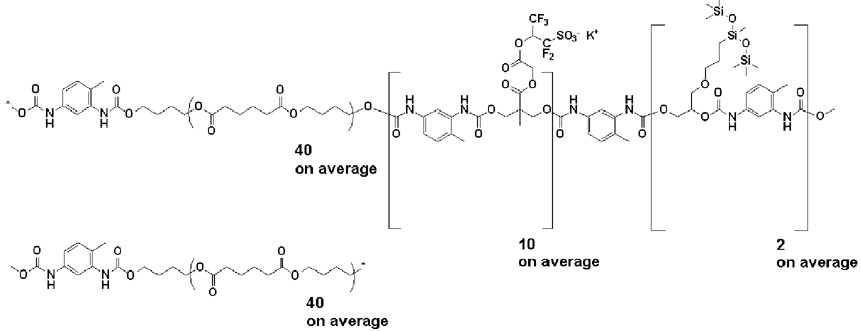

In the formula, "*" represents a bonding point.

### Ionic polymer 14

Mw=10,300
Mw/Mn=2.23

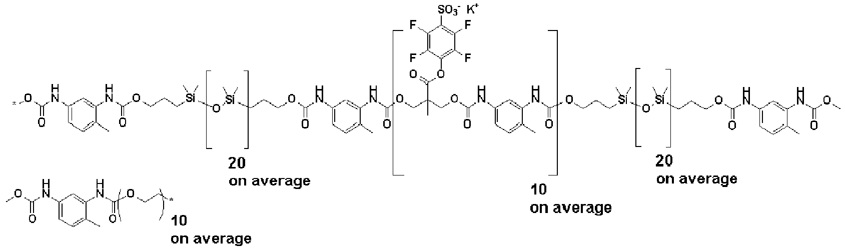

In the formula, "*" represents a bonding point.

### Ionic polymer 15

Mw=38,000
Mw/Mn=3.10

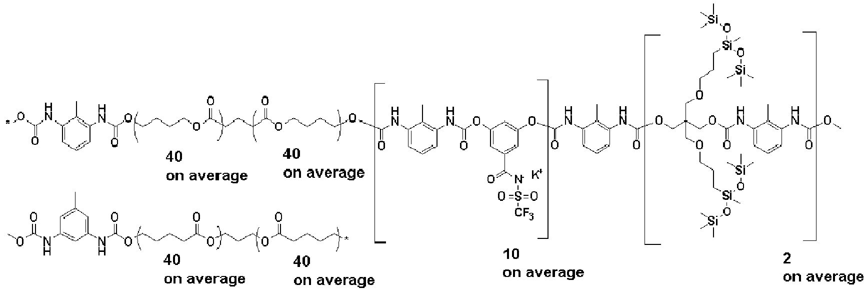

In the formula, "*" represents a bonding point.

### Ionic polymer 16

Mw=24,000
Mw/Mn=2.82

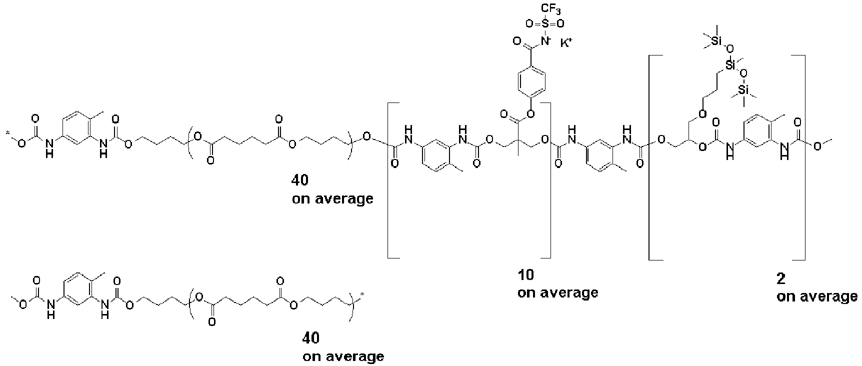

In the formula, "*" represents a bonding point.

### Ionic polymer 17

Mw=24,400
Mw/Mn=2.89

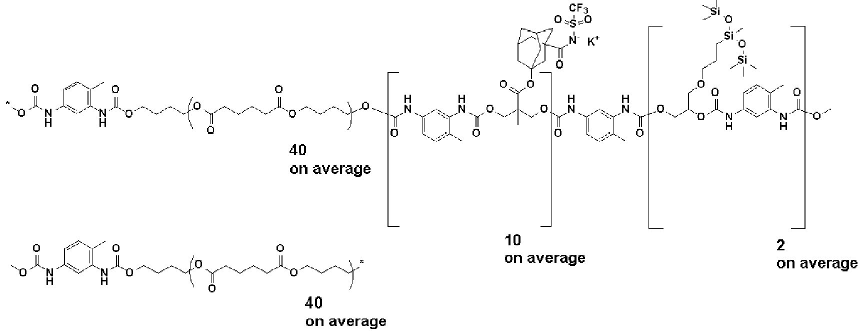

In the formula, "*" represents a bonding point.

### Ionic polymer 18

Mw=9,900
Mw/Mn=2.08

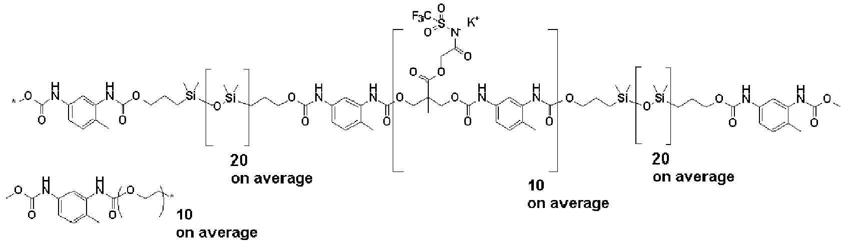

In the formula, "*" represents a bonding point.

### Ionic polymer 19

Mw=9,700
Mw/Mn=2.02

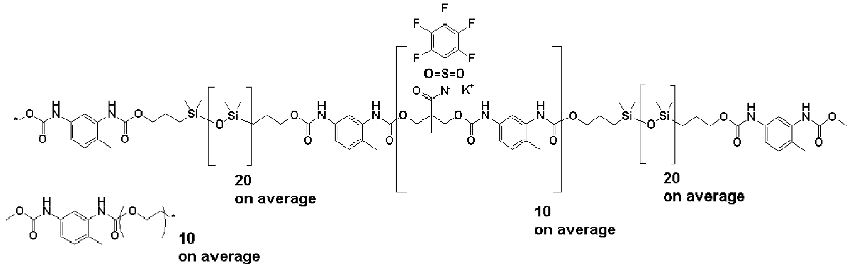

In the formula, "*" represents a bonding point.

Blend polyurethanes 1 and 2 blended with the bio-electrode solution are shown below.

### Blend polyurethane 1

Mw=21,000
Mw/Mn=2.62

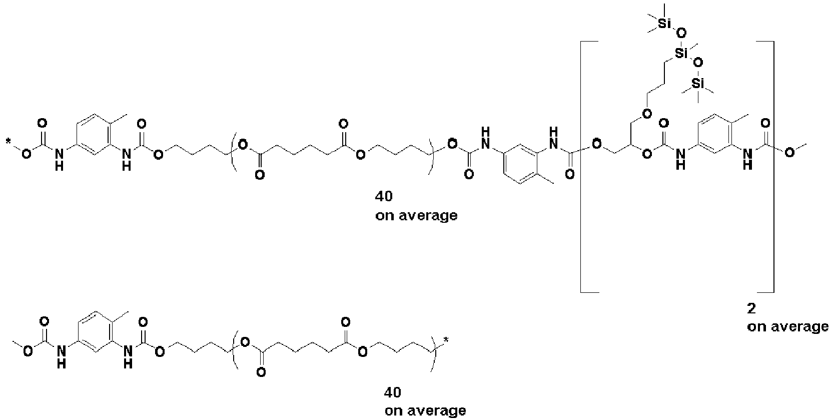

In the formula, "*" represents a bonding point.

### Blend polyurethane 2

Mw=35,000
Mw/Mn=2.83

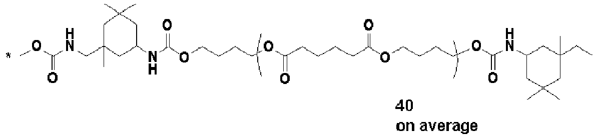

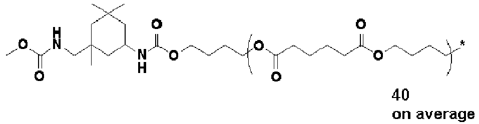

In the formula, "*" represents a bonding point.

Lithium titanate and conductively improvers (carbon black, carbon nanotube, graphite, silver powder, and polyglycerol silicone 1), which were blended as an additive with the bio-electrode composition solution, were as follows.
Lithium titanate: manufactured by Sigma-Aldrich, size: 200 nm or less
Carbon black: manufactured by Denka Company Limited, Denka Black Li-400
Carbon nanotube: manufactured by Sigma-Aldrich, diameter: 110 to 170 nm, length: 5 to 9 um
Graphite: manufactured by Sigma-Aldrich, diameter: 20 um or less
Silver powder: Silver flake manufactured by Sigma-Aldrich, diameter: 10 um

### Polyglycerol silicone 1

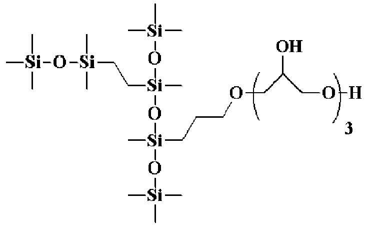

Solvents blended with the bio-electrode solution are described below.
BE: Diethylene glycol monobutyl ether
GBL: Gamma-butyrolactone

### Examples 1 to 19 and Comparative Examples 1 to 2

With compositions described in Tables 1 and 2, the ionic polymer, the resin, the solvent, and the additive (lithium titanate and the conductive improver) were blended to prepare bio-electrode solutions (bio-electrode solutions 1 to 19 and a comparative bio-electrode solution 1).

**[Table 1]**

| Bio-electrode solution | Ionic polymer (parts by mass) | Resin (parts by mass) | Solvent (parts by mass) | Additive (parts by mass) |
|---|---|---|---|---|
| Bio-electrode solution 1 | Ionic polymer 1 (100) | - | Cyclopentanone (60) | Polyglycerol silicone 1 (10) |
| | | | | Carbon black (10) |
| Bio-electrode solution 2 | Ionic polymer 2 (100) | - | Cyclopentanone (60) | Polyglycerol silicone 1 (10) |
| | | | | Carbon black (10) |
| Bio-electrode solution 3 | Ionic polymer 3 (20) | Blend urethane 1 (80) | Cyclopentanone (60) | Carbon nanotube (10) |
| Bio-electrode solution 4 | Ionic polymer 4 (30) | Blend urethane 1 (70) | Cyclopentanone (60) | Carbon nanotube (2) |
| Bio-electrode solution 5 | Ionic polymer 5 (100) | Blend urethane 2 (70) | Cyclopentanone (30) | Polyglycerol silicone 1 (10) |
| | | | 2-Heptanone (30) | Carbon black (10) |
| Bio-electrode solution 6 | Ionic polymer 6 (100) | - | Cyclopentanone (45) | Polyglycerol silicone 1 (12) |
| | | | BE (15) | Carbon black (10) |
| Bio-electrode solution 7 | Ionic polymer 7 (100) | - | Cyclopentanone (60) | Polyglycerol silicone 1 (14) |
| | | | | Carbon black (10) |
| Bio-electrode solution 8 | Ionic polymer 8 (100) | - | Cyclopentanone (50) | Polyglycerol silicone 1 (12) |
| | | | GBL (10) | Silver powder (30) |
| Bio-electrode solution 9 | Ionic polymer 9 (100) | - | Cyclopentanone (60) | Polyglycerol silicone 1 (14) |
| | | | | Carbon black (10) |
| Bio-electrode solution 10 | Ionic polymer 10 (100) | - | Cyclopentanone (60) | Polyglycerol silicone 1 (14) |
| | | | | Carbon black (10) |
| Bio-electrode solution 11 | Ionic polymer 11 (100) | - | Cyclopentanone (60) | Polyglycerol silicone 1 (14) |
| | | | | Carbon black (10) |
| Bio-electrode solution 12 | Ionic polymer 12 (100) | - | Cyclopentanone (60) | Polyglycerol silicone 1 (5) |
| | | | | Carbon black (10) |
| Bio-electrode solution 13 | Ionic polymer 13 (100) | - | Cyclopentanone (60) | Polyglycerol silicone 1 (5) |
| | | | | Carbon black (10) |
| Bio-electrode solution 14 | Ionic polymer 14 (100) | - | Cyclopentanone (60) | Polyglycerol silicone 1 (2) |
| | | | | Graphite (4) |
| Bio-electrode solution 15 | Ionic polymer 15 (100) | - | Cyclopentanone (60) | Lithium titanate (10) |
| Bio-electrode solution 16 | Ionic polymer 16 (100) | - | Cyclopentanone (60) | Polyglycerol silicone 1 (4) |
| | | | | Carbon black (12) |
| Bio-electrode solution 17 | Ionic polymer 17 (100) | - | Cyclopentanone (60) | Polyglycerol silicone 1 (4) |
| | | | | Carbon black (12) |
| Bio-electrode solution 18 | Ionic polymer 18 (100) | - | Cyclopentanone (60) | Polyglycerol silicone 1 (4) |
| | | | | Carbon black (12) |
| Bio-electrode solution 19 | Ionic polymer 19 (100) | - | Cyclopentanone (60) | Polyglycerol silicone 1 (4) |
| | | | | Carbon black (12) |

**[Table 2]**

| Bio-electrode solution | Ionic polymer (parts by mass) | Resin (parts by mass) | Solvent (parts by mass) | Additive (parts by mass) |
|---|---|---|---|---|
| Comparative bio-electrode solution 1 | - | Blend urethane 1 (100) | - | Carbon black (10) |

### (Preparation of Conductive Substrate X)

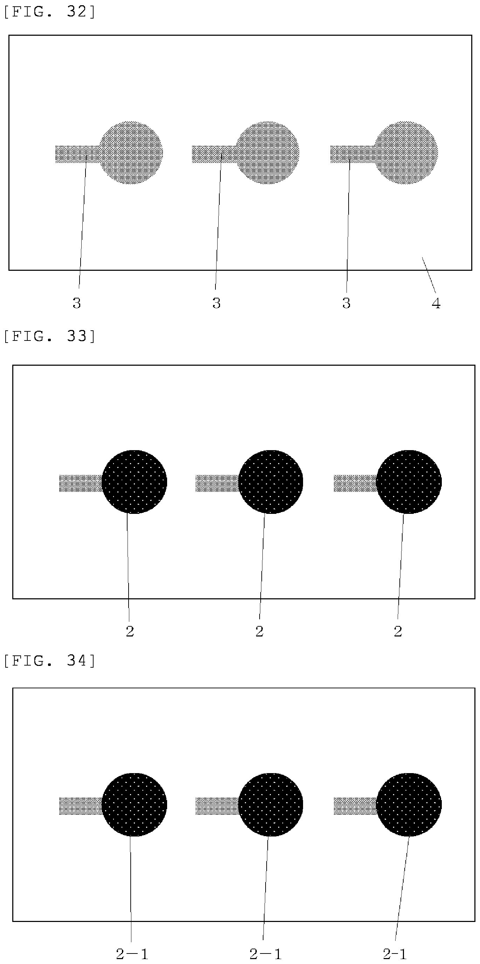

As in FIG. 32, a conductive paste, DOTITE FA-333, manufactured by Fujikura Kasei Co., Ltd., was applied on a polyethylene terephthalate (PET) film (substrate 4) having a film thickness of 50 um by screen printing, and baked in an oven at 120°C for 10 minutes to print a conductive pattern having a keyhole shape with 20 mm in diameter of circle (conductive layer 3), which was used as a conductive substrate X.

### (Preparation of Living-Body Contact Layer)

The bio-electrode solution described in Tables 1 and 2 was stencil-printed on the PET substrate in a size of 25 mm × 100 mm to produce an ionic polymer layer with 150 to 300 um in film thickness. Similarly, the bio-electrode solution was stencil-printed as in FIG. 33 on a circular pattern of a conductive paste illustrated in FIG. 32 to form a living-body contact layer 2.

### (Preparation of Mold)

Used was a mold made of stainless steel in which holes having a cross section illustrated in FIG. 14 were formed with a layout illustrated in FIG. 11. The holes were arranged at an angle of 60 degrees relative to the horizontal direction, the pitch of the holes in the 60-degree direction was 200 um, and the height of the pillars was 120 µm.

### (Preparation of Micropillar-Type Bio-Electrode)

As illustrated in FIG. 34, the mold was heated at 120°C and pressurized at a pressure of 50 kg/cm² for 5 minutes on each of the living-body contact layers with a size of 25 mm × 100 mm and with circles with 20 mm, and then the mold was released to form micropillar-type living-body contact layers 2-1.

The layer with a size of 25 mm × 100 mm was used for measuring a skin adhesive force. The layer was attached to skin on an inside of an upper arm at a pressure of 2 kg, and then a peeling test was performed at an angle of 180 degrees. Table 3 shows the results.

### (Measurement of Bio-Signal)

The layer with circle of 20 mm in diameter illustrated in FIG. 34 was used for measuring a bio-signal (ECG). Three of the bio-electrodes in FIG. 34 were cut and attached to skin. Since the bio-electrode of Comparative Example 1 had no adhesive force, the bio-electrode was attached to the skin with a cellophane tape.

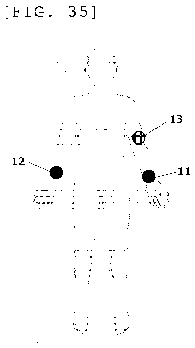

A used ECG measurement device was NeXus10 MARKII, manufactured by KISSEI COMTEC Co., Ltd. In FIG. 35, the sign 11 represents a positive electrode, the sign 12 represents a negative electrode, and the sing 13 represents an earth.

A case where PQRST waves of the ECG signal were observed was judged as a good signal, and a case where the waves were not observed was judged as a poor signal. Table 3 shows the results.

**[Table 3]**

| Example | Bio-electrode solution | Surface shape of living-body contact layer | Conductive substrate | Adhessive force (N/25-mm) | ECG signal |
|---|---|---|---|---|---|
| Example 1 | Bio-electrode solution 1 | Micropillar | Conductive substrate x | 0.4 | Good |
| Example 2 | Bio-electrode solution 2 | Micropillar | Conductive substrate x | 0.5 | Good |
| Example 3 | Bio-electrode solution 3 | Micropillar | Conductive substrate x | 0.3 | Good |
| Example 4 | Bio-electrode solution 4 | Micropillar | Conductive substrate x | 0.9 | Good |
| Example 5 | Bio-electrode solution 5 | Micropillar | Conductive substrate x | 0.8 | Good |
| Example 6 | Bio-electrode solution 6 | Micropillar | Conductive substrate x | 1.1 | Good |
| Example 7 | Bio-electrode solution 7 | Micropillar | Conductive substrate x | 1.2 | Good |
| Example 8 | Bio-electrode solution 8 | Micropillar | Conductive substrate x | 0.7 | Good |
| Example 9 | Bio-electrode solution 9 | Micropillar | Conductive substrate x | 1.1 | Good |
| Example 10 | Bio-electrode solution 10 | Micropillar | Conductive substrate x | 1.0 | Good |
| Example 11 | Bio-electrode solution 11 | Micropillar | Conductive substrate x | 0.9 | Good |
| Example 12 | Bio-electrode solution 12 | Micropillar | Conductive substrate x | 1.3 | Good |
| Example 13 | Bio-electrode solution 13 | Micropillar | Conductive substrate x | 1.4 | Good |
| Example 14 | Bio-electrode solution 14 | Micropillar | Conductive substrate x | 1.5 | Good |
| Example 15 | Bio-electrode solution 15 | Micropillar | Conductive substrate x | 1.1 | Good |
| Example 16 | Bio-electrode solution 16 | Micropillar | Conductive substrate x | 1.2 | Good |
| Example 17 | Bio-electrode solution 17 | Micropillar | Conductive substrate x | 0.8 | Good |
| Example 18 | Bio-electrode solution 18 | Micropillar | Conductive substrate x | 1.2 | Good |
| Example 19 | Bio-electrode solution 19 | Micropillar | Conductive substrate x | 1.3 | Good |
| Comparative Example 1 | Bio-electrode solution 1 | Plane | Conductive substrate x | 0 | Good |
| Comparative Example 2 | Comparative bio-electrode solution 1 | Micropillar | Conductive substrate x | 0.5 | Poor |

As shown in Table 3, Examples 1 to 19, which were the bio-electrodes of the present invention using the living-body contact layer that had the micropillars having a diameter within a range of 0.01 to 500 um and a height within a range of 0.1 to 1,000 um, that had one or more repeating units-a selected from a fluorosulfonic acid, a fluorosulfonimide, and a fluorosulfonamide as the ionic polymer of the material for forming the living-body contact layer, and that had a weight-average molecular weight within a range of 1,000 to 500,000, yielded the excellent adhesive force to obtain the bio-signal.

Meanwhile, Comparative Example 1, which was the bio-electrode even using the ionic polymer of the material for forming the living-body contact layer that had one or more repeating units-a selected from a fluorosulfonic acid, a fluorosulfonimide, and a fluorosulfonamide and that had a weight-average molecular weight within a range of 1,000 to 500,000 but the living-body contact layer had no micropillar having a diameter within a range of 0.01 to 500 um and having a height within a range of 0.1 to 1,000 um, yielded the bio-signal but no adhesive force. Comparative Example 2, which was the bio-electrode even using the living-body contact layer having the micropillars having a diameter within a range of 0.01 to 500 um and a height within a range of 0.1 to 1,000 um but using no ionic polymer as the material for forming the living-body contact layer, had the adhesive force but failed to obtain the bio-signal.

The present description includes the following embodiments.
[1]: A bio-electrode, comprising:
   a living-body contact layer; and
   a substrate, wherein
   the living-body contact layer has a micropillar having a diameter within a range of 0.01 to 500 um and a height within a range of 0.1 to 1,000 um, and has an ionic polymer (A) as a material, and
   the component (A) has a repeating unit-a of one or more selected from a fluorosulfonic acid, a fluorosulfonimide, and a fluorosulfonamide, and has a weight-average molecular weight within a range of 1,000 to 500,000.
[2]: The bio-electrode of the above [1], wherein the repeating unit-a has a partial structure represented by the following general formulae (1)-1 to (1)-4, 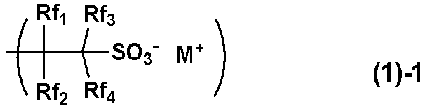 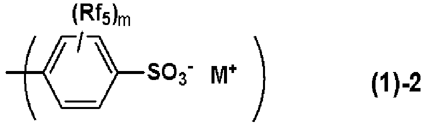 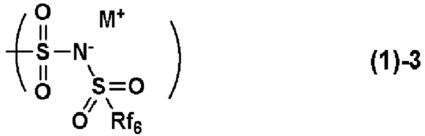 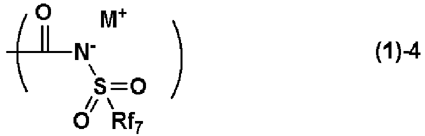 wherein Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; M⁺ represents an ion selected from an ammonium ion, a sodium ion, a potassium ion, and a silver ion; and "m" represents an integer of 1 to 4.
[3]: The bio-electrode of the above [1] or [2], wherein the repeating unit-a has one or more selected from repeating units-A1 to -A4 represented by the following general formulae (2), 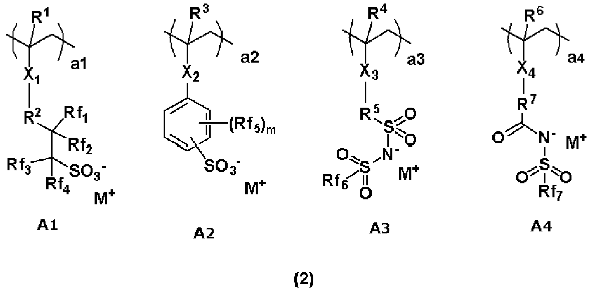 wherein R¹, R³, R⁴, and R⁶ each independently represent a hydrogen atom or a methyl group; R², R⁵, and R⁷ each independently represent a single bond or a linear, branched, or cyclic hydrocarbon group having 1 to 13 carbon atoms and optionally having an ester group, an ether group, or both of them; X₁, X₂, X₃, and X₄ each independently represent any one of a single bond, a phenylene group, a naphthylene group, an ether group, an ester group, and an amide group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; "m" represents an integer of 1 to 4; a1, a2, a3, and a4 satisfy 0≤a1≤1.0, 0≤a2≤1.0, 0≤a3≤1.0, 0≤a4≤1.0, and 0<a1+a2+a3+a4≤1.0; and M⁺ represents an ion selected from an ammonium ion, a sodium ion, a potassium ion, and a silver ion.
[4]: The bio-electrode of any one of the above [1] to [3], wherein the repeating unit-a has one or more selected from repeating units-A5 to -A7 represented by the following general formulae (4), 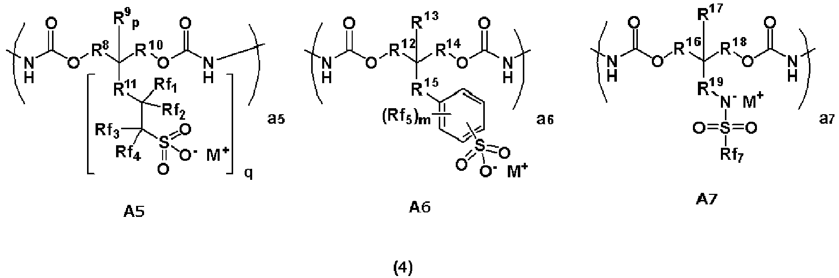 wherein R⁸, R¹⁰, R¹², R¹⁴, R¹⁶, and R¹⁸ each independently represent a single bond or a linear or branched alkylene group having 1 to 6 carbon atoms; R⁹, R¹³, and R¹⁷ each independently represent a hydrogen atom, a hydroxy group, or a linear or branched alkyl group or alkoxy group having 1 to 6 carbon atoms; R¹¹ and R¹⁹ represent a single bond or a linear, branched, or cyclic hydrocarbylene group having 1 to 20 carbon atoms and optionally having one or more selected from a carbonyl group, an ether group, and an ester group, and optionally bonded to R⁸ or R¹⁶ to form a ring; R¹⁵ represents a single bond or a linear, branched, or cyclic hydrocarbylene group having 1 to 20 carbon atoms and optionally having one or more selected from a carbonyl group, an ether group, and an ester group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₇ represents a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₇ having at least one or more fluorine atoms; "m" represents an integer of 1 to 4; a5, a6, and a7 satisfy 0≤a5≤1.0, 0≤a6≤1.0, 0≤a7≤1.0, and 0<a5+a6+a7≤1.0; M⁺ represents an ion selected from an ammonium ion, a sodium ion, a potassium ion, and a silver ion; "p" represents 0 or 1; and "q" represents 1 or 2.
[5]: The bio-electrode of any one of the above [2] to [4], wherein the repeating unit-a contains an ammonium ion represented by the following general formula (3) as the ammonium ion constituting the ammonium salt, 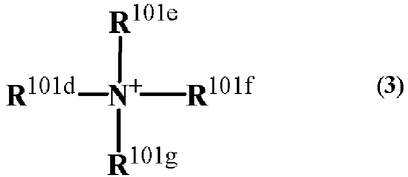 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, optionally having one or more selected from an ether group, a carbonyl group, an ester group, a hydroxy group, a carboxyl group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring together with a nitrogen atom to which these groups are bonded, and when forming the ring, R^{101d} and R^{101e}, or R^{101d}, R^{101e}, and R^{101f} form an alkylene group having 3 to 10 carbon atoms or a heteroaromatic ring having the nitrogen atom in the general formula (3) in the ring.
[6]: The bio-electrode of any one of the above [1] to [5], further comprising a resin other than the component (A) as a component (B) contained in the material of the living-body contact layer.
[7]: The bio-electrode of the above [6], wherein the component (B) is one or more selected from a silicone resin, a (meth)acrylate resin, and a urethane resin.
[8]: A method for manufacturing the bio-electrode of any one of the above [1] to [7], the method comprising a step of manufacturing the micropillar by inserting a resin containing the component (A) into a mold to be a template, and releasing the resin from the mold.
[9]: A method for manufacturing the bio-electrode of any one of the above [1] to [7], the method comprising a step of manufacturing the micropillar by inserting a resin containing the component (A) into a pattern of a resin to be a template, and dissolving a pattern of the resin with water or an alkaline aqueous solution to release the resin containing the component (A) .

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A bio-electrode, comprising:
a living-body contact layer; and
a substrate, wherein
the living-body contact layer has a micropillar having a diameter within a range of 0.01 to 500 um and a height within a range of 0.1 to 1,000 um, and has an ionic polymer (A) as a material, and
the component (A) has a repeating unit-a of one or more selected from a fluorosulfonic acid, a fluorosulfonimide, and a fluorosulfonamide, and has a weight-average molecular weight within a range of 1,000 to 500,000.

2. The bio-electrode according to claim 1, wherein the repeating unit-a has a partial structure represented by the following general formulae (1)-1 to (1)-4, 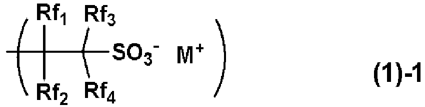 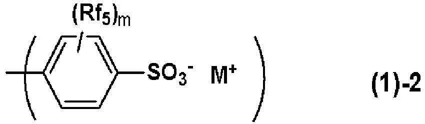 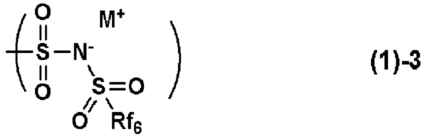 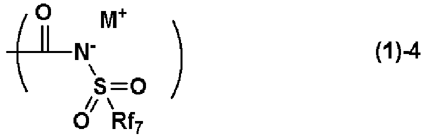 wherein Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; M⁺ represents an ion selected from an ammonium ion, a sodium ion, a potassium ion, and a silver ion; and "m" represents an integer of 1 to 4.

3. The bio-electrode according to claim 1 or 2, wherein the repeating unit-a has one or more selected from repeating units-A1 to -A4 represented by the following general formulae (2), 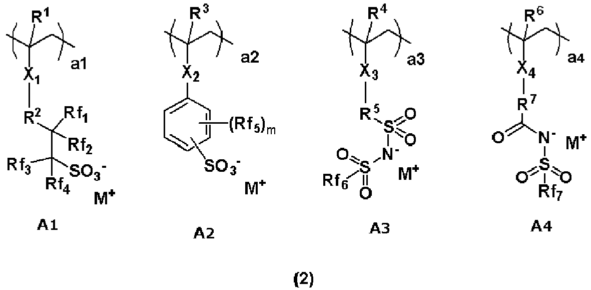 wherein R¹, R³, R⁴, and R⁶ each independently represent a hydrogen atom or a methyl group; R², R⁵, and R⁷ each independently represent a single bond or a linear, branched, or cyclic hydrocarbon group having 1 to 13 carbon atoms and optionally having an ester group, an ether group, or both of them; X₁, X₂, X₃, and X₄ each independently represent any one of a single bond, a phenylene group, a naphthylene group, an ether group, an ester group, and an amide group; Rf₂ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₆ and Rf₇ represent a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₆ and Rf₇ having at least one or more fluorine atoms; "m" represents an integer of 1 to 4; a1, a2, a3, and a4 satisfy 0≤a1≤1.0, 0≤a2≤1.0, 0≤a3≤1.0, 0≤a4≤1.0, and 0<a1+a2+a3+a4≤1.0; and M⁺ represents an ion selected from an ammonium ion, a sodium ion, a potassium ion, and a silver ion.

4. The bio-electrode according to any one of claims 1 to 3, wherein the repeating unit-a has one or more selected from repeating units-A5 to -A7 represented by the following general formulae (4), 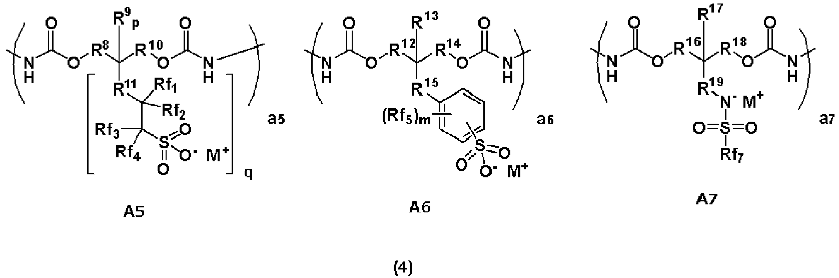 wherein R⁸, R¹⁰, R¹², R¹⁴, R¹⁶, and R¹⁸ each independently represent a single bond or a linear or branched alkylene group having 1 to 6 carbon atoms; R⁹, R¹³, and R¹⁷ each independently represent a hydrogen atom, a hydroxy group, or a linear or branched alkyl group or alkoxy group having 1 to 6 carbon atoms; R¹¹ and R¹⁹ represent a single bond or a linear, branched, or cyclic hydrocarbylene group having 1 to 20 carbon atoms and optionally having one or more selected from a carbonyl group, an ether group, and an ester group, and optionally bonded to R⁸ or R¹⁶ to form a ring; R¹⁵ represents a single bond or a linear, branched, or cyclic hydrocarbylene group having 1 to 20 carbon atoms and optionally having one or more selected from a carbonyl group, an ether group, and an ester group; Rf₁ and Rf₂ represent a hydrogen atom, a fluorine atom, an oxygen atom, a methyl group, or a trifluoromethyl group, and when Rf₁ and Rf₂ represent an oxygen atom, Rf₁ and Rf₂ represent one oxygen atom bonded to one carbon atom to form a carbonyl group; Rf₃ and Rf₄ represent a hydrogen atom, a fluorine atom, or a trifluoromethyl group, and one or more of Rf₁ to Rf₄ represent a fluorine atom or a trifluoromethyl group; Rf₅ represents a fluorine atom or a linear or branched alkyl group having 1 to 4 carbon atoms and having at least one or more fluorine atoms; Rf₇ represents a linear, branched, or cyclic alkyl group having 1 to 10 carbon atoms, an alkenyl group having 2 to 6 carbon atoms, an alkynyl group having 2 to 6 carbon atoms, or an aryl group having 6 to 10 carbon atoms, and optionally substituted with a fluorine atom, Rf₇ having at least one or more fluorine atoms; "m" represents an integer of 1 to 4; a5, a6, and a7 satisfy 0≤a5≤1.0, 0≤a6≤1.0, 0≤a7≤1.0, and 0<a5+a6+a7≤1.0; M⁺ represents an ion selected from an ammonium ion, a sodium ion, a potassium ion, and a silver ion; "p" represents 0 or 1; and "q" represents 1 or 2.

5. The bio-electrode according to any one of claims 2 to 4, wherein the repeating unit-a contains an ammonium ion represented by the following general formula (3) as the ammonium ion constituting the ammonium salt, 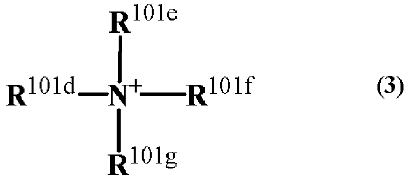 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, optionally having one or more selected from an ether group, a carbonyl group, an ester group, a hydroxy group, a carboxyl group, an amino group, a nitro group, a sulfonyl group, a sulfinyl group, a halogen atom, and a sulfur atom; R^{101d} and R^{101e}, or R^{101d}, R^{101e}, and R^{101f} optionally form a ring together with a nitrogen atom to which these groups are bonded, and when forming the ring, R^{101d} and R^{101e}, or R^{101d}, R^{101e}, and R^{101f} form an alkylene group having 3 to 10 carbon atoms or a heteroaromatic ring having the nitrogen atom in the general formula (3) in the ring.

6. The bio-electrode according to any one of claims 1 to 5, further comprising a resin other than the component (A) as a component (B) contained in the material of the living-body contact layer.

7. The bio-electrode according to claim 6, wherein the component (B) is one or more selected from a silicone resin, a (meth)acrylate resin, and a urethane resin.

8. A method for manufacturing the bio-electrode according to any one of claims 1 to 7, the method comprising a step of manufacturing the micropillar by inserting a resin containing the component (A) into a mold to be a template, and releasing the resin from the mold.

9. A method for manufacturing the bio-electrode according to any one of claims 1 to 7, the method comprising a step of manufacturing the micropillar by inserting a resin containing the component (A) into a pattern of a resin to be a template, and dissolving a pattern of the resin with water or an alkaline aqueous solution to release the resin containing the component (A) .
